(19) Europäisches Patentamt
European Patent Office
Office européen des brevets

(11) **EP 4 573 214 B1**

(12) **EUROPEAN PATENT SPECIFICATION**

(45) Date of publication and mention
of the grant of the patent:
**11.03.2026 Bulletin 2026/11**

(21) Application number: **23755500.8**

(22) Date of filing: **05.08.2023**

(51) International Patent Classification (IPC):
***C12Q 1/6853*** (2018.01)     ***C12Q 1/6806*** (2018.01)

(52) Cooperative Patent Classification (CPC):
(C-Sets available)
**C12Q 1/6853; C12Q 1/6806**     (Cont.)

(86) International application number:
**PCT/SE2023/050782**

(87) International publication number:
**WO 2024/039272 (22.02.2024 Gazette 2024/08)**

(54) **NUCLEIC ACID AMPLIFICATION**

NUKLEINSÄUREAMPLIFIKATION

AMPLIFICATION D'ACIDES NUCLÉIQUES

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority: **15.08.2022 SE 2250964**

(43) Date of publication of application:
**25.06.2025 Bulletin 2025/26**

(73) Proprietor: **Simsen Diagnostics AB**
**411 26 Göteborg (SE)**

(72) Inventors:
• **STÅHLBERG, Anders**
**428 34 Kållered (SE)**

• **JOHANSSON, Gustav**
**421 51 Västra Frölunda (SE)**
• **ANDERSSON, Daniel**
**414 83 Göteborg (SE)**

(74) Representative: **Barker Brettell Sweden AB**
**Kungsbroplan 3**
**112 27 Stockholm (SE)**

(56) References cited:
**WO-A1-2016/138080     WO-A1-2019/140298
WO-A1-2021/035056**

Remarks:
The complete document including Reference
Table(s) and the Sequence Listing(s) can be
downloaded from the EPO website

(52) Cooperative Patent Classification (CPC): (Cont.)

C-Sets
**C12Q 1/6806, C12Q 2525/155, C12Q 2525/179,
C12Q 2527/101, C12Q 2527/107, C12Q 2535/122,
C12Q 2537/101, C12Q 2549/119, C12Q 2563/179;
C12Q 1/6853, C12Q 2525/155, C12Q 2525/179,
C12Q 2527/101, C12Q 2527/107, C12Q 2535/122,
C12Q 2537/101, C12Q 2549/119, C12Q 2563/179**

**Description**

TECHNICAL FIELD

The present embodiments generally relate to nucleic acid amplification and in particular to nucleic acid amplification using barcoded primers.

BACKGROUND

Massive parallel sequencing enables a growing number of clinical and basic research applications within many diverse areas, including diagnostics, treatment stratification, drug discovery, forensics, evolutionary studies and environmental DNA testing. Essentially, any type of biological sample can be analyzed, comprising complex samples matrices, such as tissues, body fluids and environmental samples, highly variable sample sizes, ranging from individual molecules to billions of cells, and DNA with different integrity, such intact DNA extracted from living cells to highly fragmented DNA that are typical for tissue fixation, body fluids and forensics samples. Furthermore, numerous approaches exist to analyze anything from a limited number of targeted gene sequences to entire genomes. Conventional sequencing has the sensitivity to detect Variant Allele Frequencies (VAFs) down to 1 - 5%. This is not adequate for several emerging applications, such as circulating tumor DNA analysis, requiring reliable detection of VAFs < 0.1%, sometimes even individual molecules. To overcome this issue, Unique Molecular Identifiers (UMIs), also known as molecular barcodes, are introduced in library construction to enable technical noise reduction in the downstream bioinformatical data analysis. The UMI, typically consisting of an 8 - 12 nucleotides long and randomized sequence, is added to target DNA using either PCR or hybridization capture-based approaches. Thus, all sequence reads with identical UMI can be bioinformatically traced back to the same template DNA molecule, generating consensus reads that enable polymerase-induced error correction. However, while superior quality of sequencing data is provided by UMIs, most experimental protocols are complicated and includes multi-step protocols. The primary experimental limitation is that the random sequence of UMIs is prone to generate non-specific PCR products that interfere with library construction, especially in PCR-based approaches.

U.S. Patent No. 10,557,134 discloses approaches for improved detection, identification, and/or quantification of target nucleic acids. These approaches provide a means of detecting, identifying, and/or quantifying rare target nucleic acid molecules, including deoxyribonucleic acid (DNA) and ribonucleic acid (RNA) molecules, from the same sample, and in the same reaction, by using hairpin barcode primers to incorporate unique barcodes into target nucleic acids in a polymerase chain reaction (PCR) pre-amplification step.

U.S. Patent No. 9,506,119 and U.S. Publication No. 2014/0255929 disclose the use of sequence tags to improve sequence determination of amplicons of related sequences, particularly large and complex amplicons, such as those comprising recombined nucleic acids encoding immune receptor molecules. Sequence reads having the same sequence tags are aligned after which final base calls are determined from an average base call from sequence read base calls at each position. Sequence reads comprising series of incorporation signals are aligned by common sequence tags and base calls in homopolymer regions are made as a function incorporation signal values at each flow position.

WO 2016/138080 discloses approaches for detection, identification, and/or quantification of target nucleic acids. These approaches provide a means of detecting, identifying, and/or quantifying rare target nucleic acid molecules, including DNA and RNA molecules, from the same sample, and in the same reaction, by using "hairpin barcode primers" to incorporate unique barcodes into target nucleic acids in a PCR pre-amplification step.

SUMMARY

It is a general objective to provide a method for amplifying nucleic acid molecules in a sample.

This and other objectives are met by embodiments as disclosed herein.

The present invention is defined in the independent claims. Further embodiments are defined in the dependent claims.

An aspect of the invention relates to a method of amplifying a target nucleic acid molecule in a sample. The method comprises contacting a sample comprising a target nucleic acid molecule with a forward primer and a reverse primer. The forward primer comprises, from a 5' end to a 3' end, an adapter sequence and a target-specific sequence and the reverse primer comprises, from a 5' end to a 3' end, an adapter sequence and a target-specific sequence. The forward primer is a hairpin barcode forward primer and/or the reverse primer is a hairpin barcode reverse primer. The hairpin barcode forward primer comprises, from the 5' end to the 3' end, the adapter sequence, a unique molecular identifier (UMI) sequence comprising a structured UMI, and the target-specific sequence complementary to a portion of the target nucleic acid molecule. The hairpin barcode reverse primer comprises, from the 5' end to the 3' end, the adapter sequence, a UMI sequence comprising a structured UMI, and the target-specific sequence complementary to a portion of the target nucleic acid molecule. At least a portion of the adapter sequence of the hairpin barcode forward primer and/or the hairpin barcode reverse primer is complementary to at least a portion of the UMI sequence of the hairpin barcode forward primer and/or the hairpin barcode reverse primer. The adapter sequence and the UMI sequence are configured to hybridize to each other at or under a closed annealing temperature and not hybridize to each other at or above an open annealing temperature. The method also comprises amplifying the target nucleic acid molecule by performing polymerase chain reaction (PCR) pre-amplification of the target nucleic acid molecule to form a plurality of barcoded PCR products. The PCR pre-amplification has an annealing temperature equal to or less than the closed annealing temperature of the hairpin barcode forward primer and/or the hairpin barcode reverse primer. The method further comprises contacting the plurality of barcoded PCR products with an adapter-specific forward primer and an adapter-specific reverse primer and amplifying the barcoded PCR products by performing PCR amplification on the barcoded PCR products to form amplified barcoded PCR products. At least a portion of cycles of the PCR amplification has an annealing temperature equal to or greater than the open annealing temperature of the hairpin barcode forward primer and/or the hairpin barcode reverse primer.

Another aspect of the invention relates to a method for quantifying nucleic acid molecules. The method comprises contacting a sample comprising nucleic acid molecules with P forward primers and Q reverse primers. P is an integer equal to or larger than one and Q is an integer equal to or larger than one. The P forward primers comprise, from a 5' end to a 3' end, an adapter sequence and a target-specific sequence and the Q reverse primers comprise, from a 5' end to a 3' end, an adapter sequence and a target-specific sequence. The P forward are P hairpin barcode forward primers and/or the Q reverse primers are Q hairpin barcode reverse primers. Each hairpin barcode forward primer comprises, from the 5' end to the 3' end, the adapter sequence, a UMI sequence comprising a structured UMI, and the target-specific sequence complementary to a respective portion of a nucleic acid molecule. Each hairpin barcode reverse primer comprises, from the 5' end to the 3' end, the adapter sequence, a UMI sequence comprising a structured UMI, and the target-specific sequence complementary to a respective portion of a nucleic acid molecule. At least a portion of the adapter sequence of the hairpin barcode forward primer and/or the hairpin barcode reverse primer is complementary to at least a portion of the UMI sequence of the hairpin barcode forward primer and/or the hairpin barcode reverse primer. The adapter sequence and the UMI sequence are configured to hybridize to each other at or under a closed annealing temperature and not hybridize to each other at or above an open annealing temperature. The method also comprises amplifying the nucleic acid molecules by performing PCR pre-amplification of the nucleic acid molecules to form a plurality of barcoded PCR products. The PCR pre-amplification has an annealing temperature equal to or less than the closed annealing temperature of the hairpin barcode forward primers and/or the hairpin barcode reverse primers. The method further comprises contacting the plurality of barcoded PCR products with an adapter-specific forward primer and an adapter-specific reverse primer and amplifying the barcoded PCR products by performing PCR amplification on the barcoded PCR products to form a library of amplified barcoded PCR products. At least a portion of cycles of the PCR amplification has an annealing temperature equal to or greater than the open annealing temperature of the hairpin barcode forward primers and/or the hairpin barcode reverse primers. The method additionally comprises sequencing at least a respective portion of the amplified barcoded PCR products to form respective sequence reads comprising the UMI(s) and nucleic acid molecule sequence(s). The method also comprises demultiplexing the sequence reads based on nucleic acid sequences of the UMIs and mapping the demultiplexed sequence reads to respective known nucleic acid molecule sequences based on nucleic acid sequences of the nucleic acid molecule sequence(s). The method further comprises quantifying unique nucleic acid molecules based on the demultiplexed and mapped sequence reads.

The present invention provides an amplification method solving issues with non-uniform amplification of nucleic acid molecules and polymerase-induced errors. The invention can be applied to various sample types, including enriched and non-enriched cell populations. The method is simple to conduct, does not require any target nucleic acid molecule capture and provides quantitative information of amplified nucleic acid molecules.

BRIEF DESCRIPTION OF THE DRAWINGS

The embodiments, together with further objects and advantages thereof, may best be understood by making reference to the following description taken together with the accompanying drawings, in which:

Figure 1. Flow chart illustrating a method of amplifying a target nucleic acid molecule according to an embodiment.

Figure 2. Flow chart illustrating a method for quantifying nucleic acid molecules according to an embodiment.

Figure 3. Schematic illustrations of primers (A, B, C) and amplification steps (A) that can be used in a method of amplifying a target nucleic acid molecule according to various embodiments.

Figure 4. Schematic illustration of primers (A, B, C) that can be used in a method of amplifying a target nucleic acid molecule according to various embodiments.

Figure 5. Illustration of different hairpin barcode primer structures. (A) Prior art hairpin barcode primer. (B) Modified hairpin barcode primer with barcode distributed in hairpin loop. (C) Double-loop hairpin barcode primer with barcode distributed in the hairpin loops. (D) Integrated barcode with barcode along stem region.

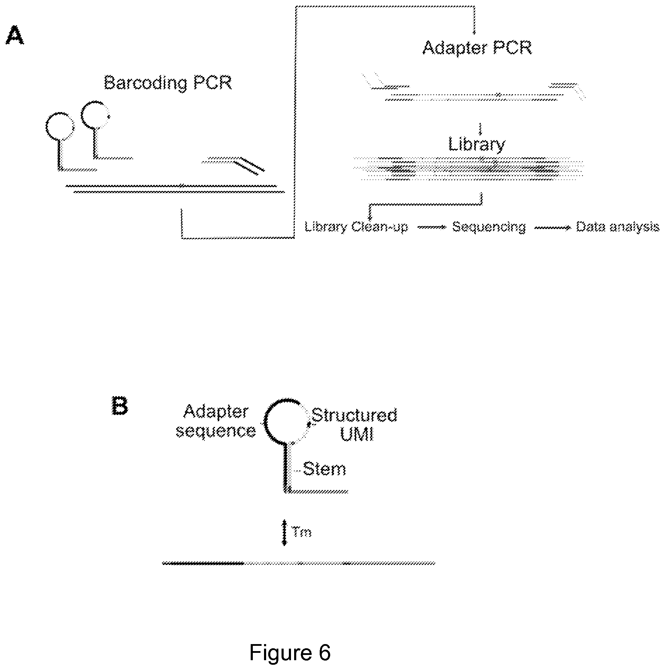

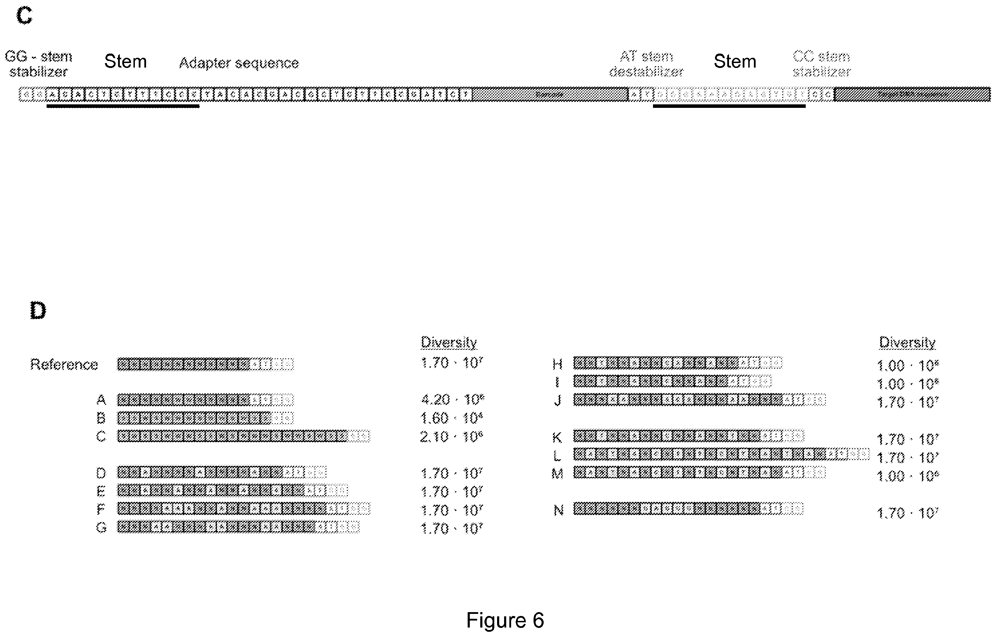

Figure 6. Digital sequencing using SiMSen-Seq and structured UMIs. (A) Schematic structure and function of barcode primers with stem-loop protected UMI. Stem opening and closing is temperature dependent. Tm, melting temperature. (B) Overview of the SiMSen-Seq workflow. (C) Sequence design of forward barcoding primer. Different primer elements are indicated by color and name. The stem is stabilized by two nucleotide pairs (GG- and CC stem stabilizers) and destabilized by two nucleotides (AT stem destabilizer). The AT stem destabilizer prohibits the UMI from extending the stem. (D) Design of structured UMIs. The sequence contexts of reference UMI and 14 structured UMIs (A - N) are shown. Designs B and C lack the AT stem destabilizer. Nucleotide N represents any nucleotide type; nucleotide S represents cytosine or guanine; nucleotide W represents adenine or thymine.

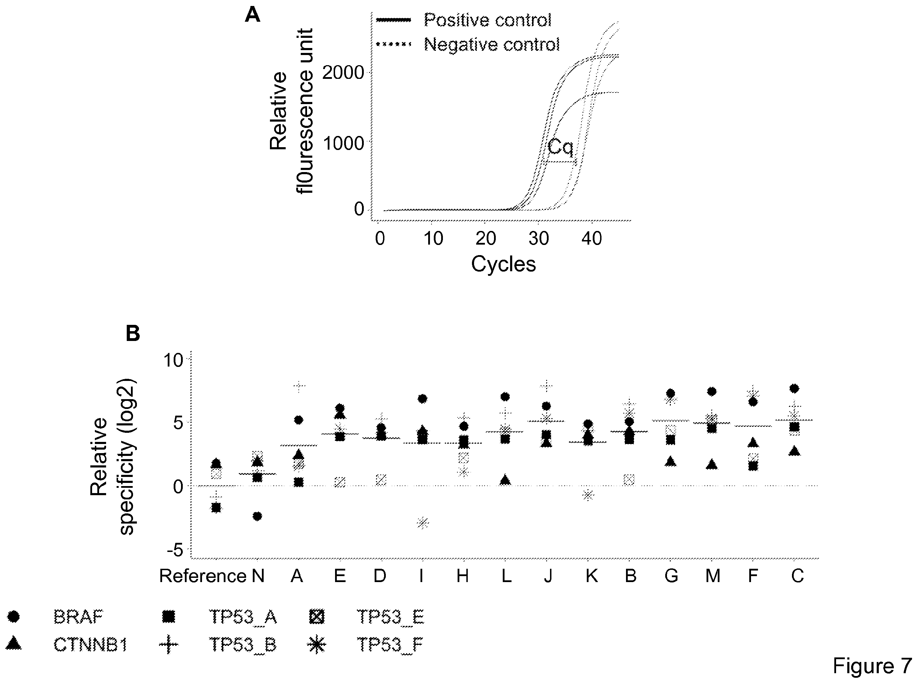

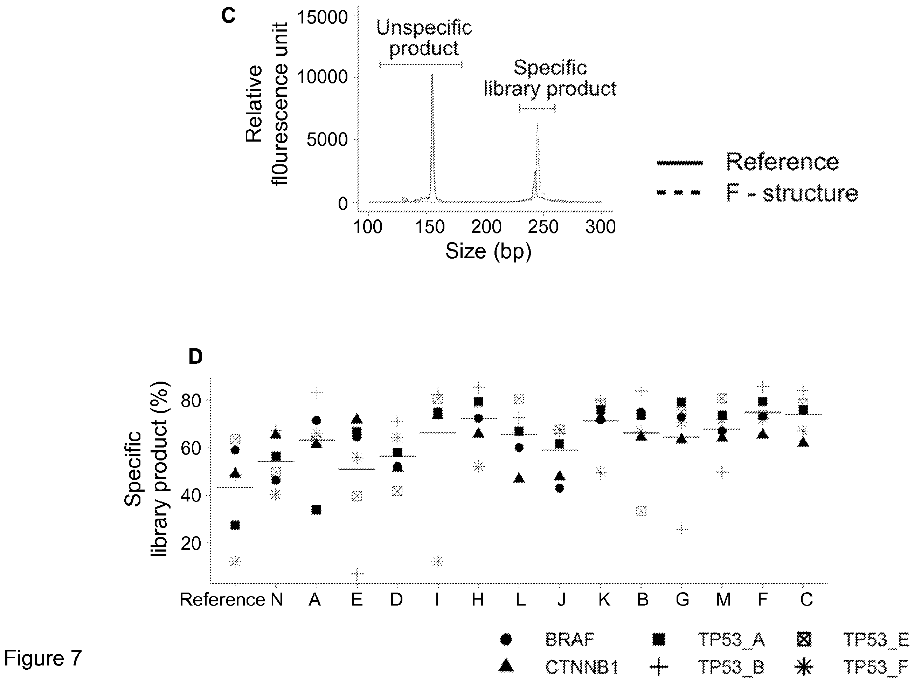

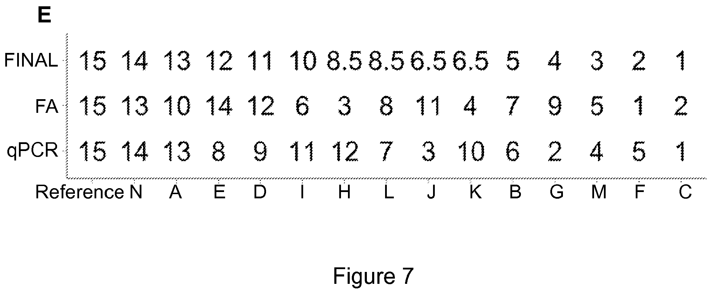

Figure 7. Assay performance using different structured UMIs. (A) Assay performance using quantitative PCR. The relative specificity is indicated as $\Delta Cq$, which is the difference in cycle of quantification values between samples with (full lines) and without (hatched lines) DNA. The figure shows the amplification curves of a representative assay (TP53_A, UMI design F), n = 3. (B) Relative specificity using quantitative PCR. Data for all structured UMIs (A-N) and the reference UMI are shown for six assays. Mean value for each assay is shown, n = 3. The mean of all assays is indicated by a bar for each UMI design. Data are scaled to the reference UMI with a mean value of zero. (C) Assay performance using capillary gel electrophoresis. The electropherograms exemplify representative libraries for a representative assay (TP53_A) using reference UMI and UMI design (J), respectively, n = 1. The specific library product is indicated with a bar. (D) Library yield determined with capillary gel electrophoresis. The percentage of correctly formed library relative all generated PCR product is shown for six assays with mean indicated by a bar, n = 1. (E) Performance ranking of structured UMIs. The total ranking sum of all the different structured UMIs based on their relative performance using quantitative PCR and capillary gel electrophoresis data. The order of UMI designs in subfigures B and D are based on their final ranks.

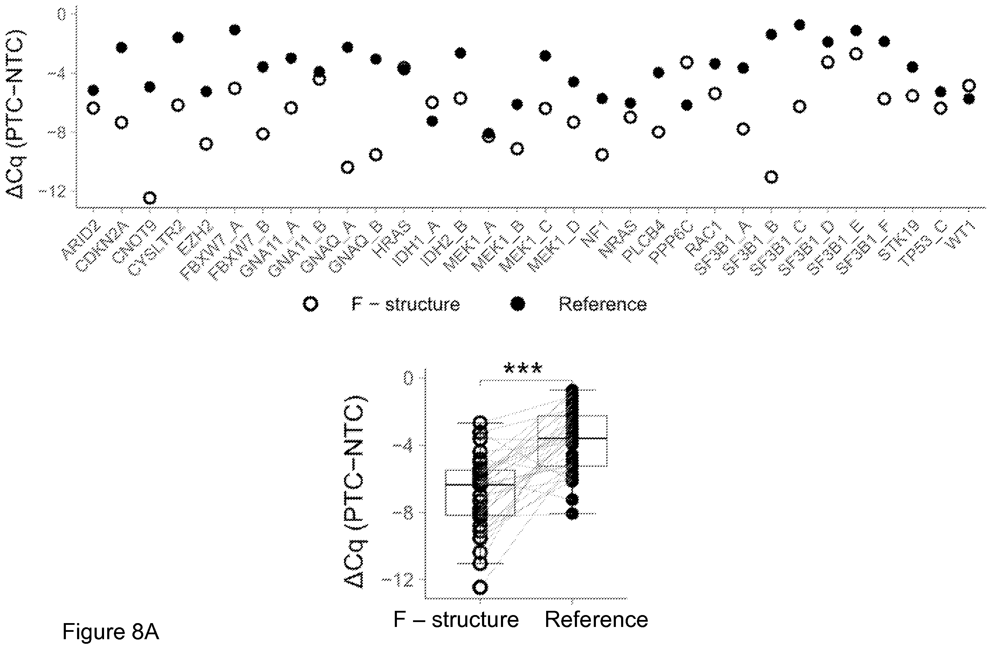

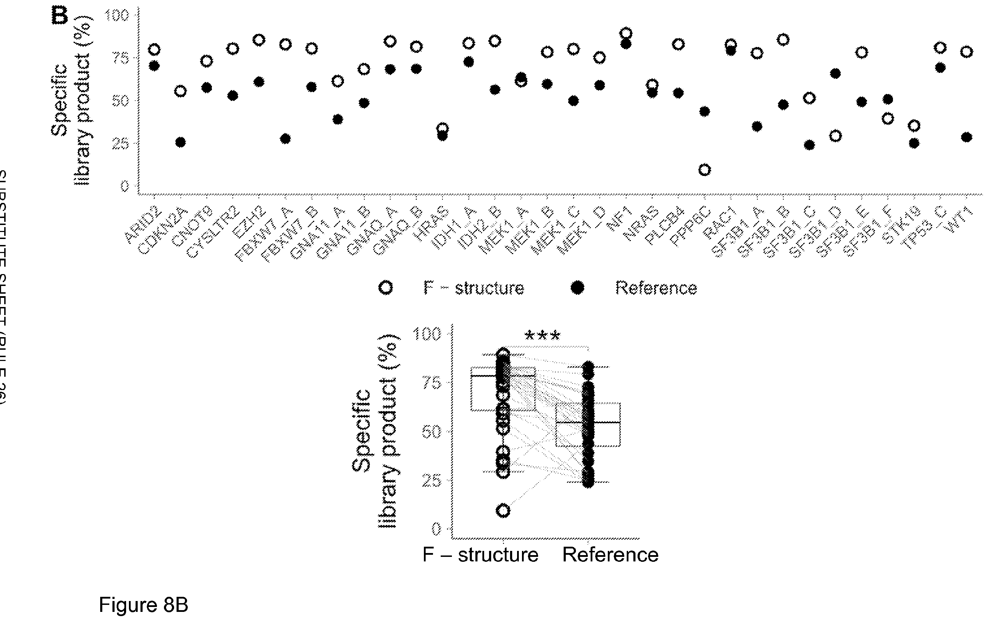

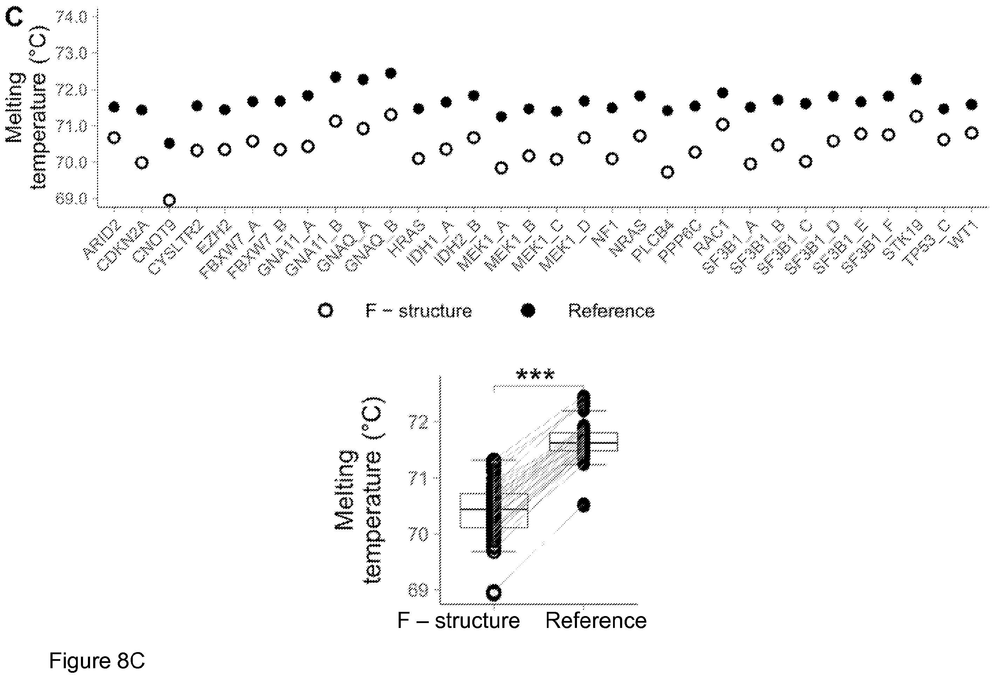

Figure 8. Validation of improved assay performance using structured UMIs. (A) Relative specificity using quantitative PCR. Data for UMI design (F) and the reference UMI are shown for 32 assays. Mean $\Delta Cq$ value of samples with 20 ng DNA (PTC) compared with water (NTC) is shown for each assay and UMI design, n = 3. Box plot of all data is shown to the right. *** $p \leq 0.001$, paired Student's t-test. (B) Specificity in correct library products using capillary gel electrophoresis. Data for UMI design (F) and the reference UMI are shown for 32 assays, n = 1. Box plot of all data is shown to the right. *** $p \leq 0.001$, paired Student's t-test. (C) Melting temperature of forward barcoding primer. Data for UMI design (F) and the reference UMI are shown for 32 assays. Mean melting temperature is shown for each assay and UMI design, n = 2. Box plot of all data is shown to the right. *** $p \leq 0.001$, paired Student's t-test.

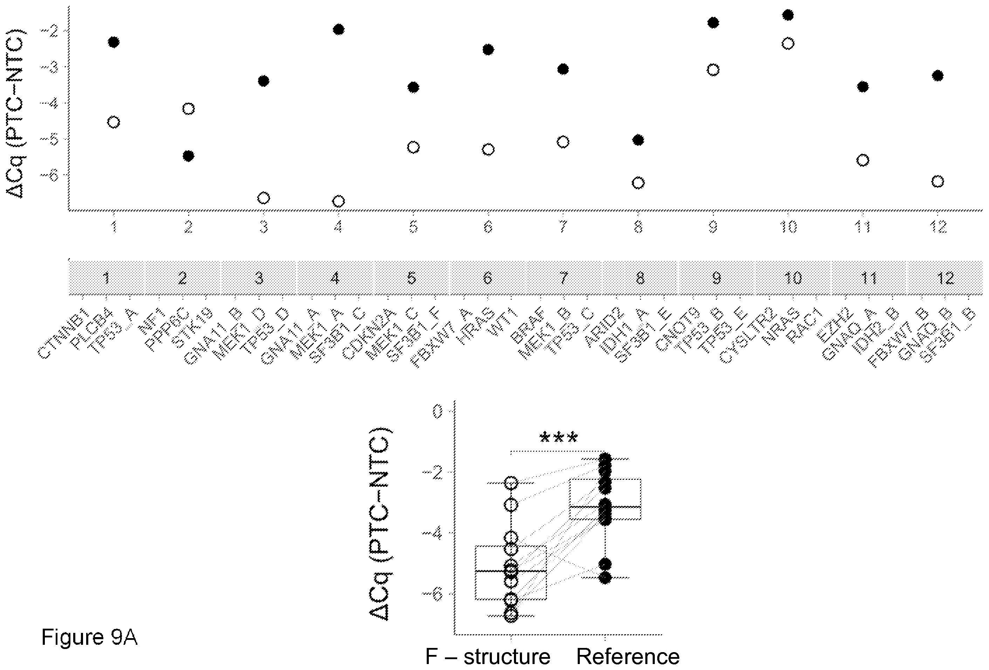

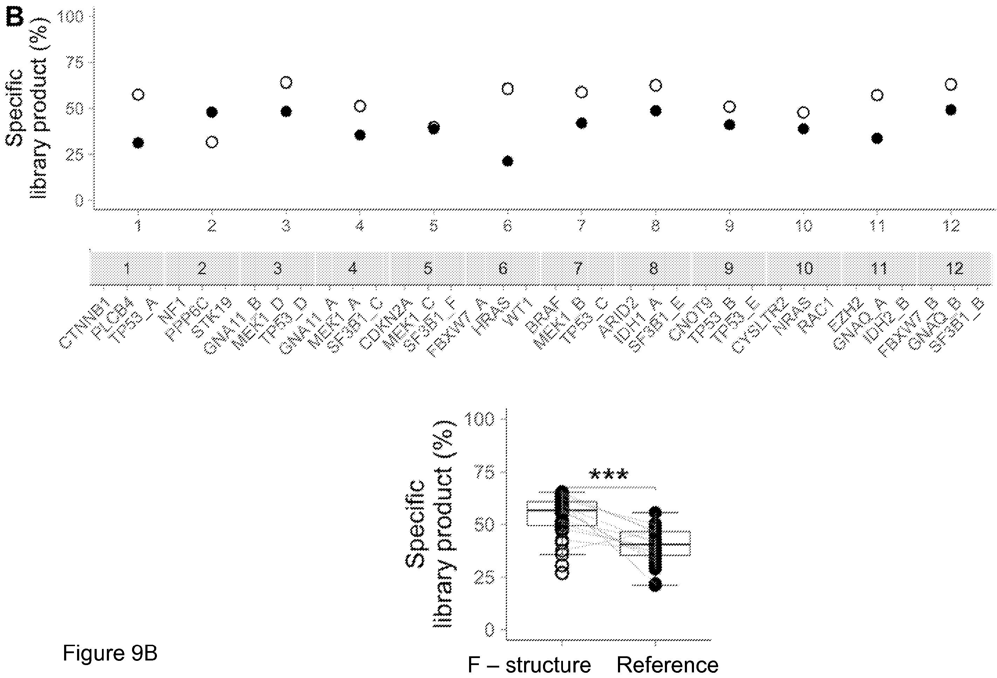

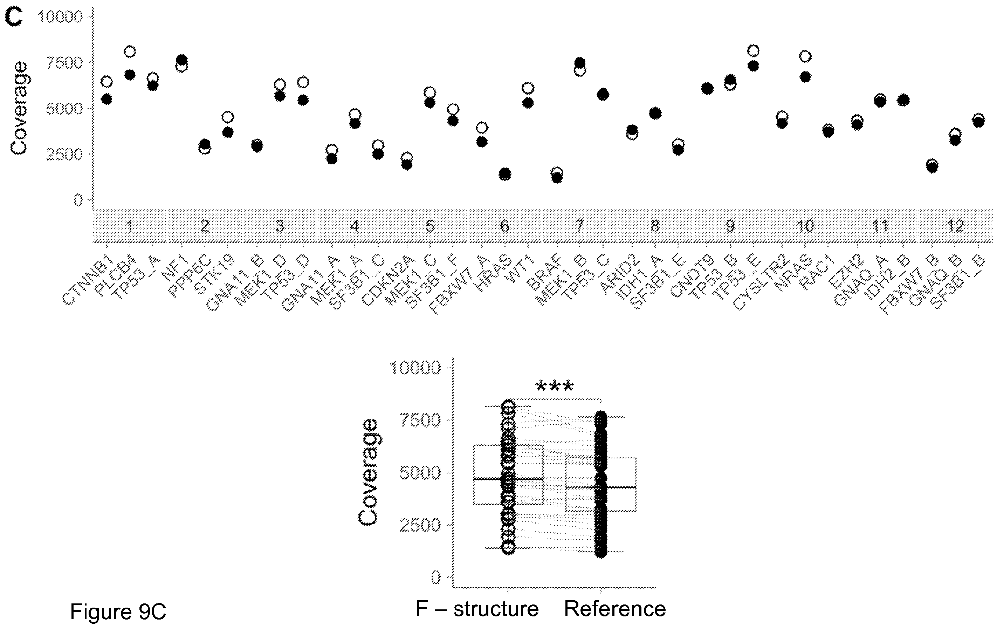

Figure 9. Evaluation of 12 different 3-plexes. (A) Relative specificity using quantitative PCR. Twelve 3-plexes were analyzed using UMI design (F) and reference UMI. Mean value for each assay is shown, n =3. Box plot of all data is shown to the right. *** $p \leq 0.001$, paired Student's t-test. (B) Specificity in correct library products using capillary gel electrophoresis. Twelve 3-plexes were analyzed using UMI design (F) and reference UMI. Mean value for each assay is shown, n = 3. Box plot of all data is shown to the right. *** $p \leq 0.001$, paired Student's t-test. (C) On-target amplification assessed by digital sequencing. Number of consensus reads are shown for each assay in respective 3-plex using UMI design (F) and reference UMI. Mean value for each assay is shown, n = 3. Box plot of all data is shown to the right. *** $p \leq 0.001$, paired Student's t-test.

Figure 10. Performance of 16-plex. The coverage calculated as consensus reads is shown for all individual assays. Mean coverage is shown for each assay, n = 3. Box plot of all data is shown to the right. * $p \leq 0.05$, paired Student's t-test.

Figure 11. Library yield determined with capillary gel electrophoresis. The electropherograms for UMI design (F) and reference UMI are shown, n = 1.

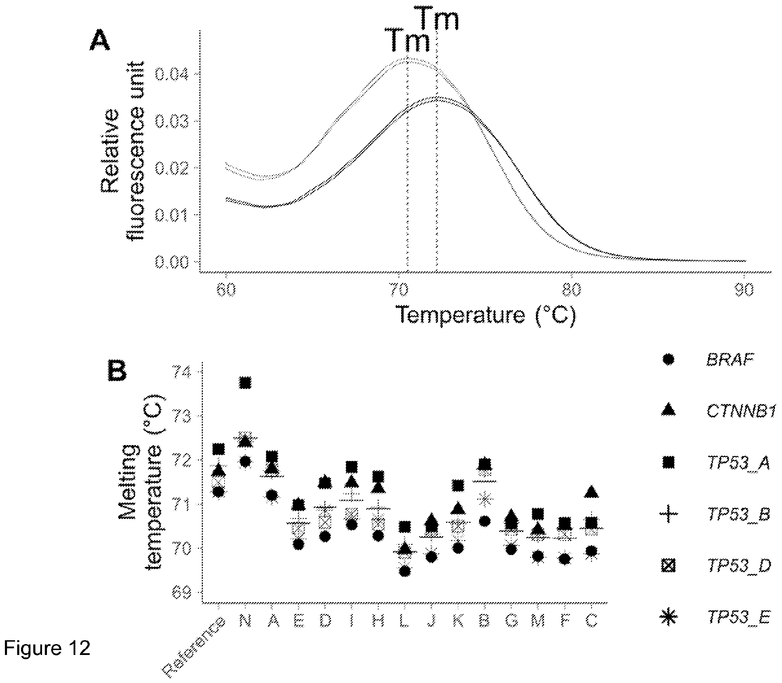

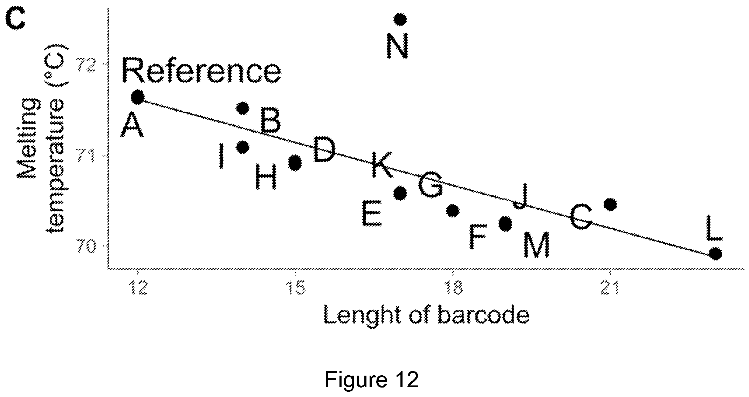

Figure 12. Melting curve analysis. (A) Melting curves shown for SiMSen-Seq forward primers with UMI design (F) and reference UMI in red and black, respectively. The dotted vertical lines indicate melting temperatures (Tm) of respective SiMSen-Seq primer stem-loop structure. n = 3. (B) Melting temperatures of the stem structures for all structured UMIs (A-N) and reference UMI is shown for six assays with mean indicated by a bar. Mean melting temperature is shown for each assay and UMI design, n = 3. (C) Melting temperature versus nucleotide length of loop structure. The spearman correlation coefficient ($\rho$) was - 0.86, p < 0.001. The linear fit is to guide the eye.

Figure 13. Comparison of assay performance. The coverage of individual assays in 3-plexes is correlated to (A-B) the performance of single-plexes assessed with quantitative PCR and (C) the performance of single-plexes assessed with capillary gel electrophoresis. Cq (PTC) is the cycle of quantification value for samples with 20 ng DNA. The spearman correlation coefficient ($\rho$) is calculated for UMI design (F) (n = 30), reference UMI (n =30) and all together (n = 60). The linear fit is to guide the eye.

Figure 14. Dynamic range of 3-plex 6 and UMI design (F). Linear regression is shown for each assay and consensus reads are used as coverage, n = 3.

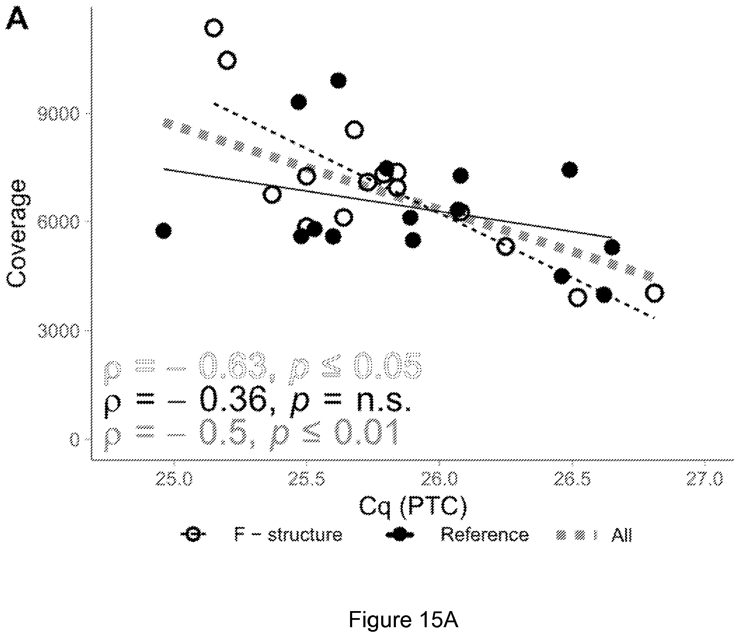

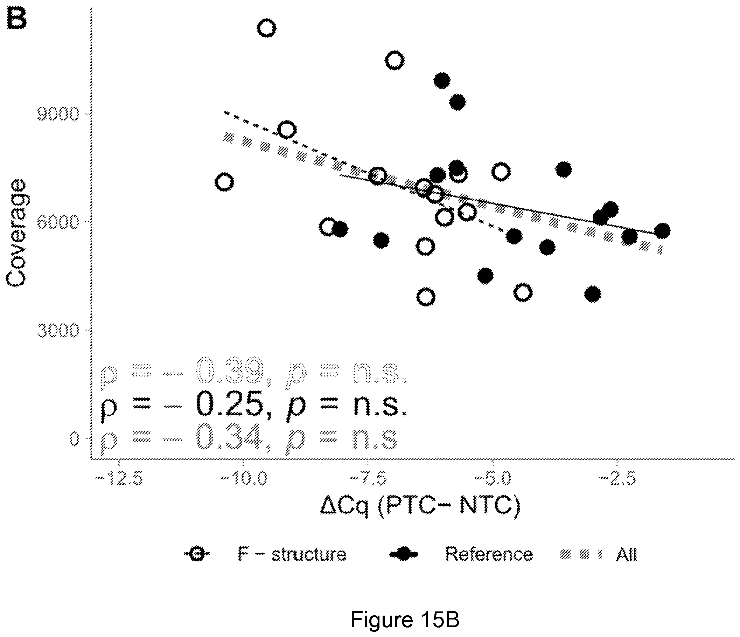

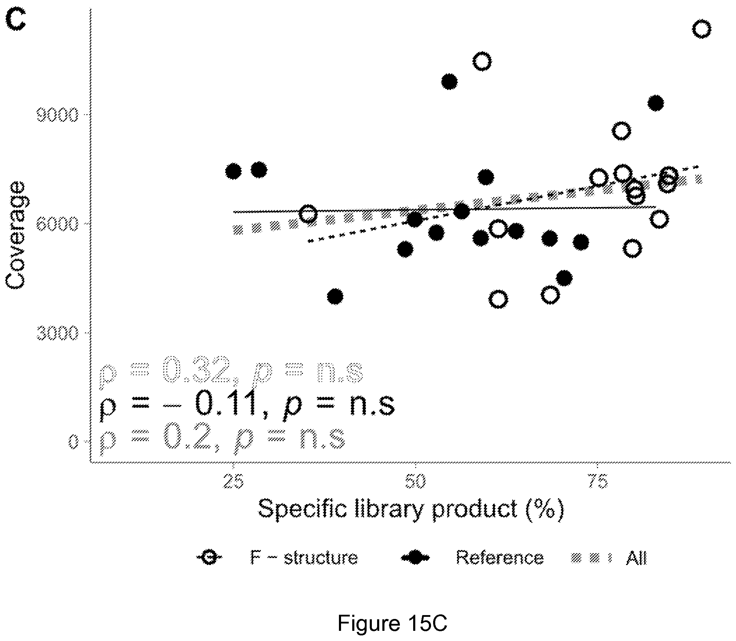

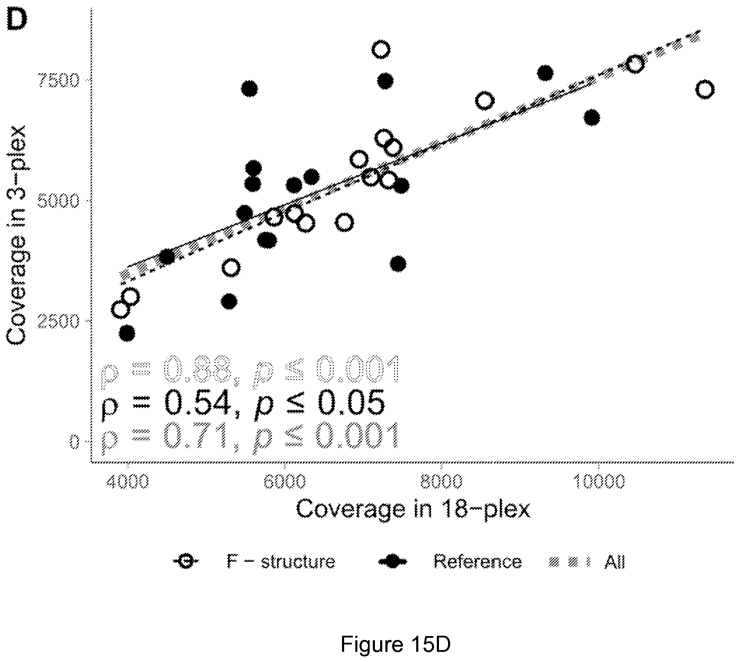

Figure 15. Comparison of assay performance. The coverage of individual assays in 16-plexes is correlated to (A-B) the performance of single-plexes assessed with quantitative PCR and (C) the performance of single-plexes assessed with capillary gel electrophoresis. Cq (PTC) is the cycle of quantification value for samples with 20 ng DNA. The spearman correlation coefficient ($\rho$) is calculated for UMI design (F) (n = 15), reference UMI (n = 15) and all together (n = 30). The linear fit is to guide the eye. (D) Coverages of individual assays in 3 plexes versus 16-plex, n = 16 and n = 32 for all assays.

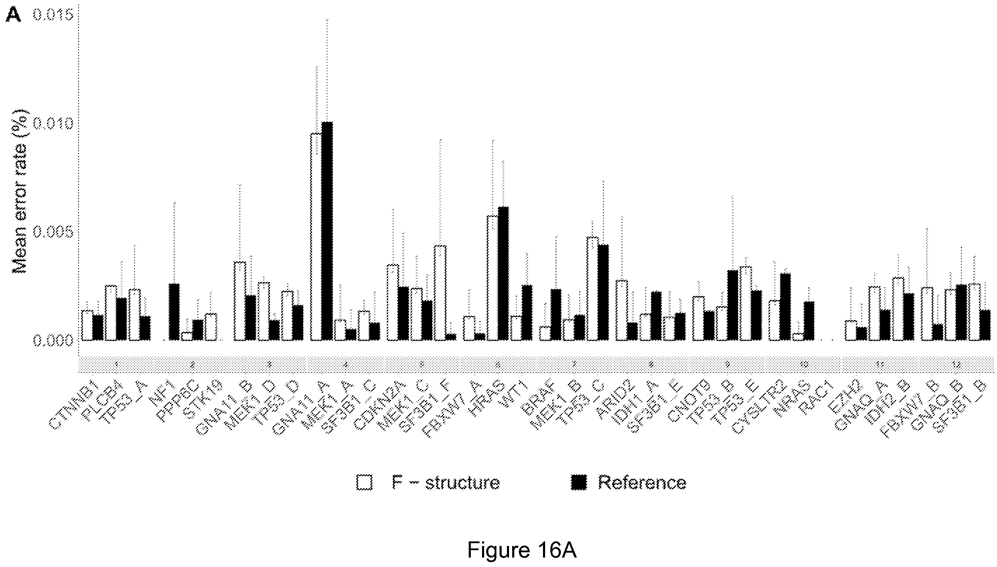

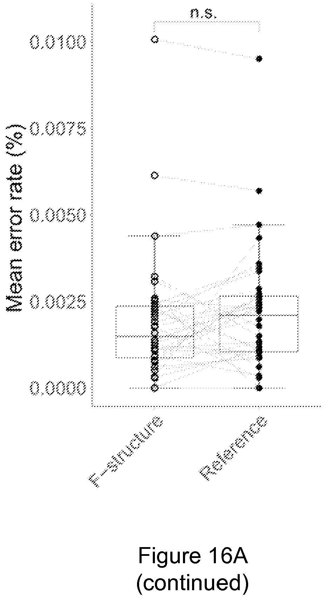

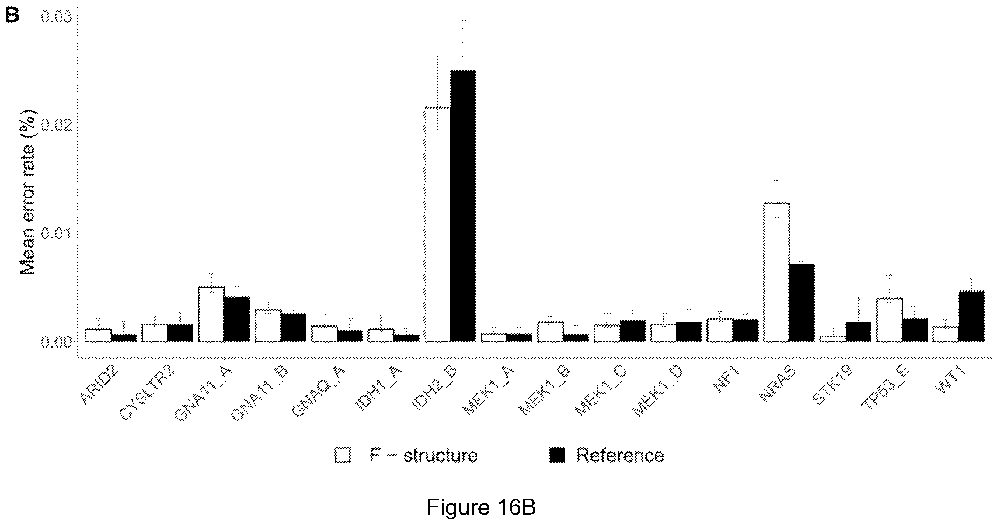

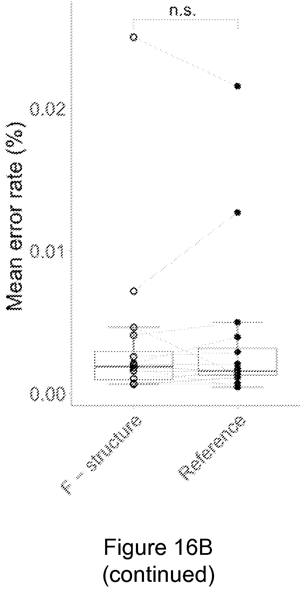

Figure 16. Evaluation of background error rates. (A) Sequencing error rate for 3-plexes. The error rate was calculated as the total number of non-reference alleles per nucleotide position divided by coverage for each nucleotide position. Then the average of all nucleotide positions was calculated per amplicon. Mean $\pm$ SD is shown, n = 3. Nucleotide positions corresponding to known single nucleotide positions (rs788018, rs4685 and rs1800372) were omitted for background noise visualization purposes. Box plot of all data is shown to the right. Paired Student's t-test. n.s., non-significant. (B) Sequencing error rate for 16-plex. Mean $\pm$ SD is shown, n = 3. Box plot of all data is shown to the right. Paired Student's t-test. n.s., non-significant.

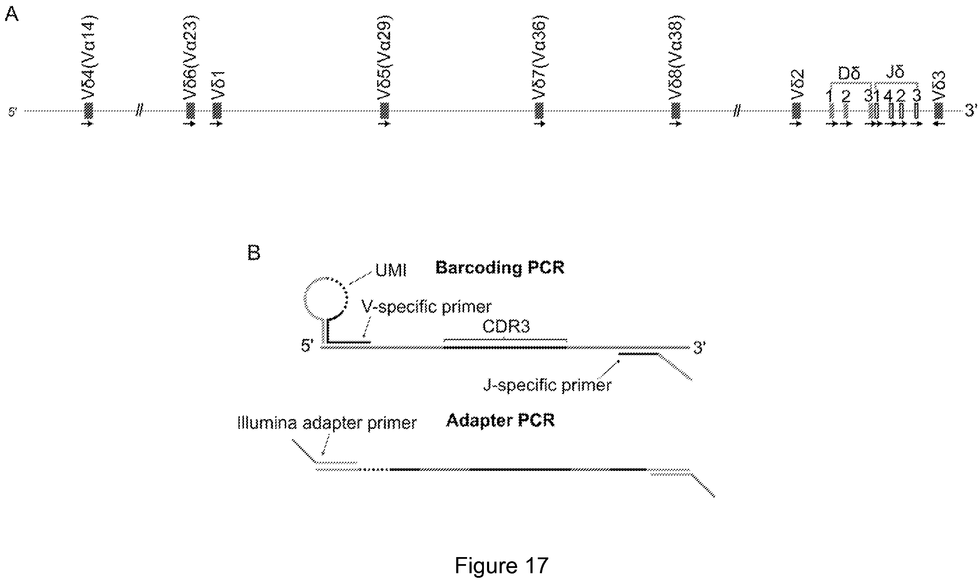

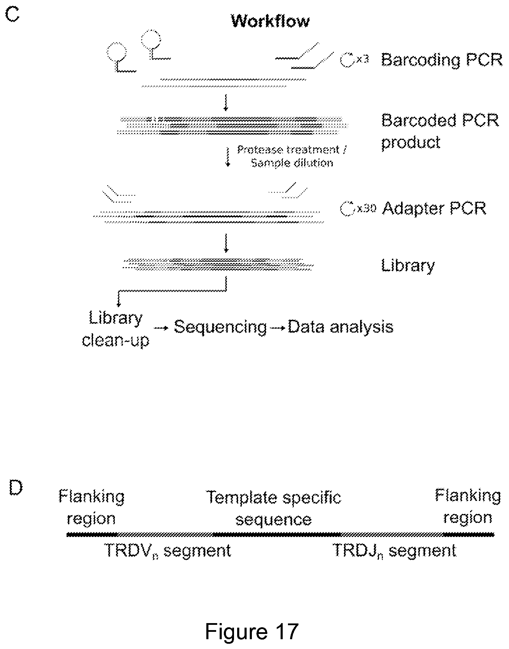

Figure 17. Illustration of $\delta$ TCR repertoire sequencing. (A) Schematic overview of the unrearranged TCR $\delta$ (TRD) locus, arrows show transcription direction. TRD variable 4 (TRDV4) to TRDV8 are shared between the TCR $\alpha$ (TRA) and TRD locus (see Table 1). (B) Amplification consists of two rounds of PCR. In the first barcoding PCR, target primers bind to the V and J genes, generating specific PCR products with UMIs and flanked adapter sequences. In the second adapter PCR, barcoded DNA is amplified with ILLUMINA® adapter primers. (C) The experimental workflow for VDJ-sequencing. (D) Design of synthetic reference molecules used for assay validation. The 32 gBlock molecules contain a TRDV segment consisting of the 97-163 last base pairs of the TRDV gene, and a 48-59 base pairs long TRD joining (TRDJ) segment containing the complete TRDJ gene. An internal unique template specific sequence for each primer pair combination is inserted between the TRDV and TRDJ segments. All synthetic gBlock molecules are also flanked by a non-amplified general sequence. All synthetic reference molecules are shown in Table 1.

Figure 18. Illustration of amount of off-target sequence reads. Mean value for each tri-plex is shown, n =3. Box plots of all mean values are shown to the right. ** p $\leq$ 0.01, paired Student's t-test.

Figure 19. Illustration of mean off-target reads for all individual assays, n = 3. *** p $\leq$ 0.001, unpaired Student's t-test.

Figure 20. Illustration of sensitivity to detect mutated DNA molecules.

DETAILED DESCRIPTION

The present embodiments generally relate to nucleic acid amplification and in particular to nucleic acid amplification using barcoded primers.

Unique molecular identifiers (UMIs), or molecular barcodes (MBCs), are short sequences or tags added to DNA fragments in some next-generation sequencing (NGS) library preparation protocols to identify the input DNA molecule. These UMIs are added before the actual PCR amplification, and can be used to reduce errors and quantitative bias introduced by the amplification. UMIs can be added to DNA molecules by either ligation- or PCR-based approaches. Ligation-based UMI approaches require that target DNA is captured before the analysis, otherwise, all genomic DNA will be analyzed. Another limitation is that molecules are lost due to limited ligation efficiency. In comparison, PCR-based UMI approaches are simpler since no capture step is needed. PCR-based methods are potentially also more sensitive since they do not suffer from ineffective capture and ligation steps. However, introduction of UMIs into PCR primers may cause massive formation of non-specific PCR products caused by the random nucleotide sequence of UMIs.

This problem is solved according to the present invention by shielding the UMIs in secondary structures in hairpin barcode forward primers and/or hairpin barcode reverse primers. Hence, in order to minimize the formation of non-specific PCR products, the UMI is protected in the hairpin barcode primer, such as at least partly inside a hairpin loop, which opens and closes its secondary structure in a temperature-dependent manner.

The hairpin barcode primers of the present invention, in addition, comprise structured UMI sequences protected at least partly inside the hairpin loops. Experimental data as presented herein indicates that using structured UMI sequences, such as by distributing the UMI parts at least partly inside one or more hairpin loops, significantly improves the purity of the barcoded PCR products as compared to using hairpin barcode primers with a non-structured UMI sequence with consecutive random nucleotides. Furthermore, the purity of the library as obtained following PCR-based amplification is considerably higher as compared to using non-structured UMI sequences in hairpin barcode primers.

The hairpin barcode primers of the present invention therefore comprises so-called structured UMIs, i.e., UMIs comprising predefined or degenerated nucleotides at specific positions within the random nucleotides of the structured UMIs. As is shown herein, such structured UMIs reduce the formation of non-specific amplification (PCR) products as compared to traditional UMIs only consisting of a sequence of random nucleotides. The presence of predefined or degenerated nucleotides within the structured UMIs reduce the possibility of forming stable internal structures and dimers, which could cause the formation of non-specific PCR products. Furthermore, the structured UMIs of the invention resulted in less off-target reads, which thereby saves sequencing capacity. Accordingly, a library of amplified nucleic acid molecules generated by the invention has a higher quality and is thereby more suitable for sequencing as compared to using primers with traditional non-structured UMIs.

Accordingly, the usage of hairpin barcode primers of the invention enables an improved amplification of a target nucleic acid sequence in a sample.

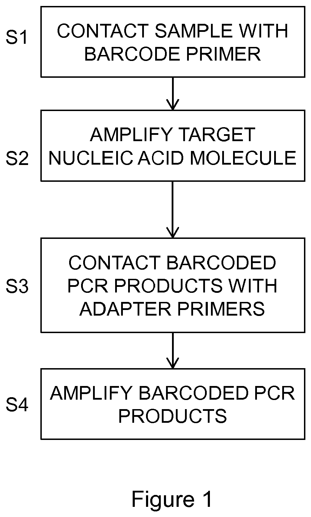

An aspect of the invention relates to a method of amplifying a target nucleic acid molecule in a sample, see Figures 1 and 3A to 3C and 4A to 4C. The method comprises contacting, in step S1, a sample comprising a target nucleic acid molecule 1 with a forward primer 10 and a reverse primers 20.

The forward primer 10 comprises, from a 5' end 10A to a 3' end 10B, an adapter sequence 11 and a target-specific sequence 16. Correspondingly, the reverse primer 20 comprises, from a 5' end 20A to a 3' end 20B, an adapter sequence 21 and a target-specific sequence 26.

According to the invention, the forward primer 10 is a hairpin barcode forward primer 10 and/or the reverse primer 20 is a hairpin barcode reverse primer 20. In such a case, the hairpin barcode forward primer 10 comprises, from the 5' end 10A to the 3' end 10B, the adapter sequence 11, a unique molecular identifier (UMI) sequence 17 comprising a structured UMI 14, and the target-specific sequence 16 complementary to a portion 2 of the target nucleic acid molecule 1. Correspondingly, the hairpin barcode reverse primer 20 comprises, from the 5' end 20A to the 3' end 20B, the adapter sequence 21, a UMI sequence 27 comprising a structured UMI 24, and the target-specific sequence 26 complementary to a portion 3 of the target nucleic acid molecule 1. At least a portion 12, 22 of the adapter sequence 11, 21 of the hairpin barcode forward primer 10 and/or the hairpin barcode reverse primer 20 is complementary to at least a portion 15, 25 of the UMI sequence 17, 27 of the hairpin barcode forward primer 10 and/or the hairpin barcode reverse primer 20. The adapter sequence 11, 21 and the UMI sequence 17, 27 are configured to hybridize to each other at or under a closed annealing temperature and not hybridize to each other at or above an open annealing temperature.

According to the invention, the UMI sequence 17 of the hairpin barcode forward primer 10 comprises a structured UMI 14. Correspondingly, the UMI sequence 27 of the hairpin barcode reverse primer 20 comprises a structured 24. Structured UMI 14, 24 as used herein means that the UMI 14, 24 does not only consist of random nucleotides. In clear contrast, the structured UMI 14, 24 also comprises one or more parts or sequences having predefined nucleotides, i.e., not random nucleotides, and/or degenerated nucleotides, i.e., not fully random nucleotides but restricted in terms of excluding at least one nucleotide.

In the former case, the structured UMI 14, 24 comprises at least one, preferably multiple UMI parts 14A, 14B, 24A, 24B separated by a respective predefined nucleic acid sequence 14C, 24C. This predefined nucleic acid sequence 14C, 24C thereby has a fixed or predefined set of one or more nucleotides, whereas the nucleotide(s) in the UMI part(s) 14A, 14B, 24A, 24B are randomly generated from the set of nucleotides, such as adenine (A), cytosine (C), guanine (G) and thymine (T) for a DNA-based hairpin barcode primer 10, 20 or A, C, G and uracil (U) for an RNA-based barcode primer 10, 20.

In the latter case, one or more degenerated nucleotides are not fully randomized, i.e., cannot be selected among the full set of A, C, G and T or A, C, G and U. Examples of such degenerated nucleotides are weak (W), i.e., A or T; strong (S), i.e., C or G; amino (M), i.e., A or C; keto (K), i.e., G or T; purine (R), i.e., A or G; pyrimidine (Y), i.e., C or T; not A (B), i.e., C, G or T; not C (D), i.e., A, G or T; not G (H), i.e., A, C or T; or not T (V), i.e., A, C or G.

The method as shown in Figure 1 also comprises amplifying, in step S2, the target nucleic acid molecule 1 by performing polymerase chain reaction (PCR) pre-amplification of the target nucleic acid molecule 1 to form a plurality of barcoded PCR products 50. The PCR pre-amplification as performed in step S2 has an annealing temperature equal to or less than the closed annealing temperature of the hairpin barcode forward primer 10 and/or the hairpin barcode reverse primers 20.

The method further comprises contacting, in step S3, the plurality of barcoded PCR products 50 with an adapter-specific forward primer 30 and an adapter-specific reverse primer 40 and amplifying, in step S4, the barcoded PCR products 50 by performing PCR amplification on the barcoded PCR products 50 to form amplified barcoded PCR products 60. In an embodiment, at least a portion of cycles of the PCR amplification has an annealing temperature greater than or equal to the open annealing temperature of the hairpin barcode forward primers 10 and/or the hairpin barcode reverse primer 20.

The amplifying method of the invention as shown in Figure 1 could be used to amplify any target nucleic acid molecule in any sample comprising such nucleic acid molecules. The amplifying method is in particular suitable for amplifying nucleic acid molecules in complex samples comprising a mixture of a plurality of different nucleic acid molecules.

Illustrative, but non-limiting, examples of such samples are biological samples comprising nucleic acid molecules, such as cellular samples, tissue samples, or body fluid samples. Alternatively, the sample could be a sample comprising nucleic acid molecules purified or isolated from such a cellular, tissue, or body fluid sample. The sample can then be an animal sample, such a mammalian sample, and preferably a human sample. Particular examples of samples include plasma sample, serum sample, sputum sample, skin sample, spinal fluid sample, lymph fluid sample, synovial fluid sample, urine sample, tear sample, blood cells sample, tissue sample from an organ or a tumor, whole blood sample, bone marrow sample, amniotic fluid sample, hair sample, semen sample, anal secretions sample, vaginal secretions sample, perspiration sample, saliva sample, and buccal swabs sample. The sample could alternatively be a sample obtained from an *in vitro* cell culture. Other examples include microbial or microorganism samples, such as bacterial samples, virus samples, yeast samples, etc. Also environmental samples could be used including, but not limited to, water samples, soil samples, waste samples, etc.

The target nucleic acid molecule could be an RNA molecule or DNA molecule, including a complementary DNA (cDNA) molecule and modified RNA and DNA molecules, such containing nucleic acid analogues, preferably a DNA molecule.

The amplifying method of the invention can be used in various applications. An illustrative example of such an application is detection of cell-free DNA (cfDNA) in liquid biopsies. Such applications offer great potential for use in non-invasive prenatal testing and as a cancer biomarker. Fetal and tumor DNA fractions, however, can be extremely low in such liquid biopsy samples and ultra-sensitive methods are required for their detection. Hairpin barcode primer with a structured UMI can then be used to generate consensus reads for each original DNA molecule in the liquid biopsy sample, reduce background sequencing noise and allow detection of variant alleles below 0.1% frequency in clonal cell line DNA and in cell-free plasma DNA. The amplifying method of the invention can therefore be used to enable sensitive mutation detection in liquid biopsies. For instance, hairpin barcode primers with structured UMIs can be used instead of the barcode primers disclosed in Nucleic Acids Research (2016) 44(11): e105.

The amplifying method of the invention can also be used in detection of extremely rare variant alleles within a complex mixture of DNA molecules, such as the detection of circulating tumor DNA in the plasma of cancer patients. Barcoding of DNA template molecules early in NGS library construction provides a way to identify and bioinformatically remove polymerase errors that otherwise make detection of these rare variants very difficult. Hairpin barcode primers with structured UMIs can be used to generate targeted barcoded libraries with minimal DNA input, flexible target selection and a very simple, short library construction protocol. For instance, hairpin barcode primers with structured UMIs can be used instead of the barcode primers disclosed in Nature Protocols (2017) 12(4): 664-682.

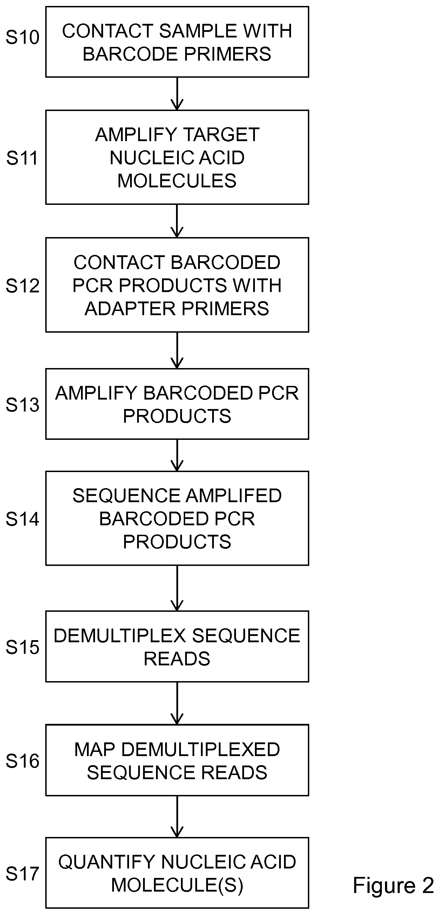

Another aspect of the invention relates to a method for quantifying nucleic acid molecules, see Figures 2, 3A to 3C and 4A to 4C. The method comprises contacting, in step S10, a sample comprising nucleic acid molecules 1 with *P* forward primers 10 and Q reverse primers 20.

In an embodiment, *P* is an integer equal to or larger than one and Q is an integer equal to or larger than one. The *P* forward primers 10 comprise, from a 5' end 10A to a 3' end 10B, an adapter sequence 11 and a target-specific sequence 16. Correspondingly, the Q reverse primers 20 comprise, from a 5' end 20A to a 3' end 20B, an adapter sequence 21 and a target-specific sequence 26.

In an embodiment, the *P* forward primers 10 are *P* hairpin barcode forward primers 10 and/or the Q reverse primers 20 are Q hairpin barcode reverse primers 20. In such an embodiment, each hairpin barcode forward primer 10 comprises, from the 5' end 10A to the 3' end 10B, the adapter sequence 11, a UMI sequence 17 comprising a structured UMI 14, and the target-specific sequence 16 complementary to a respective portion 2 of a nucleic acid molecule 1. Correspondingly, each hairpin barcode reverse primer 20 comprises, from the 5' end 20A to the 3' end 20B, the adapter sequence 23, a UMI sequence 27 comprising a structured UMI 24, and the target-specific sequence 26 complementary to a respective portion 3 of a nucleic acid molecule 1. At least a portion 12, 22 of the adapter sequence 11, 21 of the hairpin barcode forward primer 10 and/or the hairpin barcode reverse primer 20 is complementary to at least a portion 15, 25 of the UMI sequence 17, 27 of the hairpin barcode forward primer 10 and/or the hairpin barcode reverse primer 20. The adapter sequence 11, 21 and the UMI sequence 17, 27 are configured to hybridize to each other at or under a closed annealing temperature and not hybridize to each other at or above an open annealing temperature.

The method as shown in Figure 2 also comprises amplifying, in step S11, the nucleic acid molecules 1 by performing PCR pre-amplification of the nucleic acid molecules 1 to form a plurality of barcoded PCR products 50. The PCR pre-amplification as performed in step S11 has an annealing temperature equal to or less than the closed annealing temperature of the hairpin barcode forward primers 10 and/or the hairpin barcode reverse primers 20.

The method further comprises contacting, in step S12, the plurality of barcoded PCR products 50 with an adapter-specific forward primer 30 and an adapter-specific reverse primer 40 and amplifying, in step S13, the barcoded PCR products 50 by performing PCR amplification on the barcoded PCR products 50 to form a library of amplified barcoded PCR products 60. In an embodiment, at least a portion of cycles of the PCR amplification has an annealing temperature greater than or equal to the open annealing temperature of the hairpin barcode forward primers 10 and/or the hairpin barcode reverse primers 20.

The method also comprises sequencing, in step S14, at least a respective portion of the amplified barcoded PCR products 60 to form respective sequence reads comprising the UMI(s) 64 and nucleic acid molecule sequence(s) 66. The method further comprises demultiplexing, in step S15, the sequence reads based on nucleic acid sequences of the UMIs 64 and mapping, in step S16, the demultiplexed sequence reads to respective known nucleic acid molecule sequences based on nucleic acid sequences of the nucleic acid molecule sequence(s) 66. The method also comprises quantifying, in step S17, unique nucleic acid molecules 1 based on the demultiplexed and mapped sequence reads.

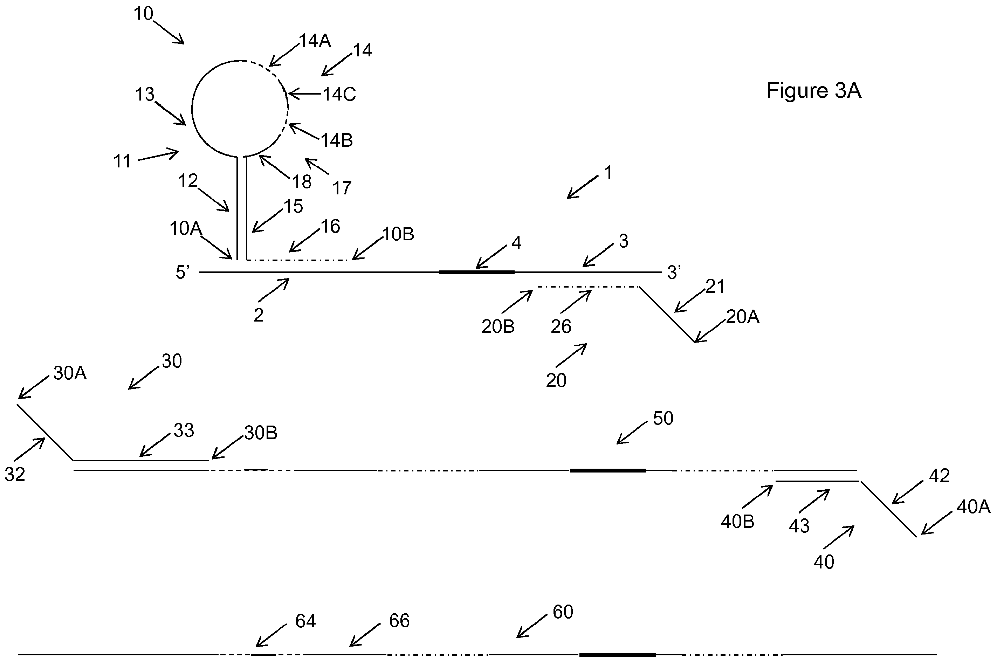

In an embodiment, the forward primer 10 used in Figure 1 is a hairpin barcode primer 10, whereas the reverse primer 20 does not form any hairpin or loop structure as shown in Figure 3A. In such an embodiment, step S1 comprises contacting the sample comprising the target nucleic acid molecule 1 with a hairpin barcode forward primer 10 and a reverse primer 20. The hairpin barcode forward primer 10 then comprises, from the 5' end 10A to the 3' end 10B, the adapter sequence 11, the UMI sequence 17, and the target-specific sequence 16. The at least one reverse primer 20 comprises, from the 5' end 20A to the 3' end 20B, the adapter sequence 21 and the target-specific sequence 26.

Correspondingly, in an embodiment, the *P* forwards primers 10 used in Figure 2 are *P* hairpin barcode forward primers 10, whereas the *Q* reverse primers 20 do not form any hairpin or loop structure as shown in Figure 3A. In such an embodiment, step S10 comprises contacting the sample comprising nucleic acid molecules 1 with a set of *P*>1 hairpin barcode forward primers 10 and at least one reverse primer 20. Each hairpin barcode forward primer 10 of the set comprises, from the 5' end 10A to the 3' end 10B, the adapter sequence 11, the UMI sequence 17, and the target-specific sequence 16 complementary to a respective portion 2 of a nucleic acid molecule 1. The at least one reverse primer 20 comprises, from the 5' end 20A to the 3' end 20B, the adapter sequence 21 and the target-specific sequence 26.

In an embodiment, all hairpin barcode forward primers 10 comprise the same adapter sequence 11. However, the hairpin barcode forward primers 10 comprise different UMI sequences 17 comprising different structured UMIs 14. The set may comprise hairpin barcode forward primers 10 all having the same target-specific sequence 16 (but different structured UMIs 14), multiple, i.e., at least two, hairpin barcode forward primers 10 having the same target-specific sequence 16 or the hairpin barcode forward primers 10 in the set may have different target-specific sequences 16.

It is, however, also possible to use a mixture of hairpin barcode forward primers 10 that also have different adapter sequences 11. For instance, a first set of hairpin barcode forward primers 10 all have the same adapter sequence 11 but different UMI sequences 17. This first set of hairpin barcode forward primers 10 could have the same or different target-specific sequence 16. This first set of hairpin barcode forward primers 10 is then mixed with at least a second set of hairpin barcode forward primers 10 all having the same adapter sequence 11 but different UMI sequences 17. This second set of hairpin barcode forward primers 10 could have the same or different target-specific sequence 16. The adapter sequence 11 of the hairpin barcode forward primers 10 in the second set is, though, different from the adapter sequence 11 of the hairpin barcode forward primers 10 of the first set.

In an embodiment, a mixture of hairpin barcode forward primers 10 that have different designs of UMI sequences 17, such as different designs of the structured UMI 14, could be used. For instance, the length of the structured UMI 14 in terms of number of nucleotide sequences and/or the particular design of the structured UMI 14 in terms of distribution of random nucleotides and predefined nucleotides could differ between different hairpin barcode forward primers 10. Such a mixture of different hairpin barcode forward primers 10 could be useful in case the sample comprises a mixture of different target nucleic acid molecules, which may be present in different in different amounts or levels in the sample, such as few copy number versus high copy number.

An illustrative, but non-limiting, example of a hairpin barcode forward primer 10 that can be used in accordance with the embodiments have the following sequence (SEQ ID NO: 1):

```
5'-

GGACACTCTTTCCCTACACGACGCTCTTCCGATCTNANTNANCNTNTNCNTNANATGGGAAA

GAGTGTCC-target-specific sequence-3'
```

In this illustrative example, the adapter sequence 11 comprises, preferably consists of, the nucleotide sequence GGACACTCTTTCCCTACACGACGCTCTTCCGATCT (SEQ ID NO: 4) that comprises a part (GGACACTCTTTCCC SEQ ID NO: 2), denoted 5' stem sequence 12 herein, which is complementary to and capable of hybridizing to a part (GGGAAAGAGTGTCC (SEQ ID NO: 3), denoted 3' stem sequence 15 herein, of the UMI sequence 17 NANTNANCNTNTNCNTNANATGGGAAAGAGTGTCC (SEQ ID NO: 99). The UMI sequence 17 comprises a structured UMI 14 represented, in this example, by NANTNANCNTNTNCNTNAN (SEQ ID NO: 90) and having alternating random nucleotides (N) 14A, 14B and predefined nucleotides (A, T or C) 14C. The adapter sequence 11 of the hairpin barcode forward primer 10 additionally comprises, in this example, a nucleotide sequence 13 that is not complementary to the UMI sequence 17. This non-complementary sequence 13 may be positioned between the 5' stem sequence 12 and the UMI sequence 17. For instance, a non-complementary sequence 13 of ACACTCTTTCCCTACACGACGCTCTTCCGATCT (SEQ ID NO: 100) could be used in the above illustrated example.

In an embodiment, a single or common reverse primer 20 is used together with the P hairpin barcode forward primers 10 in Figure 2. The common reverse primer 20 comprises a target-specific sequence 26 that is complementary to sequence or region 3 of the nucleic acid molecule 1 that is common for the different nucleic acid molecules.

In another embodiment, multiple different reverse primers 20 are used together with the P hairpin barcode forward primers 10 in Figure 2. Each reverse primer 20 then preferably comprises a respective target-specific sequence 26 that is complementary to a respective sequence or region 3 of the nucleic acid molecule 1 that is specific for a given nucleic acid molecule or specific for a given group of nucleic acid molecules, i.e., is not common for all different nucleic acid molecules.

In an embodiment, all reverse primers 20 comprise the same adapter sequence 26. However, the reverse primers 20 may comprise different target-specific sequences 26.

An illustrative, but non-limiting, example of reverse primers 20 that can be used in accordance with the embodiments have the following sequence (SEQ ID NO: 5):

```
5'-GTGACTGGAGTTCAGACGTGTGCTCTTCCGATCT-target-specific   region-
3'
```

In an embodiment, the adapter sequence 11 of the P hairpin barcode forward primers 10 is different from the adapter sequence 21 of the at least one reverse primer 20.

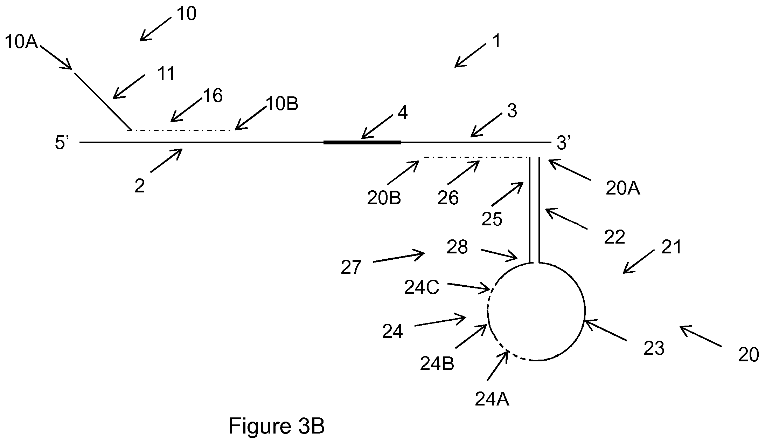

In another embodiment, the reverse primer 20 used in Figure 1 is a hairpin barcode primer 20, whereas the forward primer 10 does not form any hairpin or loop structure as shown in Figure 3B. In such an embodiment, step S1 comprises contacting the sample comprising the target nucleic acid molecule 1 with a forward primer 10 and a hairpin barcode reverse primer 20. The hairpin barcode reverse primer 20 then comprises, from the 5' end 20A to the 3' end 20B, the adapter sequence 21, the UMI sequence 27, and the target-specific sequence 26. The forward primer 10 comprises, from the 5' end 10A to the 3' end 10B, the adapter sequence 11 and the target-specific sequence 16.

Correspondingly, in another embodiment, the Q reverse primers 20 in Figure 2 are Q hairpin barcode reverse primers 20, whereas the P forward primers 10 do not form any hairpin or loop structure as shown in Figure 3B. In such an embodiment, step S10 comprises contacting the sample comprising nucleic acid molecules 1 with a set of Q>1 hairpin barcode reverse primers 20 and at least one forward primer 10. Each hairpin barcode reverse primer 20 of the set comprises, from the 5' end 20A to the 3' end 20B, the adapter sequence 21, the UMI sequence 27, and the target-specific sequence 26 complementary to a respective portion 3 of a nucleic acid molecule. The at least one forward primer 10 comprises, from the 5' end 10A to the 3' end 10B, the adapter sequence 11 and the target-specific sequence 16.

In an embodiment, all Q hairpin barcode reverse primers 20 comprise the same adapter sequence 21. However, the Q hairpin barcode reverse primers 20 comprise different UMI sequences 27 comprising different structured UMIs 24. The set may comprise hairpin barcode reverse primers 20 all having the same target-specific sequence 26 (but different structured UMIs 24), multiple hairpin barcode reverse primers 20 having the same target-specific sequence 26 or the hairpin barcode reverse primers 20 in the set may have different target-specific sequences 26.

It is, however, also possible to use a mixture of hairpin barcode reverse primers 20 that also have different adapter sequences 21. For instance, a first set of hairpin barcode reverse primers 20 all have the same adapter sequence 21 but different UMI sequences 27. This first set of hairpin barcode reverse primers 20 could have the same or different target-specific sequence 26. This first set of hairpin barcode reverse primers 20 is then mixed with at least a second set of hairpin barcode reverse primers 20 all having the same adapter sequence 21 but different UMI sequences 27. This second set of hairpin barcode reverse primers 20 could have the same or different target-specific sequence 26. The adapter sequence 21 of the hairpin barcode reverse primers 20 in the second set is, though, different from the adapter sequence 21 of the hairpin barcode reverse primers 20 of the first set.

In an embodiment, a mixture of hairpin barcode reverse primers 20 that have different designs of UMI sequences 27, such as different designs of the structured UMI 24, could be used. For instance, the length of the structured UMI 24 in terms of number of nucleotide sequences and/or the particular design of the structured UMI 24 in terms of distribution of random nucleotides and predefined nucleotides could differ between different hairpin barcode reverse primers 20. Such a mixture of different hairpin barcode reverse primers 20 could be useful in case the sample comprises a mixture of different target nucleic acid molecules, which may be present in different in different amounts or levels in the sample, such as few copy number versus high copy number.

In an embodiment, a single or common forward primer 10 is used together with the Q hairpin barcode reverse primers 20 in Figure 2. The common forward primer 10 comprises target-specific sequence 16 that is complementary to sequence or region 2 of the nucleic acid molecule 1 that is common for the different nucleic acid molecules 1.

In another embodiment, multiple different forward primers 10 are used together with the Q hairpin barcode reverse primers 20 in Figure 2. Each forward primer 10 then preferably comprises a respective target-specific sequence 16 that is complementary to a respective sequence or region 2 of the nucleic acid molecule 1 that is specific for a given nucleic acid molecule or specific for a given group of nucleic acid molecules, i.e., is not common for all different nucleic acid molecules.

In an embodiment, all forward primers 10 comprise the same adapter sequence 11. However, the forward primers 10 may comprise different target-specific sequences 16.

In an embodiment, the adapter sequence 21 of the hairpin barcode reverse primers 20 is different from the adapter sequence 11 of the at least one forward primer 10.

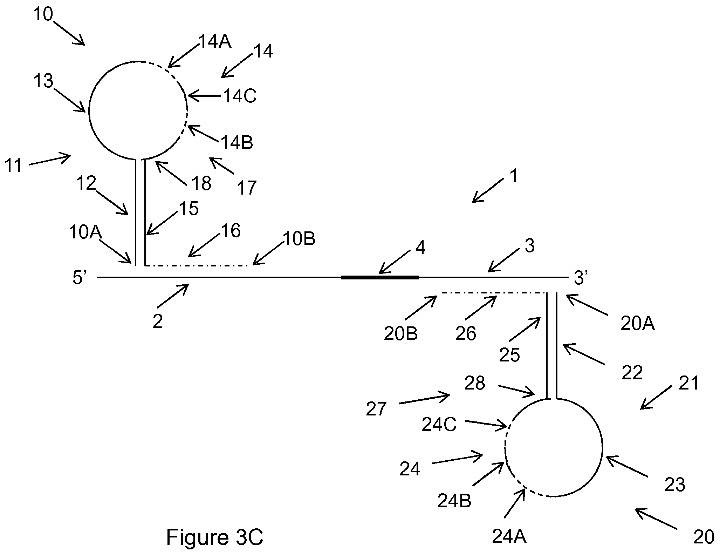

In a further embodiment, both the forward primer 10 and the reverse primer 20 used in Figure 1 are hairpin barcoded primers as shown in Figure 3C. In such an embodiment, step S1 comprises contacting the sample comprising the target nucleic acid molecule 1 with a hairpin barcode forward primer 10 and a hairpin barcode reverse primer 20. The hairpin barcode forward primer 10 then comprises, from the 5' end 10A to the 3' end 10B, the adapter sequence 11, a UMI sequence 17 comprising a first structured UMI 14, and the target-specific sequence 16 and the hairpin barcode reverse primer 20 comprises, from the 5' end 20A to the 3' end 20B, the adapter sequence 21, a UMI sequence 27 comprising a second structured UMI 24, and the target-specific sequence 26.

Correspondingly, in a further embodiment, both the P forward primers 10 and the Q reverse primers 20 used in Figure 2 are hairpin barcoded primers as shown in Figure 3C. In such an embodiment, step S10 comprises contacting the sample comprising nucleic acid molecules 1 with a first set of P>1 hairpin barcode forward primers 10 and a second set of Q>1 hairpin barcode reverse primers 20. Each hairpin barcode forward primer 10 of the first set comprises, from the 5' end 10A to the 3' end 10B, the adapter sequence 11, a UMI sequence 17 comprising a first structured UMI 14, and the target-specific sequence 16 complementary to a respective portion 2 of a nucleic acid molecule 1 and each hairpin barcode reverse primer 20 of the second set comprises, from the 5' end 20A to the 3' end 270B, the adapter sequence 21, a UMI sequence comprising a second structured UMI 24, and the target-specific sequence 26 complementary to a respective portion 3 of a nucleic acid molecule 1.

In an embodiment, all hairpin barcode forward primers 10 of the first set comprise the same adapter sequence 11. However, the P hairpin barcode forward primers 10 comprise different UMI sequences 17 comprising different structured UMIs 14.

In an embodiment, all hairpin barcode reverse primers 20 of the second set comprise the same adapter sequence 21. However, the Q hairpin barcode reverse primers 20 comprise different UMI sequences 17 comprising different structured UMIs 24.

In an embodiment, the adapter sequences 11, 21 comprises a 5' stem sequence 12, 22 and the UMI sequences 17, 27 comprises a 3' stem sequence 15, 25. In such an embodiment, the 5' stem sequence 12 of the P hairpin barcode forward primers 10 is the same as the 5' stem sequence 22 of the Q hairpin barcode reverse primers 20. In another embodiment, the 5' stem sequence 12 of the P hairpin barcode forward primers 10 is different from the 5' stem sequence 22 of the Q hairpin barcode reverse primers 20. Correspondingly, the 3' stem sequence 15 of the P hairpin barcode forward primers 10 could be the same as or different from the 3' stem sequence 25 of the Q hairpin barcode reverse primers 20. However, the adapter sequence 11 of the P hairpin barcode forward primers 10 is preferably different from the adapter sequence 21 of the Q hairpin barcode reverse primers 20.

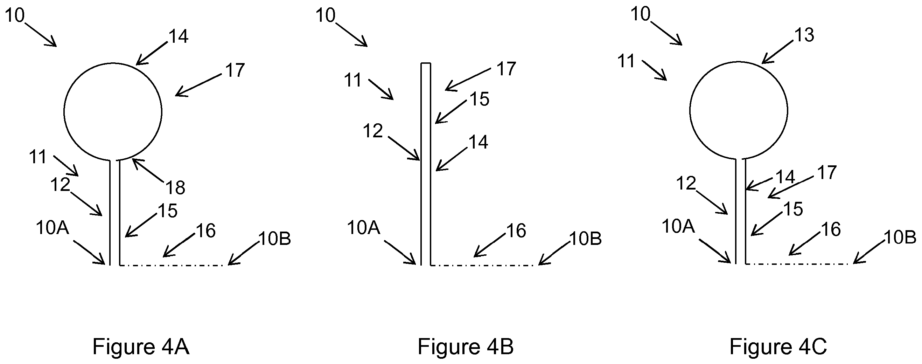

The hairpin barcode forward primer(s) 10 and/or the hairpin barcode reverse primer(s) 20 form a hairpin structure shielding the structured UMI 14, 24, in an embodiment, inside the hairpin loop as shown in Figures 3A to 3C, 4A to 4C at a temperature equal to or below the closed annealing temperature. Hence, equal to or below this closed annealing temperature at least a portion 12, 22 of the adapter sequence 11, 21 hybridizes to at least a portion 15, 25 of the UMI sequence 17, 27. Correspondingly, when the temperature increases to or above the open annealing temperature, the least a portion 12, 22 of the adapter sequence 11, 21 no longer hybridizes to the at least a portion 15, 25 of the UMI sequence 17, 27.

The length of the respective portions 12, 22, 15, 25 of the sequences 12, 22, 15, 25 of the hairpin barcode forward primer(s) 10 and/or the hairpin barcode reverse primer(s) 20 that are configured to hybridize to each other define at least partly the closed and open annealing temperatures. Generally, the longer the hybridizing portions of the adapter sequences 11, 21 and the UMI sequences 17, 27, i.e., the higher number of nucleotides in these hybridizing portions, the higher the closed and open annealing temperatures. The closed and open annealing temperatures are not only defined based on the length of the hybridizing portions but also by the particular combination of nucleotides in the hybridizing portions. For instance, hybridizing portions having a higher GC content (G-C and C-G base pairing) have generally a higher temperature of melting (Tm) and thereby a higher closed and opened annealing temperature as compared to hybridizing portions of the same length but lower GC content, i.e., a higher AT content (AT and T-A base pairing). Also modified nucleotides and nucleic acid analogues, for instance, peptide nucleic acid (PNA), locked nucleic acid (LNA), glycol nucleic acid (GNA), threose nucleic acid (TNA) and Zip nucleic acid (ZNA), present in the adapter sequence 11, 21 and/or the UMI sequence 17, 27 could be used to modify the closed and open annealing temperature. Such modified nucleotides and nucleic acid analogues could then be used to define and tailor the closed and open annealing temperatures of the hairpin barcode forward primers 10 and/or the hairpin barcode reverse primers 20. For instance, LNA generally increases the Tm of two complementary sequences as compared to the naturally occurring nucleotides A, T, G and C. Hence, introducing one or more of LNAs in the adapter sequence 11, 21 and/or the UMI sequence 17, 27 could be used to achieve a target closed or open annealing temperature but using shorter hybridizing portions of the adapter sequences 11, 21 and shorter hybridizing portions of the UMI sequences 17, 27 as compared to when using only naturally occurring nucleotides.

In a particular embodiment, the hybridizing portions of the adapter sequence 11, 21 and of the UMI sequences 17, 27, such as the 5' stem sequence 12, 22 and the 3' stem sequence 15, 25, comprises 5-15 nucleotides, preferably 8-15 nucleotides, and more preferably 12-15 nucleotides.

In an embodiment, the hairpin barcode forward primer(s) 10 comprises, from the 5' end 10A to the 3' end 10B, the adapter sequence 11, the UMI sequence 17 comprising the structured UMI 14 and a stem sequence 15, and the target-specific sequence 16. Correspondingly, the hairpin barcode reverse primer(s) 20 comprises, from the 5' end 20A to the 3' end 20B, the adapter sequence 21, the UMI sequence 27 comprising the structured UMI 24 and a stem sequence 25, and the target-specific sequence 26. In this embodiment, at least a portion 12, 22 of the adapter sequence 11, 21 of the hairpin barcode forward primer 10 and/or the hairpin barcode reverse primer 20 is complementary to at least a portion of the stem sequence 15, 25 of the hairpin barcode forward primer 10 and/or the hairpin barcode reverse primer 20. The adapter sequence 11, 21 and the stem sequence 15, 25 are configured to hybridize to each other at or under the closed annealing temperature and not hybridize to each other at or above the open annealing temperature.

In a particular embodiment, the hairpin barcode forward primer(s) 10 comprises, from the 5' end 10A to the 3' end 10B, the adapter sequence 11 comprising a 5' stem sequence 12, the UMI sequence 17 comprising the structured UMI 14 and a 3' stem sequence 15, and the target-specific sequence 16. In a particular embodiment, the barcode reverse primer(s) 20 comprises, from the 5' end 20A to the 3' end 20B, the adapter sequence 21 comprising a 5' stem sequence 22, the UMI sequence 27 comprising the structured UMI 24 and a 3' stem sequence 25, and the target-specific sequence 26. In these embodiments, at least a portion of the 5' stem sequence 12, 22 of the hairpin barcode forward primer 10 and/or the hairpin barcode reverse primer 20 is complementary to at least a portion of the 3' stem sequence 15, 25 of the hairpin barcode forward primer 10 and/or the hairpin barcode reverse primer 20. The 5' stem sequence 12, 22 and the 3' stem sequence 15, 25 are configured to hybridize to each other at or under the closed annealing temperature and not hybridize to each other at or above the open annealing temperature.

Figures 3A to 3C illustrate an embodiment where the hairpin barcode forward/reverse primer(s) 10, 20 comprises an adapter sequence 11, 21 comprising the 5' stem sequence 12, 22 and a non-complementary sequence 13, 23 and the UMI sequence 17, 27 comprises the structured UMI 14, 24 and the 3' stem sequence 15, 25. The 5' and 3' stem sequences 12, 22, 15, 25 are then configured to hybridize to each other at or under the closed annealing temperature to form a loop comprising the non-complementary sequence 13, 23 and the structured UMI 14, 24. The structured UMI 14, 24 is thereby protected inside the loop of the hairpin barcode forward/reverse primer(s) 10, 20.

Figures 4A to 4C illustrate other embodiments of a hairpin barcode forward primer 10. These embodiments also apply to a corresponding hairpin barcode reverse primer 20. In Figure 4A, the adapter sequence 11 does not comprise any non-complementary sequence but merely the so-called 5' stem sequence 12. This means that when this 5' stem sequence 12 is hybridized to the 3' stem sequence 15 of the UMI sequence 17 at or under the closed annealing temperature the formed loop mainly consists of the structured UMI 14.

Figure 4B illustrates another embodiment of a hairpin barcode forward primer 10. In this embodiment, the hybridizing portion 15 of the UMI sequence 17 is overlapping with the structured UMI 14. This means that at least a portion of the structured UMI 14 is complementary to at least portion 12 of the adapter sequence 11 to thereby hybridize to each other as shown in Figure 4B at or under the closed annealing temperature. This is possible since the UMI 14 is a structured UMI 14 comprising predefined nucleotide sequences. The adapter sequence 11 can then be generated to have complementary nucleotides at corresponding positions to base pair with the predefined nucleotide sequences of the structured UMI 14. It is also possible to generate a nucleotide sequence in the adapter sequence 11 that is fully complementary to the nucleotides in the structured UMI 14 enzymatically, such as using a polymerase with the structured UMI 14 as template. In such a case, this portion 12 of the adapter sequence 11 can then be made complementary to the complete structured UMI 14 including both the random and predefined nucleotides of the structured UMI 14.

Figure 4B also illustrates that the hairpin barcode forward primer 10 does not necessary have to form any loop. In clear contrast, the adapter sequence 11 and the UMI sequence 17 could hybridize to each other to protect the structured UMI 14 inside the hybridized part of the hairpin barcode forward primer 10.

Figure 4C illustrates yet another embodiment of the hairpin barcode forward primer 10. In this embodiment, the adapter sequence 11 comprises the 5' stem sequence 15 and the non-complementary sequence 13. The UMI sequence 17 comprises the structured UMI 14. In this embodiment, at least a portion of the structured UMI 14 is complementary to at least a portion of the 5' stem sequence 12 so that the structured UMI 14 is hybridized to the 5' stem sequence 12 at or under the closed annealing temperature. In this embodiment, the loop mainly consists of the non-complementary sequence 13 of the adapter sequence 11.

In an embodiment, see Figures 3A to 3C, 4A the UMI sequence 17, 27 comprises at least one destabilizing nucleotide 18, 28, preferably at least two destabilizing nucleotides 18, 28, between the structured UMI 14, 24 and the 3' stem sequence 15, 25. Hence, in an embodiment, the hairpin barcode forward primer(s) 10 and/or the hairpin barcode reverse primer(s) 20 further comprises at least one destabilizing nucleotide 18, 28 between the structured UMI 14, 24 and the 3' stem sequence 15, 25. In a particular embodiment, the hairpin barcode forward primer(s) 10 and/or the hairpin barcode reverse primer(s) 20 comprise at least two destabilizing nucleotides 18, 28, between the structured UMI 14, 24 and the 3' stem sequence 15, 25. Such destabilizing nucleotide(s) can be incorporated to ensure that the structured UMI 14, 24 itself does not, by random chance, complement the adapter sequence 11, 21 and results in a longer, more stable stem having higher closed and open annealing temperatures.

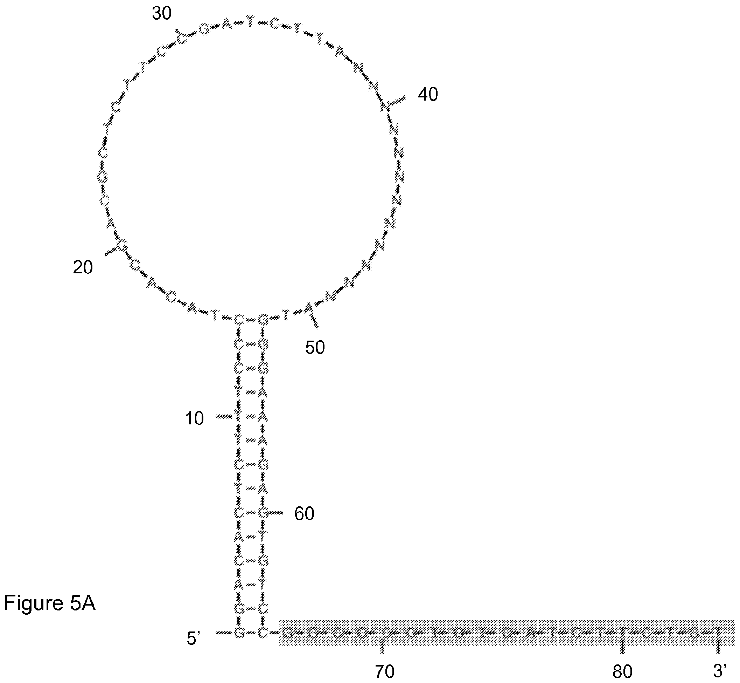

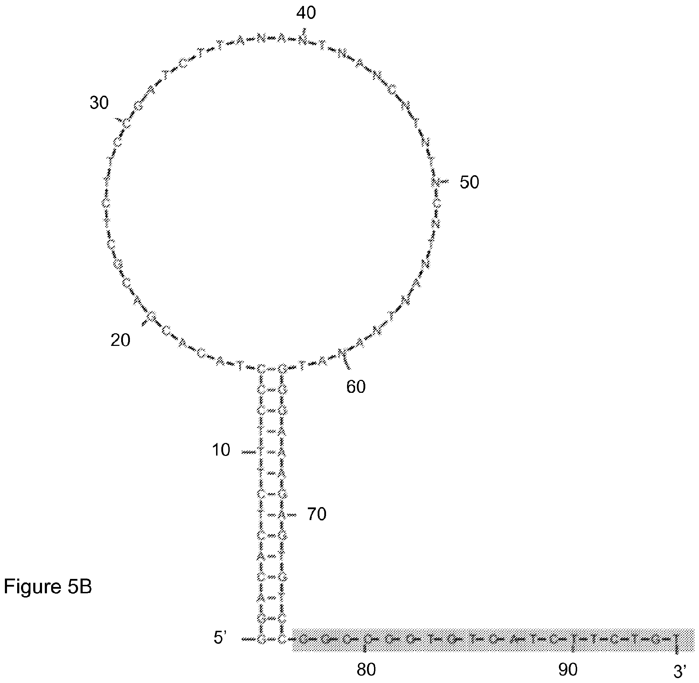

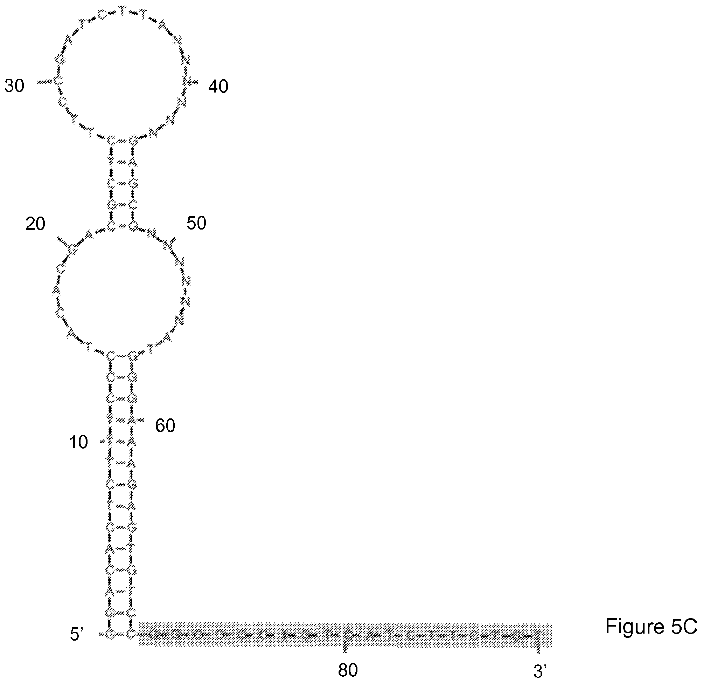

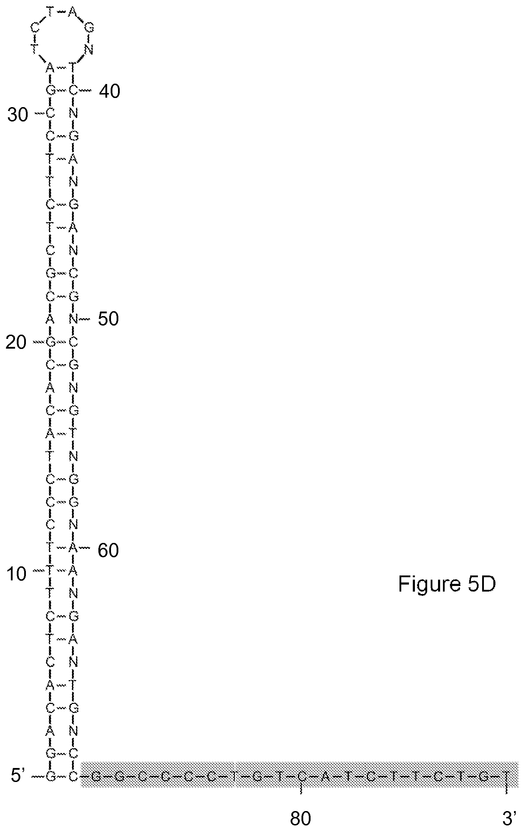

Figure 5A illustrates a standard hairpin barcode primer with a continuous UMI, i.e., not a structured UMI. Figure 5B illustrates an embodiment of a forward hairpin barcode primer with a structured UMI, in which the random nucleotides (N) are distributed over the structured UMI with alternating predefined nucleotides and alternating random nucleotides. Figure 5C illustrates an embodiment of a double-loop forward hairpin barcode primer having a double loop with structured UMI with a first UMI part in a first loop and a second UMI part in a second loop and with predefined nucleotides between the first and second UMI parts. These predefined nucleotides are capable of hybridizing to complementary nucleotides of the adapter sequence. Figure 5D illustrates an embodiment of a forward barcode primer with an integrated barcode partly forming part of the loop and partly forming part of the hybridizing portion of the UMI sequence.

The structured UMI 14, 24 of the hairpin barcode forward primers 10 or of the hairpin barcode reverse primers 20 could include the same number of nucleotides. In such an embodiment, all the structured UMIs 14, 24 have the same length in terms of number of nucleotides. The embodiments are, however, not limited thereto. In other embodiments, a further level of "randomization" of the structured UMIs 14, 24 could be achieved by having different, such as random, number of nucleotides in the structured UMIs 14, 24 in different hairpin barcode forward or reverse primers 10, 20. In the latter case, this could be achieved by (randomly) providing a length of a structured UMI 14, 24 within a predefined interval of available lengths, such as from u nucleotides up to v nucleotides, wherein $u, v$ are positive numbers and $u < v$. In such an embodiment, the different hairpin barcode forward primers 10 and/or hairpin barcode reverse primers 20 have lengths of the structured UMIs 14, 24 within the interval of from $u$ up to $v$ but not all such hairpin barcode primers 10, 20 need to have the same lengths of their respective structured UMIs 14, 24.

In the following various embodiments of the structured UMI 14, 24 are described. In these embodiments, reference is typically made to nucleotides in terms of A, T, C or G. In such embodiments, the hairpin barcode forward/reverse primer(s) 10, 20 are DNA primers. These embodiments, however, also apply to RNA primers, in which case the nucleotides are A, U, C or G. It is also possible to include modified nucleotides and nucleic acid analogues, for instance, PNA, LNA, GNA, TNA and/or ZNA, in the structured UMI 14, 24.

In an embodiment, the structured UMI 14, 24 comprises two UMI parts 14A, 14B, 24A, 24B separated by one predefined nucleotide sequence 14C, 24C. In such a case, the structured UMI 14, 24 could be in the form of $N_1N_2 \cdots N_{k_1}M_1 \cdots M_lN_{k_1+1}N_{k_1+2} \cdots N_{k_2}$, wherein $N_1N_2 \cdots N_{k_1}$ represents the first UMI part 14A, 24A, $N_{k_1+1}N_{k_1+2} \cdots N_{k_2}$ represents the second UMI part 14B, 24B and $M_1 \cdots M_l$ represents the predefined nucleotide sequence 14C, 24C. In such an embodiment, each $N$ is independently a random nucleotide among A, T, C and G, each $M$ is independently a respective predefined nucleotide among A, T, C and G, and $k_1$, $k_2$ and $l$ are positive integers.

In a preferred embodiment, the structured UMI 14, 24 comprises more than two UMI parts 14A, 14B, 24A, 24B separated by a respective predefined nucleotide sequence 14C, 24C. In such an embodiment, the structured UMI 14, 24 comprises alternating UMI parts 14A, 14B, 24A, 24B and predefined nucleotide sequences 14C, 24C.

In an embodiment, each UMI part 14A, 14B, 24A, 24B of the structured UMI 14, 24 comprises a same number of nucleotides. This same number of nucleotides is preferably selected within a range of from one up to eight, preferably from one up to six, and even more preferably from one up to four, such as one, two, three or four.

In another embodiment, at least two UMI parts 14A, 14B, 24A, 24B of the structured UMI 14, 24 comprise or have different number of nucleotides. Hence, in this embodiment, not all UMI parts 14A, 14B, 24A, 24B of a structured UMI 14, 24 have the same length in terms of number of nucleotides. The length of the different UMI parts 14A, 14B, 24A, 24B may be selected within a range of from one up to eight, preferably from one up to six, and even more preferably from one up to four, such as one, two, three or four.

In an embodiment, if the structured UMI 14, 24 comprises multiple predefined nucleotide sequences 14C, 24C they may all have the same number of nucleotides. In another embodiment, at least two predefined nucleotide sequences 14C, 24C of a structured UMI 14, 24 comprise different number of nucleotides and thereby have different lengths.

In an embodiment, the structured UMI 14, 24 comprises multiple UMI parts 14A, 14B, 24A, 24B separated by a respective predefined nucleotide sequence 14C, 24C.

The number of nucleotides in the predefined nucleotide sequence(s) 14C, 24C of a structured UMI 14, 24 could have the same or different number of nucleotides as compared to the number of nucleotides in the UMI parts 14A, 14B, 24A, 24B of the structured UMI 14, 24. In a preferred embodiment, the predefined nucleotide sequence(s) 14C, 24C of a structured UMI 14, 24 has the same or lower number of nucleotides as compared to the UMI parts 14A, 14B, 24A, 24B of the structured UMI 14, 24.

As mentioned in the foregoing, a preferred embodiment of structured UMI 14, 24 comprises alternating UMI parts 14A, 14B, 24A, 24B, each comprising one or more random nucleotides, and respective predefined nucleotide sequences 14C, 24C, each comprising one or more predefined nucleotides.

In an embodiment, the structured UMI 14, 24 is selected from the group consisting of

$$N_1 M_1 N_2 M_2 N_3 M_3 \cdots N_k;$$

$$N_1 M_1 N_2 M_2 N_3 M_3 \cdots N_k M_k;$$

$$N_1 \cdots N_{k_1} M_1 N_{k_1+1} \cdots N_{k_2} M_2 N_{k_2+1} \cdots N_{k_n};$$

$$N_1 \cdots N_{k_1} M_1 N_{k_1+1} \cdots N_{k_2} M_2 N_{k_2+1} \cdots N_{k_n} M_n;$$

$$N_1 \cdots N_{k_1} M_1 \cdots M_{l_1} N_{k_1+1} \cdots N_{k_2} M_{l_1+1} \cdots M_{l_2} \cdots N_{k_{n-1}+1} \cdots N_{k_n};$$

and

$$N_1 \cdots N_{k_1} M_1 \cdots M_{l_1} N_{k_1+1} \cdots N_{k_2} M_{l_1+1} \cdots M_{l_2} \cdots N_{k_{n-1}+1} \cdots N_{k_n} M_{l_{n-1}+1} \cdots M_{l_n}.$$

In this embodiment, each $N$ is independently a random nucleotide among A, T, C and G (or A, U, C and G) and each $M$ is independently a respective predefined nucleotide among A, T, C and G (or A, U, C and G). $k$ is an integer from 3 up to 20, preferably from 4 up to 20, and more preferably from 6 up to 15, each $k_i$, i=1...n is independently an integer from 2 up to 8, preferably from 2 up to 6, and more preferably from 2 up to 4 and each $l_j$, j=1...n is independently an integer from 1 up to 8, preferably from 1 up to 6, and more preferably from 1 up to 4. N is an integer from 3 up to 20, preferably from 4 up to 20, and more preferably from 6 up to 15. It is also possible to use longer structured UMIs 14, 24, such as by having $k$ larger than 20.

It is also possible to provide structured UMI 14, 24 where the random nucleotides are randomly among a subset of the available nucleotides A, T, C and G (or A, U, C and G). For instance, the random nucleotide could be a random nucleotide among three or these available nucleotides A, T, C and G (or A, U, C and G).

In a particular embodiment, each $l_j$ is equal to or lower than each $k_i$.

In an embodiment, the UMI 14, 24 is $N_1 \cdots N_{k_1} M_1 \cdots M_{l_1} N_{k_1+1} \cdots N_{k_2} MN_{k_2+1} \cdots N_{k_3} M_{l_1+1} \cdots M_{l_1} N_{k_3+1} \cdots N_{k_4}$.

In a particular embodiment, each $N$ is independently a random nucleotide among A, T, C and G and each $M$ is a respective predefined nucleotide among A, T, C and G, preferably A. Hence, in this particular embodiment, all predefined nucleotides are the same nucleotide, i.e., all are A, all are T, all are C or all are G, preferably all are A.

In this particular embodiment, the structured UMI 14, 24 consists of four UMI parts separated by a respective sequence of at least one predefined nucleotide. In an embodiment, the first and fourth UMI parts (first and last UMI parts) have the same number of nucleotides, i.e., $k_1 - 1 = k_4 - k_{3+1}$. In an embodiment, the second and third UMI parts (intermediate UMI parts) also have the same number of nucleotides, i.e., $k_2 - k_{1+1} = k_3 - k_{2+1}$. Furthermore, the first and third sequences of at least one predefined nucleotide preferably have the same number of predefined nucleotides, i.e., $l_1 - 1 = l_2 - l_{1+1}$.

In a particular preferred embodiment, the structured UMI 14, 24 is $N_1 N_2 N_3 N_4 M_1 M_2 M_3 N_5 N_6 M_4 N_7 N_8 M_5 M_6 M_7 N_9 N_{10} N_{11} N_{12}$.

In the above-described embodiment, the UMI parts 14A, 14B, 24A, 24B of the structured UMI 14, 24 are exemplified as comparing fully random nucleotides, i.e., N. It is, however, possible that at least one or more of the nucleotides of the UMI parts 14A, 14B, 24A, 24B of the structured UMI 14, 24 is or are degenerated nucleotides, e.g., W, S, M, K, R, Y, B, D, H or V.

In the embodiments described above, the respective *M* could a predefined degenerative nucleotide such as W or S. For instance, a predefined nucleotide W for *M* implies that *M* is A or T (or A or U) but is not G or C. Correspondingly, a predefined nucleotide *S* for *M* means that *M* is G or C but not A or T (or A or U).

Particular examples of structured UMIs 14, 24 that could be used in the hairpin barcode primer 10, 20 include *NNNNMMMNMNNMMMNNNN, NNNNMMNNNMMNNNMMNNN,* and *NNNNMMNNNMMNNMMNNN,* in which each *M* is the same predefined nucleotide selected among A, T, C and G, such as A. Alternative versions of these structured UMIs 14, 24 include *NNNNSWSNNWNNSWSNNNN, NNNNWSWNNSNNWSWNNNN, NNNSWNNNSWNNNSWNNN, NNNWSNNNWSNNNWSNNN, NNNSWNNSWNNSWNNN* and *NNNWSNNWSNNWSNNN.* This alternative versions have alternating S and W as predefined nucleotide M, which gives a balanced GC content of the structured UMIs 14, 24.

Each hairpin barcode primer 10 and/or hairpin barcode reverse primer 20 preferably comprises a respective unique structured UMI 14, 24 having a random sequence that is different from the random sequences of structured UMIs 14, 24 in other hairpin barcode primers 10 and/or hairpin barcode reverse primers 20. The total length of the structured UMIs 14, 24 is equal to the combined lengths of the UMI parts 14A, 14B, 24A, 24B. This total length of the structured UMIs 14, 24 is preferably selected at least partly based on the number of nucleic acid molecules 1 in the sample. For instance, the number of unique structured UMIs 14, 24 is equal for $4^h$ for a structured UMI 14, 24 having a total length of $h$ nucleotides. This number $4^h$ should preferably be significantly larger than the number of nucleic acid molecules 1 in the sample.

The amplification of the nucleic acid molecule in step S2 in Figure 1 and step S11 in Figure 2 comprises performing PCR pre-amplification at an annealing temperature equal to or less than the closed annealing temperature of the hairpin barcode forward primer(s) 10 and/or the hairpin barcode reverse primer(s) 20. Accordingly, at this PCR pre-amplification in step S2 and S11 at least a majority of the hairpin barcode forward primers 10 and/or hairpin barcode reverse primers 20 have an intact hairpin loop, i.e., the adapter sequence 11, 21 is hybridized to the UMI sequence 17, 27. This in turn significantly reduces the amount of non-specific PCR products that may otherwise occur during the pre-amplification by the random nucleotide sequence of the structured UMI 14, 24. Hence, the vast majority of the PCR products from the PCR-amplification in step S2 and S11 are the desired barcoded PCR products 50 corresponding to an amplified portion 2, 3, 4 of the (target) nucleic acid molecule 1.

The closed annealing temperature Is lower than a melting temperature of the adapter sequence 11, 21 and the UMI sequence 17, 27. Correspondingly, the open annealing temperature is higher than a melting temperature of the adapter sequence 11, 21 and the UMI sequence 17, 27.

In an example, the closed annealing temperature is equal to or less than 65°C. Hence, in such an example, the PCR pre-amplification of step S2 and S11 is preferably performed at an annealing temperature equal to or less than to 65°C. For instance, the PCR pre-amplification of step S2 and S11 could be performed at an annealing temperature selected within an interval of from 60°C up to 65°C, preferably from 60°C up to 64°C, such as at about 62°C.

The PCR pre-amplification of the (target) nucleic acid molecule 1 is performed using a polymerase, preferably a DNA polymerase, and more preferably a heat-stable DNA polymerase. Non-limiting, but illustrative, examples of DNA polymerases that can be used according to the embodiments include *Thermus thermophilus* (Tth) DNA polymerase, *Bacillus stearothermophilus* DNA polymerase, *Thermus aquaticus* (Taq) DNA polymerase, *Thermus flavus* (Tfl) polymerase, Vent® DNA polymerase, Pfu polymerase, and *Escherichia coli* DNA polymerase I. In some embodiments, the DNA polymerase lacks 5'-nuclease activity. Examples of such polymerases include Klenow fragment of DNA polymerase 1, Stoeffel fragment of Taq polymerase, Pfu polymerase or Vent® polymerase. In an embodiment, the DNA polymerase is a so-called thermoactivated DNA polymerase, also referred to as, hot-start DNA polymerase. Specific examples of DNA polymerases include Takara PRIME STAR GXL polymerase I, Clontec"s ADVANTAGE HD Polymerase, NEB Q5® High-Fidelity DNA Polymerases NEB PHUSION® High-Fidelity DNA Polymerases, ThermoFisher PLATINUM® Taq DNA Polymerase High Fidelity, ThermoFisher ACCUPRIME™ Pfx DNA Polymerase, ThermoFisher ACCUPRIME™ Taq DNA Polymerase High Fidelity, ThermoFisher Phusion™ High Fidelity Polymerase, ThermoFisher Platinum™ SuperFi™ II DNA Polhymerase, Promega Pfu DNA Polymerase, and Qiagen HOTSTAR HIFIDELITY Polymerase. The above presented examples of DNA polymerases that can be used in the PCR pre-amplification in step S2 and S11 may also be used in the PCR amplification in step S4 in Figure 1 and step S13 in Figure 2.

In an embodiment, step S2 and S11 comprises amplifying the (target) nucleic acid molecules 1 by performing 1-40 cycles of PCR pre-amplification of the (target) nucleic acid molecules 1 to form the plurality of barcoded PCR products 50. In preferred embodiments, step S2 and S11 comprises amplifying the (target) nucleic acid molecules 1 by performing 1-30 cycles, and more preferably 1-25 cycles, such as 2-20 cycles and more preferably 2-15 cycles of PCR pre-amplification of the (target) nucleic acid molecules 1 to form the plurality of barcoded PCR products 50. Hence, it is generally preferred to perform a rather low number of PCR cycles in the PCR pre-amplification in step S2 and S11. This low number of PCR cycles, together with the protecting structured UMIs 14, 24 with Q hairpin loops, significantly reduces the amount of non-specific PCR products produced in step S2 and S11.

The barcoded PCR products 50 obtained in the amplification in step S2 and S11 are then contacted in step S3 or S12 with an adapter-specific forward primer 30 and an adapter-specific reverse primer 40 as indicated in Figure 3A.

In an embodiment, the adapter-specific forward primer 30 comprises a sequence equal to or complementary to, preferably equal to, the adapter sequence 13 of the forward primer(s) 10, such as of the hairpin barcode forward primer(s) 10. Correspondingly, the adapter-specific reverse primer 40 comprises a sequence equal to or complementary to, preferably equal to, the adapter sequence 23 of the reverse primer(s) 20.

In a particular embodiment, the adapter-specific forward primer 30 comprises, from a 5' end 30A to a 3' end 30B, one of a P5 sequence and a P7 sequence 32 and the sequence 33 equal to or complementary to, preferably equal to, at least a portion of the adapter sequence 11 of the forward primer(s) 10. In this particular embodiment, the adapter-specific reverse primer 40 comprises, from a 5' end 40A to a 3' end 40B, the other of the P5 sequence and the P7 sequence 42 and the sequence 43 equal to or complementary to, preferably equal to, at least a portion of the adapter sequence 21 of the reverse primer(s) 20. In a particular embodiment, the adapter-specific reverse primer 40 comprises, from the 5' end 40A to the 3' end 40B, the other of the P5 sequence and the P7 sequence 42, an index sequence and the sequence 43 equal to or complementary to, preferably equal to, at least a portion of the adapter sequence 21 of the reverse primer(s) 20.

In an embodiment, the P5 and P7 sequences are P5 and P7 ILLUMINA® sequences. In an embodiment, the P5 sequence comprises AATGATACGGCGACCACCGA (SEQ ID NO: 6). For instance, the P5 sequence could comprise, such as consist of, AATGATACGGCGACCACCGAGATCTACAC (SEQ ID NO: 7). In this latter example, at least one of the 3' nucleotides of the P5 sequence may be common for the P5 sequence and the following sequence 33. Correspondingly, in an embodiment, the P7 comprises, preferably consists of, CAAGCAGAAGACGGCATACGAGAT (SEQ ID NO: 8).

The PCR amplification in step S4 and S13 is performed at an annealing temperature equal to or greater than the open annealing temperature of the hairpin barcode forward primer(s) 10 and/or the hairpin barcode reverse primer(s) 20. At such an annealing temperature, at least a significant portion of the barcoded PCR products 50 are in an open structure, i.e., there is no significant hairpin loop formation by hybridization of complementary stem portions of the barcoded PCR products 50. In an example, the open annealing temperature is at least 70°C. In such a particular example, the PCR amplification in step S4 is performed at a temperature equal to or above 70°C, such as 71°C, 72°C or even higher.

In an embodiment, the open annealing temperature of the hairpin barcode forward primer(s) 10 and/or the hairpin barcode reverse primer(s) 20 is higher than the closed annealing temperature of the hairpin barcode forward primer(s) 10 and/or the hairpin barcode reverse primer(s) 20. In a particular embodiment, the open annealing temperature is at least 1°C, preferably at least 2°C, such at least 3°C or 4°C, and more preferably at least 5°C higher than the closed annealing temperature.

In an embodiment, step S4 in Figure 1 and step S13 in Figure 2 comprise amplifying the barcoded PCR products 50 by performing at least 2 cycles of PCR amplification on the barcoded PCR products 50 to form (a library of) amplified barcoded PCR products 60. In preferred embodiments, step S4 and S13 comprise amplifying the barcoded PCR products 50 by performing at least 3 cycles, such as at least 5 cycles, and more preferably at least 10 cycles of PCR amplification on the barcoded PCR products 50 to form the (library of) amplified barcoded PCR products 60. For instance, at least 15 cycles, such as at least 20 cycles, such as at least 25 cycles or at least 30 cycles of PCR amplification are performed on the barcoded PCR products 50 to form the (library of) amplified barcoded PCR products 60. The number of PCR cycles is preferably selected to achieve sufficient number of amplified barcoded PCR products 60 for sequencing even for nucleic acid molecules 1 present in a low copy number in the sample.

In a general embodiment, the number of PCR cycles in the amplification in step S4 and S13 is higher than the number of PCR cycles in the amplification in step S2 and S11.

In an optional, but preferred embodiment, the method comprises an additional step following step S2 and prior to step S4 in Figure 1 and following step S11 and prior to step S13 in Figure 2. This additional step then comprises degrading the polymerase used for amplifying the (target) nucleic acid molecules 1 in the PCR pre-amplification (in step S2 or S11) prior to amplifying the barcoded PCR products in the PCR amplification (in step S4 or S13).

Various techniques could be used to degrade polymerases including, but not limited to, heat treatment, chemical treatment, addition of protease inhibitors, sample dilution and enzymatic treatment. For instance, a protease could be added to the amplification products from step S2 or S11 to enzymatically degrade the polymerase used for amplifying the (target) nucleic acid molecules 1 in the PCR pre-amplification. Such a degradation of the polymerase once the PCR pre-amplification has been completed additionally inhibits formation of non-specific PCR products.

With reference to Figure 2, in an optional embodiment, the library of amplified barcoded PCR products 60 obtained in step S13 is first cleaned up prior to sequencing in step S14. For instance, the library may be purified to remove contaminants, such as dNTPs, salts, primers, etc., that may otherwise interfere with the sequencing. An example of such a purification is to use beads, such as magnetic beads, to bind the amplified barcoded PCR products 60 and thereby remove the amplified barcoded PCR products 60 from the remaining reagents from the PCR amplification in step S13. An illustrative, but non-limiting, example of a purification kit that could be used is AMPure XP for PCR Purification.

The amplified barcoded PCR products 60 are then sequenced in step S14 of Figure 2. The sequencing is achieved by means of at least one sequencing primer. The result of the sequencing in step S14 is respective sequence reads comprising at least nucleotide sequences of the UMI 64 and nucleic acid molecule sequence(s) 66.

In a particular embodiment, step S14 comprises *in situ* sequencing the at least a portion of the amplified barcoded PCR products 60 immobilized onto a solid support. For instance, the preferred P5 and P7 sequences introduced into the amplified barcoded PCR products 60 by means of the adapter-specific forward primers 30 and the adapter-specific reverse primers 40 could be used to immobilize the amplified barcoded PCR products 60 onto the solid support. In such an embodiment, the solid support preferably comprises immobilized nucleotide sequences complementary to the P5 sequence and/or immobilized nucleotide sequences complementary to the P7 sequence.

The *in situ* sequencing of step 56 preferably comprises *in situ* sequencing by synthesis of the at least a portion of the amplified product 60.

For instance, if the adapter-specific forward and reverse primers 30, 40 comprise P5 and P7 sequences, respectively, the ILLUMINA® sequencing technology could be used to *in situ* sequence at least a portion of the amplified barcoded PCR products 60 by synthesis. In more detail, the amplified barcoded PCR products 60 are immobilized on a flow cell surface designed to present the amplified barcoded PCR sequences 60 in a manner that facilitates access to enzymes while ensuring high stability of surface bound amplified barcoded PCR products 60 and low non-specific binding of fluorescently labeled nucleotides.

Sequence By Synthesis (SBS) uses two or four fluorescently labeled nucleotides to sequence the amplified barcoded PCR products 60 on the flow cell surface in parallel. During each sequencing cycle, a single labeled deoxynucleoside triphosphate (dNTP) is added to the nucleic acid chain. The nucleotide label serves as a terminator for polymerization so after each dNTP incorporation, the fluorescent dye is imaged to identify the base and then enzymatically cleaved to allow incorporation of the next nucleotide.

As an illustrative, but non-limiting, example ILLUMINA® MiniSeq system could be used to sequence the amplified barcoded PCR products 60.

Accurate quantification and sequence error correction with UMIs is predicated on a one-to-one relationship between the number of unique UMI barcodes at a given genomic locus and the number of unique sequence reads that have been sequenced. However, errors within the UMI sequence, including nucleotide substitutions during amplification and nucleotide miscalling and insertions or deletions (indels) during sequencing, create additional artefactual UMIs.

There are different bioinformatics models and methods for determining whether UMIs have the same nucleotide sequence or are regarded as being different UMIs, e.g., UMI error resolving methods (Genome Research (2017) 27(3): 491-499). The simplest method is to merge all UMIs, retaining only the UMI with the highest counts. For this method, the number of networks formed at a given locus is equivalent to the estimated number of unique molecules. This method is often referred to as cluster method. This method is expected to underestimate the number of unique molecules, especially for complex networks. A more accurate method is denoted adjacency method (Genome Research (2017) 27(3): 491-499), which attempts to correctly resolve the complex networks by using node counts. The most abundant node and all nodes connected to it are removed from the network. If this does not account for all the nodes in the network, the next most abundant node and its neighbors are also removed. This is repeated until all nodes in the network are accounted for. In the method, the total number of steps to resolve the network(s) formed at a given locus is equivalent to the number of estimated unique molecules. This method allows a complex network to originate from more than one UMI, although UMIs with an edit distance of two will always be removed in separate steps. The excess of UMIs pairs with an edit distance of two observed in the data sets indicate that some of these UMIs are artefactual. Reasoning that counts for UMIs generated by a single sequencing error should be higher than those generated by two errors and UMIs resulting from errors during the PCR amplification stage should have higher counts than UMIs resulting from sequencing errors, the directional method was developed (Genome Research (2017) 27(3): 491-499). Networks were generated from the UMIs at a single locus, in which directional edges connect nodes a single edit distance apart when $n_a \geq 2n_b - 1$, where $n_a$ and $n_b$ are the counts of node a and node b. The entire directional network is then considered to have originated from the node with the highest counts. The ratio between the final counts for the true UMI and the erroneous UMI generated from a PCR error is dependent upon which PCR cycle the error occurs and the relative amplification biases for the two UMIs, but should rarely be less than twofold. This method allows UMIs separated by edit distances greater than one to be merged so long as the intermediate UMI is also observed, and with each sequential base change from the most abundant UMI, the count decreases. For this method, the number of directional networks formed is equivalent to the estimated number of unique molecules.

The sequence reads as obtained in step S14 are then demultiplexed based on the nucleic acid sequences of the UMIs 64 in step S15. In an embodiment, this step S15 comprises dividing the sequence reads into groups having a same nucleotide sequence of the UMIs 64, optionally with at most a predefined number of mismatches allowed for nucleotide sequences of UMIs 64 in a same group. In a particular embodiment, this predefined number is zero, one or two, and preferably zero or one.

Thus, the sequence reads are preferably divided into different groups based on the nucleotide sequences of the UMIs so that sequence reads having the same UMI sequence are included in the same group. In this grouping or division of sequence reads, it is possible, in an embodiment, to accept up to two nucleotide mismatches, preferably up to one mismatch, between UMI sequences that belong to the same group.

The demultiplexed sequence reads are then mapped in step S16 to respective nucleic acid molecules based on nucleic acid sequences of the nucleic acid molecule sequences 66 in the sequence reads. In an embodiment, this step S16 comprises dividing demultiplexed sequence reads into groups having a same nucleotide sequence of the nucleic acid molecule sequences 66, optionally with at most a predefined number of mismatches allowed for nucleotide sequences of nucleic acid molecule sequences 66 in a same group. In a particular embodiment, the predefined number is an integer equal to or larger than 0 but no larger than ten, preferably no larger than nine, such as no larger than eight or seven, or even no larger than six or five. Thus, within each group of sequence reads following the demultiplexing in step S15, the sequence reads are further divided into groups, or sub-groups, based on the nucleotide sequences of the nucleic acid molecule sequences 66. This means that sequence reads having the same nucleic acid molecule sequences 66 are grouped into the same group. In this grouping or division of sequence reads, it is possible, in an embodiment, to accept up to 10 nucleotide mismatches, preferably up to 5-6 nucleotide mismatches, between nucleic acid molecule sequences 66 that belong to the same group. Mismatches are allowed in order to accept possible polymerase-caused nucleotide substitutions during library preparation and sequencing procedure.

The amplification method of the invention may simultaneously, i.e., in the same reaction vessel, amplify multiple different target nucleic acid sequences. In such a case, a sample comprising nucleic acid molecules 1 is contacted with P forward primers 10 and Q reverse primers 20 in step S1 of Figure 1.

In an embodiment, P is an integer equal to or larger than one and Q is an integer equal to or larger than one. The P forward primers 10 comprise, from a 5' end 10A to a 3' end 10B, an adapter sequence 11 and a target-specific sequence 16. Correspondingly, the Q reverse primers 20 comprise, from a 5' end 20A to a 3' end 20B, an adapter sequence 21 and a target-specific sequence 26.

In an embodiment, the P forward primers 10 are P hairpin barcode forward primers 10 and/or the Q reverse primers 20 are Q hairpin barcode reverse primers 20. In such an embodiment, each hairpin barcode forward primer 10 comprises, from the 5' end 10A to the 3' end 10B, the adapter sequence 11, a UMI sequence 17 comprising a structured UMI 14, and the target-specific sequence 16 complementary to a respective portion 2 of a nucleic acid molecule 1. Correspondingly, each hairpin barcode reverse primer 20 comprises, from the 5' end 20A to the 3' end 20B the adapter sequence 21, a UMI sequence comprising a structured UMI 24, and the target-specific sequence 26 complementary to a respective portion 3 of a nucleic acid molecule 1. At least a portion 12, 22 of the adapter sequence 11, 21 of the hairpin barcode forward primer 10 and/or the hairpin barcode reverse primer 20 is complementary to at least a portion 15, 25 of the UMI sequence 17, 27 of the hairpin barcode forward primer 10 and/or the hairpin barcode reverse primer 20. The adapter sequence 11, 21 and the UMI sequence 17, 27 are configured to hybridize to each other at or under a closed annealing temperature and not hybridize to each other at or above an open annealing temperature.

This embodiment of the method also comprises amplifying, in step S2, the nucleic acid molecules 1 by performing PCR pre-amplification of the nucleic acid molecules 1 to form a plurality of barcoded PCR products 50. The PCR pre-amplification as performed in step S2 has an annealing temperature equal to or less than the closed annealing temperature of the hairpin barcode forward primers 10 and/or the hairpin barcode reverse primers 20.

The embodiment further comprises contacting, in step S3, the plurality of barcoded PCR products 50 with an adapter-specific forward primer 30 and an adapter-specific reverse primer 40 and amplifying, in step S4, the barcoded PCR products 50 by performing PCR amplification on the barcoded PCR products 50 to form a library of amplified barcoded PCR products 60. In an embodiment, at least a portion of cycles of the PCR amplification has an annealing temperature greater than or equal to the open annealing temperature of the hairpin barcode forward primers 10 and/or the hairpin barcode reverse primer 20.

In an embodiment, the amplification method also comprises sequencing at least a respective portion of the amplified barcoded PCR products 60 to form respective sequence reads comprising the UMI(s) 64 and nucleic acid sequence(s) 66. The method further comprises demultiplexing the sequence reads based on nucleic acid sequences of the UMIs 64 and mapping the demultiplexed sequence reads to respective target nucleic acid sequences based on the sequences of the nucleic acid sequences 66.

The method of quantifying nucleic acid molecules of the embodiments may advantageously be used to determine lymphocyte clonality, see Figures 17A-17D.

The sequencing of immunoreceptor repertoires of B and T lymphocytes and tracking of specific clones provide essential information regarding many aspects of immunity, such as the extent of immune responses, localization of specific responding cells, the duration of immune responses, and presence of immune memory. An embodiment of the invention provides an ultrasensitive immune repertoire sequencing approach that can be used to determine lymphocyte clonality, such as represented by T-cell receptor (TCR) and/or B-cell receptor (BCR) clonotypes in a sample.

There are several challenges when analyzing lymphocyte clonality in a sample including, for instance, determining the exact number and size of the TCR and/or BCR clonotypes, no prior information of the number of different TCR and/or BCR clonotypes in the sample, distinguishing between clonotypes with similar sequences from sequence errors introduced during amplification and/or sequencing.

As used herein, each T cell or B cell clone carries a unique clonotype defined by the nucleotide sequence that arises during the gene rearrangement process for the TCR or BCR. In most applications, the clonotype is regarded as being defined by the complementarity-determining region 3 (CDR3) of the TCR or BCR, such as the CDR3 of the alpha ($\alpha$)

chain of the TCR (TRA) for αβ TCRs and T cells, or more preferably the CDR3 of the beta (β) chain of the TCR (TRB) for αβ TCRs and T cells, or the CDR3 of the gamma (γ) chain of the TCR (TRG) for γδ TCRs and T cells, or more preferably the CDR3 of the delta (δ) chain of the TCR (TRD) for γδ TCRs and T cells, or the CDR3 of the BCR for B cells.

The TCR alpha (α) chain is generated by recombination of variable (V) and joining (J) gene segments, whereas the TCR beta (β) chain is generated by recombination the V, diversity (D) and joining (J) gene segments. Correspondingly, the TCR gamma (γ) chain involves V and J recombination, whereas the TCR delta (δ) chain is generated by V, D and J recombination.

Determination of lymphocyte clonality could thereby be used to determine the particular combination of V (TRAV) and J (TRAJ) gene segments of the TCR alpha (α) chain and/or the particular combination of V (TRBV) and D (TRBD) gene segments, D and J (TRBJ) gene segments or V, D and J gene segments of the TCR beta (β) chain for αβ T cells. Correspondingly, determination of lymphocyte clonality could be used to determine the particular combination of V (TRGV) and J (TRGJ) gene segments of the TCR gamma (γ) chain and/or the particular combination of V (TRDV) and D (TRDD) gene segments, D and J (TRDJ) gene segments or V, D and J gene segments of the TCR delta (δ) chain for γδ T cells.

The BCR is also generated based on V, D and J recombination. This means that determination of lymphocyte clonality could be used to determine the particular combination of V and J gene segments, D and J gene segments or V, D and J gene segments of the BCR for B cells.

In an embodiment, a method of determining lymphocyte clonality, see Figures 2, 3A to 3C and 4A to 4C, comprises contacting, in step S10, a sample comprising nucleic acid molecules 1 of lymphocytes with P forward primers 10 and Q reverse primers 20.

In an embodiment, P is an integer equal to or larger than one and Q is an integer equal to or larger than one. The P forward primers 10 comprise, from a 5' end 10A to a 3' end 10B, an adapter sequence 11 and a target-specific sequence 16. Correspondingly, the Q reverse primers 20 comprise, from a 5' end 20A to a 3' end 20B, an adapter sequence 21 and a target-specific sequence 26.

In an embodiment, the P forward primers 10 are P hairpin barcode forward primers 10 and/or the Q reverse primers 20 are Q hairpin barcode reverse primers 20. In such an embodiment, each hairpin barcode forward primer 10 comprises, from the 5' end 10A to the 3' end 10B, the adapter sequence 11, a UMI sequence comprising a structured UMI 14, and the target-specific sequence 16 complementary to a respective portion 2 of a TCR or BCR clonotype. Correspondingly, each hairpin barcode reverse primer 20 comprises, from the 5' end 20A to the 3' end 20B, the adapter sequence 21, a UMI sequence comprising a structured UMI 24, and the target-specific sequence 26 complementary to a respective portion 3 of a TCR or BCR clonotype. At least a portion 12, 22 of the adapter sequence 11, 21 of the hairpin barcode forward primer 10 and/or the hairpin barcode reverse primer 20 is complementary to at least a portion 15, 25 of the UMI sequence 17, 27 of the hairpin barcode forward primer 10 and/or the hairpin barcode reverse primer 20. The adapter sequence 11, 21 and the UMI sequence 17, 27 are configured to hybridize to each other at or under a closed annealing temperature and not hybridize to each other at or above an open annealing temperature.

The method as shown in Figure 2 also comprises amplifying, in step S11, the nucleic acid molecules 1 by performing PCR pre-amplification of the nucleic acid molecules 1 to form a plurality of barcoded PCR products 50. The PCR pre-amplification as performed in step S11 has an annealing temperature equal to or less than the closed annealing temperature of the hairpin barcode forward primers 10 and/or the hairpin barcode reverse primers 20.

The method further comprises contacting, in step S12, the plurality of barcoded PCR products 50 with an adapter-specific forward primer 30 and an adapter-specific reverse primer 40 and amplifying, in step S13, the barcoded PCR products 50 by performing PCR amplification on the barcoded PCR products 50 to form a library of amplified barcoded PCR products 60. In an embodiment, at least a portion of cycles of the PCR amplification has an annealing temperature greater than or equal to the open annealing temperature of the hairpin barcode forward primers 10 and/or the hairpin barcode reverse primers 20.

The method also comprises sequencing, in step S14, at least a respective portion of the amplified barcoded PCR products 60 to form respective sequence reads comprising the UMI(s) 64 and TCR or BCR sequence(s) 66. The method further comprises demultiplexing, in step S15, the sequence reads based on nucleic acid sequences of the UMIs 64 and mapping, in step S16, the demultiplexed sequence reads to respective TCR or BCR clonotypes based on nucleic acid sequences of the TCR or BCR sequences 66. The lymphocyte clonality for the sample is then determined in step S17 based on the demultiplexed and mapped sequence reads.

The sample used in the determination of lymphocyte clonality could be any sample comprising nucleic acid molecules 1 of lymphocytes, such as T cells or lymphocytes, B cells or lymphocytes or T and B cells or lymphocytes.

In an embodiment, the sample is an enriched lymphocyte sample, in which lymphocytes have been enriched and isolated from a biological sample, such as taken from a subject. For instance, lymphocytes could be enriched from buffy coats or peripheral blood mononuclear cells (PBMCs) using immunomagnetic cell separation, fluorescence-activated cell sorting (FACS) or other lymphocyte enriching techniques.

However, an advantage of the present invention is that no lymphocyte enrichment is needed in order to determine lymphocyte clonality. For instance, PBMCs could be isolated from buffy coat of a subject and used as sample in the method shown in Figure 2.

The sample could therefore be a lymphocyte enriched sample or a biological sample that is not subject to lymphocyte enrichment. Examples of the latter include a blood sample or a buffy coat sample. The buffy coat is the fraction of an anticoagulated blood sample that contains most of the lymphocytes and platelets following density gradient centrifugation. In fact, the present invention can be used for any sample comprising lymphocytes or lymphocyte nucleic acid molecules, also including tissue samples, a fixed sample, i.e., a biological sample, such as a tissue sample, that has been subject to fixation to preserve the biological sample.

True clonal variation is difficult to separate from sequencing errors. By comparing the frequency of multiple clonotypes that differ from each other by merely one or a few nucleotides, many clonality determining methods that are based on bioinformatical processes assume that low-frequency clonotype(s) is(are) a sequencing mistake and instead interpret such a low-frequency clonotype as being one of the more frequently expressed clonotypes but with one or a few sequencing errors. Hence, a sample containing a low-frequency clonotype and a high-frequency clonotype may in such a case be misinterpreted to only contain the high-frequency clonotype.

Furthermore, amplification steps in the clonality determination introduce PCR-induced amplification biases and errors, which effectively prevent accurate clonality determination and quantification. UMIs are known to be used to reduce such PCR-induced amplification biases and errors. To date, UMIs have mostly been applied to messenger ribonucleic acid (mRNA) when profiling the immune receptor repertoire of B and T cells to remove PCR duplicates and improving sequence accuracy. However, the analysis of mRNA has several disadvantages. Firstly, the number of transcripts per cell is not constant. Hence, the correct number and size of various T or B cell clones cannot be accurately estimated. Secondly, the reverse transcription efficiency is variable between sequences, and reverse transcriptase is up to 1000 times more prone to introduce errors than DNA polymerase, causing both quantification and sequence biases. Hence, the nucleic acid molecules 1 are DNA molecules 1 and the sample comprises DNA molecules 1 of lymphocytes.

Immune repertoire sequencing can be applied to both T and B cells to explore a V(D)J recombination (Clinical Immunology (2020) 66(9): 1228-1237). In another application, ultrasensitive immune repertoire sequencing is used to explore the repertoire of the recombined immunoglobulin heavy (IGH) locus. Using a set of primers specific for all the IGH variable regions and a different set of primers specific for all IGH joining regions (Table 1) an immune repertoire can be amplified and analyzed using ultrasensitive immune repertoire sequencing. In this specific implementation, the primer containing the hairpin with barcode is the primer complementary to the J region. In the specific implementation, the primers are not perfectly complementary to the variable or joining region as each primer can amplify a plurality of variable or joining regions with share homology respectively, see Figure 17.

Table 1 - primers for immune repertoire sequencing of the IGH locus

| Name | Sequence 5' - 3' | SEQ ID NO: |
|---|---|---|
| J-h-consensus_rev | GGACACTCTTTCCCTACACGACGCTCTTCCGATCT-**UMI**-ATGGGAAAGAGTGTCCCTTACCTGAGGAGACGGTGACC | 91 |
| VH1-FR3_fw | GTGACTGGAGTTCAGACGTGTGCTCTTCCGATCTTGGAGCTGAGCAGCCTGAGATCTGA | 92 |
| VH2-FR3_fw | GTGACTGGAGTTCAGACGTGTGCTCTTCCGATCTCAATGACCAACATGGACCCTGTGGA | 93 |
| VH3-FR3_fw | GTGACTGGAGTTCAGACGTGTGCTCTTCCGATCTTCTGCAAATGAACAGCCTGAGAGCC | 94 |
| VH4-FR3_fw | GTGACTGGAGTTCAGACGTGTGCTCTTCCGATCTGAGCTCTGTGACCGCCGCGGACACG | 95 |
| VH5-FR3_fw | GTGACTGGAGTTCAGACGTGTGCTCTTCCGATCTCAGCACCGCCTACCTGCAGTGGAGC | 96 |
| VH6-FR3_fw | GTGACTGGAGTTCAGACGTGTGCTCTTCCGATCTGTTCTCCCTGCAGCTGAACTCTGTG | 97 |
| VH7-FR3_fw | GTGACTGGAGTTCAGACGTGTGCTCTTCCGATCTCAGCACGGCATATCTGCAGATCAG | 98 |

UMI as referred to in Table 1 could be any structured UMI 14 as disclosed herein.

In an embodiment, step S17 of Figure 2 comprises determining the lymphocyte clonality for the sample based on the demultiplexed and mapped sequence reads in step S17. In an embodiment, this step S17 comprises identifying any TCR or BCR clonotypes present in the sample. Hence, this step S17 preferably comprises identifying all TCR or BCR clonotypes that are present in the sample to thereby identify individual T cell or B cell clones in the sample. In an embodiment, step S17 also, or additionally, comprises quantifying the TCR or BCR clonotypes present in the sample based on the demultiplexed and mapped sequence reads.

Hence, not only the identities of the different TCR or BCR clonotypes in the sample are determined in step S17 but also a quantity thereof. Such a quantification of TCR or BCR clonotypes is possible due the presence of structured UMI 14, 24 in the hairpin barcode forward primers 10 and/or the hairpin barcode reverse primers 20. The quantification can be an absolute quantification, i.e., the total copy number of each TCR or BCR clonotype present in the sample, or a relative quantification, e.g., frequencies of the different TCR or BCR clonotypes present in the sample such as relative to each other or a reference sequence present in or added to the sample or the reaction mixtures in any of the steps S11 to S13.

In a particular embodiment, quantifying the nucleic acid molecules, such as TCR or BCR clonotypes, comprises quantifying the nucleic acid molecules, such as TCR or BCR clonotypes, based on determining the number of different UMIs 64, optionally with at most a first predefined number mismatches, having the same nucleotide sequence of the nucleic acid molecule sequences, such as TCR or BCR sequence 66, optionally with at most a second predefined number of mismatches.

In a particular embodiment, the first predefined number is zero, one or two, preferably zero or one, and the second predefined number is an integer equal to or larger than 0 but no larger than ten, preferably no larger than eight and more preferably no larger than five.

For instance, the Molecular Identifier Guided Error Correction (MIGEC) pipeline could be used in the demultiplexing and mapping steps S15 and S16. MIGEC comprises de-multiplexing, adapter trimming and read overlapping for sequence reads data and extraction of UMI sequences. MIGEC also comprises assembly of consensuses for original molecules that entered library preparation by grouping reads with identical molecular identifiers and mapping of V, D and J segments, extraction of CDR3 regions and clonotype assembly for all human and mouse immune receptors (TRA/TRB/TRG/TRD and IGH/IGL/IGK). Further features of MIGEC include additional filtering of hot-spot errors in CDR3 sequence.

In an embodiment, if P is one then Q is equal to or larger than two and if Q is one then P is equal to or larger than two. In this particular embodiment, multiple different forward primer 10 and/or multiple different reverse primers 20 are used in the method.

In an embodiment, determining lymphocyte clonality comprises determining clonality of $\alpha\beta$ TCRs and $\alpha\beta$ T cells. In a particular embodiment, the P forward primers 10 comprise at least one forward primer 10 per V region of a TCR alpha ($\alpha$) chain and the Q reverse primers 20 comprise at least one reverse primer 20 per J region of the TCR alpha ($\alpha$) chain. In other particular embodiments, the *P* forward primers 10 comprise at least one forward primer 10 per V region of a TCR beta ($\beta$) chain and the *Q* reverse primers 20 comprise at least one reverse primer 20 per D region of the TCR beta ($\beta$) chain, or the *P* forward primers 10 comprise at least one forward primer 10 per D region of the TCR beta ($\beta$) chain and the *Q* reverse primers 20 comprise at least one reverse primer 20 per J region of the TCR beta ($\beta$) chain, or the *P* forward primers 10 comprise at least one forward primer 10 per V region of the TCR beta ($\beta$) chain and the Q reverse primers 20 comprise at least one reverse primer 20 per J region of the TCR beta ($\beta$) chain.

It is also possible to determine clonality of both the TCR alpha ($\alpha$) chain and the TCR beta ($\beta$) chain in the same sample. In such a case, combinations of forward and reverse primers 10, 20 according to above are used in the same reaction mixture, or the sample is split into two samples and the two clonality determinations are run in parallel.

In another embodiment, determining lymphocyte clonality comprises determining clonality of $\gamma\delta$ TCRs and $\gamma\delta$ T cells. In a particular embodiment, the P forward primers 10 comprise at least one forward primer 10 per V region of a TCR gamma ($\gamma$) chain and the Q *reverse* primers 20 comprise at least one reverse primer 20 per J region of the TCR gamma ($\gamma$) chain. In other particular embodiments, the P forward primers 10 comprise at least one forward primer 10 per V region of a TCR delta ($\delta$) chain and the Q reverse primers 20 comprise at least one reverse primer 20 per D region of the TCR delta ($\delta$) chain, or the *P* forward primers 10 comprise at least one forward primer 10 per D region of the TCR delta ($\delta$)) chain and the *Q* reverse primers 20 comprise at least one reverse primer 20 per J region of the TCR delta ($\delta$) chain, or the *P* forward primers 10 comprise at least one forward primer 10 per V region of the TCR delta ($\delta$) chain and the Q reverse primers 20 comprise at least one reverse primer 20 per J region of the TCR delta ($\delta$) chain.

It is also possible to determine clonality of both the TCR gamma ($\gamma$) chain and the TCR delta ($\delta$) chain in the same sample. In such a case, combinations of forward and reverse primers 10, 20 according to above are used in the same reaction mixture, or the sample is split into two samples and the two clonality determinations are run in parallel.

In fact, it is actually possible to determine clonality of both $\alpha\beta$ TCRs and $\alpha\beta$ T cells and $\gamma\delta$ TCRs and $\gamma\delta$ T cells in the same sample according to the present invention.

In a further embodiment, determining lymphocyte clonality comprises determining clonality of BCRs and B cells.

In particular embodiments, the P forward primers 10 comprise at least one forward primer 10 per V region of the BCR and the Q reverse primers 20 comprise at least one reverse primer 20 per D region of the BCR, or the P forward primers 10 comprise at least one forward primer 10 per D region of the BCR and the Q reverse primers 20 comprise at least one reverse primer 20 per J region of the BCR, or the P forward primers 10 comprise at least one forward primer 10 per V region of the BCR and the Q reverse primers 20 comprise at least one reverse primer 20 per J region of the BCR.

In fact, it is actually possible to determine clonality of both $\alpha\beta$ and/or $\gamma\delta$ TCRs and $\alpha\beta$ and/or $\gamma\delta$ T cells and BCRs and B cells in the same sample according to the present invention.

In a particular embodiment, the P forward primers 10 are multiple different hairpin barcode forward primers 10 comprising at least one hairpin barcode forward primer 10 per variable (V) region of a TCR or BCR. In such a particular embodiment, each target-specific sequence 16 of the hairpin barcode forward primers 10 is preferably complementary to a respective variable (V) region 2 of the TCR or BCR.

In such an embodiment, step S10 of Figure 2 preferably comprises contacting the sample with the multiple different hairpin barcode forward primers 10 and multiple different reverse primers 20. The multiple different reverse primers 20 comprise at least one reverse primer 20 per joining (J) region 3 of the TCR or BCR. In this embodiment, each reverse primer 20 comprises, from the 5' end 20A to the 3' end 20B, the adapter sequence 23 and a target-specific sequence 26 complementary to a respective joining (J) region 3 of the TCR or BCR.

The monitoring of T and/or B cell clonality has immense importance in the diagnosis and follow-up of various disease, such as hematological diseases and is a fundamental tool for the understanding of antigen specificity in immunity to infections, cancer, and in autoimmunity. Clinically, there is a need for improved methodological specificity and sensitivity to determine the immune repertoire, for example, to detect acute lymphoblastic leukemia and to monitor minimal residual disease. Moreover, in studies of immune disorders such as autoimmunity and tumor immunity, improved immune repertoire analysis will facilitate the identification of involved immune cells, be used to evaluate the efficiency of immunomodulating drugs, and explore their mode of action.

Thus, the lymphocyte clonality as determined according to the present invention is a valuable information source in diagnosis and monitoring of various diseases, and can thereby be used in disease characterization.

A method of disease characterization is disclosed. The method comprises determining lymphocyte clonality as disclosed herein of biological sample comprising nucleic acid molecules 1 of lymphocytes obtained from a subject. The method also comprises characterizing a disease of the subject based on the determined lymphocyte clonality. In an embodiment, the disease is selected from the group consisting of a hematologic disease, an infectious disease, a cancer disease and an autoimmune disease.

Characterizing a disease as used herein could, in an example, include predicting or determining a likelihood that the subject is suffering from the disease. Hence, information of the lymphocyte clonality, as determined from a sample taken from the subject, can be used to assess whether the subject is likely to suffer from the disease.

For instance, information of lymphocyte clonality could be useful in monitoring infections, such as bacterial infections or virus infections. Hence, in an embodiment, information of lymphocyte clonality as determined according to the invention could be used in monitoring, including diagnosing, a virus infection, such as a coronavirus infection, including, but not limited to, a severe acute respiratory syndrome (SARS) coronavirus 1 (SARS-CoV-1), SARS-CoV-2, Middle East Respiratory Syndrome (MERS) coronavirus (MERS-CoV).

Alternatively, or additionally, characterizing a disease could comprise monitoring disease progression, such as monitoring whether the subject recovers from a disease, whether the subject's condition deteriorates or whether subject's disease condition remains unchanged. Thus, in information of lymphocyte clonality could be used to monitor disease progression and recovery from the disease.

Characterizing a disease could further include evaluating the efficiency of various treatments of a disease, such as administration of drugs, e.g., immunomodulating drugs, and/or determining their mode of action. Hence, information of lymphocyte clonality could then be used to assess how well a subject responds to the treatment and whether the treatment seem to have any effect on the disease or, whether another potentially more effect treatment should be selected instead.

EXAMPLES

EXAMPLE 1

This Example investigated whether formation of non-specific PCR product caused by UMIs can be minimized by adding specific nucleotides within the UMIs, i.e., structured UMIs, to reduce the possibility of stable interactions. Here, we developed a digital sequencing approach based on the simple multiplexed PCR based barcoding of DNA for ultrasensitive mutation detection using next-generation sequencing (SiMSen-Seq) technology. Assay performance of several structured UMI strategies was evaluated. Assay characteristics and specificity were analyzed by quantitative PCR, capillary gel electrophoresis, melting curve analysis and sequencing. We demonstrate how both individual assays and multiplexed panels are improved using structured UMIs. Improved experimental protocols to mitigate the limitations of UMIs will facilitate the implementation of digital sequencing in clinical and basic research settings.

## Materials and methods

*Library construction*

SiMSen-Seq assays were designed and tested as previously described (Nat. Protoc. (2017) 12: 664-682). The SiMSen-Seq protocol consists of two PCR steps followed by library purification and sequencing. The first 10 $\mu$l barcoding PCR contained, 0.05 U Platinum SuperFi I DNA polymerase, 1× SuperFi Buffer (#12351010, Thermo Fisher Scientific), 0.2 mM deoxyribonucleotide triphosphate (#D7295, Merck), 40 nM of each primer (Ultramer, Integrated DNA Technologies, Table 5), 0.5 M L-carnitine inner salt (#C0158, Sigma-Aldrich) and 20 ng human genomic DNA (#11691112001, Roche Diagnostics). The standard curve was performed with 27, 10, 3, 1 and 0.33 ng DNA per reaction. The following thermal program was used on a T100 Thermal cycler (Bio-Rad Laboratories): 30 s at 98 °C, 3 cycles of amplification (98 °C for 10 s, 62 °C for 6 min, 72 °C for 30 s), 15 min at 65 °C, and 15 min at 95 °C. At the start of the 15 min incubation step at 65 °C, the DNA polymerase was inactivated by adding 20 $\mu$l TE-buffer (pH 8.0, #AM958, Thermo Fisher Scientific) supplemented with 45 ng/$\mu$l *Streptomyces griseus* protease (#P5147-100MG, Merck). The second adapter PCR was performed in 40 $\mu$l reactions, containing 1× Q5 Hot Start High-Fidelity Master Mix (#M0494S, New England BioLabs) and 400 nM of each Illumina adapter primer (desalted, Sigma-Aldrich, Table 2). The following thermal program was used on a T100 Thermal cycler: 98 °C for 3 min, followed by 30 cycles of amplification (98 °C for 10 s, 80 °C for 1 s, 72 °C for 30 s, 76 °C for 30 s, all with ramping rate at 0.2 °C/s). The 16-plex was experimentally performed as an 18-plex reaction, but two assays (TP53_C and TP53_D) were omitted in downstream analysis, since they have overlapping amplicons.

*Sequencing*

Library concentrations were assessed using on a 5200 Fragment Analyzer (Agilent Technologies) using the DNF-474 HS NGS Fragment Kit (1 - 6000 base pairs, #DNF-474-1000, Agilent Technologies). Samples were pooled to enable similar sequencing depth across all samples. The pooled library was purified with a Pippin Prep (Sage Science) using 2% agarose, dye-free, Pippin Gel Cassette (100 - 600 base pairs, #CDF2010, Sage Science), according to manufacturer's instructions. Libraries were collected within the 205-305 bp size range. Purified libraries were quantified using quantitative PCR and the NEBNext Library Quant Kit (# E7630, New England Biolabs). Briefly, 2 $\mu$l of the purified libraries was quantified in 10 $\mu$l reactions, containing 1× TATAA SYBR GrandMaster Mix Low Rox (#TA01-3750LR, TATAA Biocenter) and 400 nM of each ILLUMINA® adapter primer (SEQ ID NO: 6 and 8). The concentration was estimated using a standard curve with adjustment to the average library size. Sequencing was performed on a MiniSeq (Illumina) with 20% PhiX (#FC-110-3001, Illumina) using the MiniSeq High Output Reagent Kit (#FC-420-1002, Illumina). The final library concentration was 1.4 pM and sequencing was performed in single-end 150 base pairs mode.

*Data analysis*

Sequencing data was analyzed using the UMIErrorCorrect pipeline (version 0.21, https://github.com/stahl berggroup/umierrorcorrect). Briefly, fastq-files were preprocessed by trimming off the UMI and spacer sequence. The pipeline then aligned reads to the hg38 reference genome using Burrows-Wheeler aligner (Bioinformatics (2009) 25: 1754-1760), clustering reads into UMI families based on their UMI sequence and chromosomal position. Each UMI family was collapsed to one consensus read, counting the most common nucleotide type at each position. Final data was filtered using UMI family size cutoff ≥ 3. The number of off-target reads was calculated as the difference between the total number of observed reads and the number of reads aligned to intended target sequences. The error rate was calculated as the total number of non-reference alleles per nucleotide position divided by coverage for each nucleotide position. Then the average of all nucleotide positions was calculated per amplicon. Single nucleotide polymorphism positions identified in the Single Nucleotide Polymorphism Database of Nucleotide Sequence Variation were excluded (Nucleic Acids Res (2001) 29(1): 308-311).

Figures and statistical tests were performed using GraphPad Prism version 8.4.3 (GraphPad Software) and R version 4.2.2 (R Core Team. R: A language and environment for statistical computing. Vienna, Austria: R Foundation for Statistical Computing; 2022.), using packages ggplot2 (Wickham H. ggplot2: elegant graphics for data analysis. New York: Springer-Verlag; 2016.) and ggpubr (Kassambara A. ggpubr: "ggplot2" based publication ready plots. CRAN; 2020).

*Quantitative PCR*

The amount of UMI-tagged DNA was quantified using quantitative PCR and a CFX384 Touch Real-Time PCR Detection System (Bio-Rad Laboratories). Two microliters PCR product from the first barcoding PCR reaction was

analyzed in 10 μl reactions, containing 1× TATAA SYBR GrandMaster Mix Low Rox (#TA01-3750LR, TATAA Biocenter) and 400 nM of each Illumina adapter primer (Table 5). The following thermal program was used: 98 °C for 3 min, 45 cycles of amplification (98 °C for 10 s, 80 °C for 1 s, 72 °C for 30 s, 76 °C for 30 s, all with ramping rate at 0.2 °C/s) and melting curve analysis ranging from 60 °C to 95 °C, 0.5 °C /s increments. Cycle of quantification values were determined by regression using the Bio-Rad CFX Maestro software (version 4.1, Bio-Rad Laboratories).

*Capillary gel electrophoresis*

The PCR products from the second adapter PCR were evaluated on a 5200 Fragment Analyzer using dsDNA 915 Reagent kit (35 - 5000 base pairs, # DNF-915-K1000, Agilent Technologies), according to the manufacturer's instructions. Library purity was calculated as the fraction of specific library product (200 - 300 base pairs) compared with all formed PCR products. Analysis was performed in ProSize Data Analysis Software version 4.0.0.3 (Agilent Technologies).

*Melting curve analysis*

The melting temperature of the stem-loop structure in barcoding primers was determined by high resolution melting curve analysis using a Mic PCR machine (Bio Molecular Systems). The 20 μl reaction contained 1× SuperFi buffer, 1× SYBR Green I nucleic acid gel stain (#S9430, Sigma-Aldrich) and 1 μM forward barcoding primer (Table 5). The temperature profile was 95 °C for 3 min, 60 °C for 3 min, followed by melting analysis from 60 °C to 90 °C with ramping at 0.03 °C/s. Melting curve analysis was performed using the Mic quantitative PCR software, version 2.10.03 (Bio Molecular Systems).

Table 2 - Primer sequences

| Assay name | F primer (5'→3') | SEQ ID NO: | R primer (5'→3') | SEQ ID NO: | 3-plex |
|---|---|---|---|---|---|
| ARID2 | TGCTCATTTTGAAGTAAATCCAGATTGTTCTGTT | 10 | ACTGCAAGTCGAGAGGTATTCAGAATACATT | 11 | H |
| BRAF | TGGGACCCACTCCATCG | 12 | ATATTTCTTCATGAAGACCTCACAGTAA | 13 | G |
| CDKN2A | CAACTGCGCCGACCCC | 14 | CAGCGTGTCCAGGAAGCC | 15 | E |
| CNOT9 | TTGCACACTGTCAGCAAAAC | 16 | ACCAATAACTCCAAGGCTGGT | 17 | I |
| CTNNB1 | GTCACTGGCAGCAACAGTCTTACCTG | 18 | TTCCTCAGGATTGCCTTTACCACTCAGA | 19 | A |
| CYSLTR2 | TGGCCTGCAGGATTATGTCTTATT | 20 | CCAGGAAACGCACAACACTCAG | 21 | J |
| EZH2 | CTGAATACAGGTTATCAGTGCCT | 22 | TCAAAGATCCTGTGCAGAAAAATGAAT | 23 | M |
| FBXW7_A | TGCAGAGTTCAGTTACCTTAGGAGA | 24 | TGCATACATCAGACAGCACAGA | 25 | F |
| FBXW7_B | CCATGACAAGATTTTCCCTTACC | 26 | CACCTTATATGGGCATACTTCCACT | 27 | L |
| GNA11_A | CCACCTTGGGCTACCTGC | 28 | GGTACTCGATGATGCCGGTG | 29 | D |
| GNA11_B | GTCCTTTCAGGATGGTGGATGTG | 30 | GGATGTCACGTTCTCAAAGCAGT | 31 | C |
| GNAQ_A | ATTCGATGATCCCTGTGGTGG | 32 | GTAGCTGACCCTGCCTACCT | 33 | M |
| GNAQ_B | GCAGTGTATCCATTTTCTTCTCTCT | 34 | GTATTGTTAACCTTGCAGAATGGTC | 35 | L |

24

(continued)

| Assay name | F primer (5'→3') | SEQ ID NO: | R primer (5'→3') | SEQ ID NO: | 3-plex |
|---|---|---|---|---|---|
| HRAS | GCGATGACGGAATATAAGCTG | 36 | CTGGATCAGCTGGATGGTCA | 37 | F |
| IDH1_A | CCAACATGACTTACTTGATCCCCATAA | 38 | CTTGTGAGTGGATGGGTAAAACCTA | 39 | H |
| IDH2_B | AGTGGATCCCCTCTCCACC | 40 | TAGTCCCTGGCTGGACCAAG | 41 | M |
| MEK1_C | AGGGAGCTGCAGGTTCTG | 42 | GCCATCGCTGTAGAACGCA | 43 | E |
| MEK1_D | GCAGATGTCAAGCCCTCCAA | 44 | GCTGACCCCAAAGTCACAGA | 45 | C |
| MEK1_A | CGCCTTGAGGCCTTTCTTAC | 46 | GTCGTCATCCTTCAGTTCTCC | 47 | D |
| MEK1_B | AACCTCTCTTTCTTCCACCTTTCTC | 48 | CTTATGATCTGGTTCCGGATTGCG | 49 | G |
| NF1 | TCATCAGTTCCTCCTCAGAATTCCCCC | 50 | AAGCATACCTGGTATAAACAGTGGCACAC | 51 | B |
| NRAS | GCTGGATTGTCAGTGCGCTTTTC | 52 | GACTGAGTACAAACTGGTGGTGGTTGG | 53 | J |
| PLCB4 | CGCATTTACCCCAAGGGAGG | 54 | CAGCCAGCGTTCCAGAAAATC | 55 | A |
| PPP6C | AGCCTTCGTGCACTAGTTGAT | 56 | GTGCCTTTCTAAATTGCAGTTTGTT | 57 | B |
| RAC1 | ACTTGCCTACTGATCAGTTACACAACCAATG | 58 | GGTTAATTCCCAATTTAAGATACTTACACAGTAGGG | 59 | J |
| SF3B1_F | GGCTCCCATAACTGCCTGAAT | 60 | GGATTTGGTTGTGAAGATTCGCT | 61 | E |
| SF3B1_A | CCATGTAAGTCCTAGCATTCATTT | 62 | TGTACTCTTTTTCCTGTTGCTGT | 63 | E |
| SF3B1_B | GAGGCTACAACAGCAAAAGCTC | 64 | CTATGATCTCTACCATGAGACCTGA | 65 | L |
| SF3B1_C | AGATGGCACAGCCCATAAGAA | 66 | GAAGTCCTGGCAAGCGAGA | 67 | D |
| SF3B1_D | GCAATGGCCAAAGCACTGAT | 68 | CCTGTGTTTGGTTTTGTAGGTCT | 69 | D |
| SF3B1_E | ACCATCAATCAGTTGTTCTTCAAGT | 70 | AAATGGTGATGGAGACAATTGAG | 71 | H |
| STK19 | GTACGTCACTGCTCTGCGCCG | 72 | GGTAGGAATGGAGGGAAGAGAACCTGAA | 73 | B |
| TP53_D | GTGGCAAGTGGCTCCTGA | 74 | CATGGGCGGCATGAAC | 75 | C |
| TP53_A | GGCCCCTGTCATCTTCTGT | 76 | CAGAATGCAAGAAGCCCAGA | 77 | A |
| TP53_B | TTGCGTGTGGAGTATTTGGA | 78 | AGACCTCAGGCGGCTCATAG | 79 | I |
| TP53_C | CTTCCAGTGTGATGATGGTGAGGATGG | 80 | ACATGTGTAACAGTTCCTGCATGGGC | 81 | G |
| TP53_E | TCCTCTGTTGCTGCAGATCC | 82 | TGAGTTCCAAGGCCTCATTC | 83 | I |

(continued)

| Assay name | F primer (5'→3') | SEQ ID NO: | R primer (5'→3') | SEQ ID NO: | 3-plex |
|---|---|---|---|---|---|
| WT1 | CCTGGTGTGGGTCTTCAGGTGG | 84 | GTGTGAAACCATTCCAGTGTAAA ACTTGTCAG | 85 | F |
| TP53_F | GTGGCAAGTGGCTCCTGA | 86 | CATGGGCGGCATGAAC | 87 | G |

Additional sequence of forward (F) primer (SEQ ID NO: 9):
GGACACTCTTTCCCTACACGACGCTCTTCCGATCTNNNNNNNNNNNNNATGGGAAAGAGTGTCC
Additional sequence of reverse I primer (SEQ ID NO: 5):
GTGACTGGAGTTCAGACGTGTGCTCTTCCGATCT

Table 3 - Barcode sequences

| Name | Sequence | SEQ ID NO: |
|---|---|---|
| A | NNNNNWWNNNNN | - |
| B | SSWSWWWSWWSWSS | 88 |
| C | SWSSWWWSSWSWWWSWWSWSS | 89 |
| D | NNANNNNANNNNANN | - |
| E | NNANNANNANNANNANN | - |
| F | NNNNAAANNANNAAANNNN | - |
| G | NNNAANNNAANNNAANNN | - |
| H | NNTNNANNCANNANN | - |
| I | NNTNNANNCNNANN | - |
| J | NNNAANNNACANNNAANNN | - |
| K | NNTNNANNCNNANNTNN | - |
| L | NANTNANCNTNTNCNTNANTNAN | 90 |
| M | NANTNANCNTNTNCNTNAN | - |
| N | NNNNNNGAGCGNNNNNN | - |
| Reference | NNNNNNNNNNNN | - |

## Results

*Design of different UMI structures using digital sequencing*

To evaluate different UMI structures, we used SiMSen-Seq that consists of two sequential PCR steps, i.e., barcoding and adapter PCR, followed by purification and sequencing (Figure 6A). In the barcoding PCR step, primers with UMIs are used to label target DNA. In the subsequent adapter PCR step, barcoded target DNA is amplified by sequencing adapters. The rational of using SiMSen-Seq is that this approach already use three strategies to reduce formation of non-specific PCR products, including (i) the UMI is protected in a stem-loop structure that is closed during the annealing step in the first barcoding PCR, while open in the second adapter PCR (Figure 6B), (ii) the barcoding primer concentration is limited and (iii) the barcoding PCR is terminated by an inactivation buffer containing protease. We designed and evaluated 14 different UMI structures, named A - N. These designs were compared with a reference UMI, consisting of a 12 nucleotide long and randomized sequence (Figures 6C-6D). All structured UMIs incorporate predefined nucleotides at specific positions within the reduced sequence, reducing their capacity to form stable internal structures and dimers. Eleven designs divided the UMIs into smaller segments of randomized nucleotides. Three designs (A, B and C) utilized degenerative nucleotides to reduce the risk of forming G-quadruplexes and internal stem structures. Four designs (D, E, F and G) used variable number and positions of adenine to split the UMI. Three designs (H, I and J) used different combinations of adenine and cytosine, while designs (K, L and M) used different combinations of all nucleotide types except guanine, and design (N) used a UMI structure with a combination of adenine, cytosine and guanine nucleotides. The number of possible UMI combinations for a given design is defined by the UMI length and type of nucleotides allowed. High UMI diversity is required for analysis of large amounts of DNA to reduce the risk that two target DNA molecules are labeled with identical UMI. Three designs (A, H and M) consisted of a shorter barcode, i.e., ten randomized nucleotides, reducing the diversity from 17 to 1.0 million combinations (Figure 6D). The three designs that used degenerate nucleotides displayed a diversity of 4.2 million (A), 2.1 million (C) and 16,000 (B) combinations, respectively.

*Structured UMIs improve assay performance*

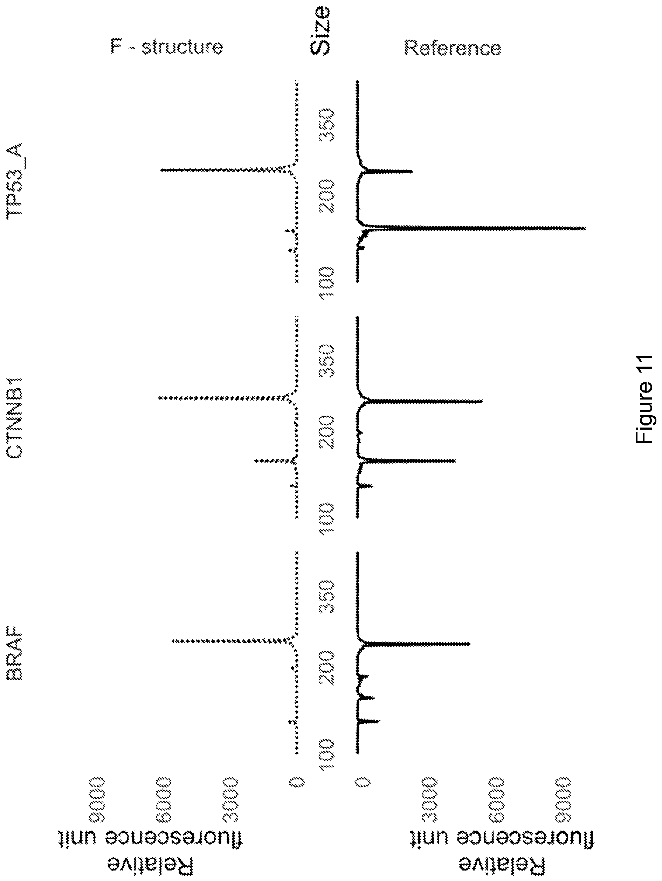

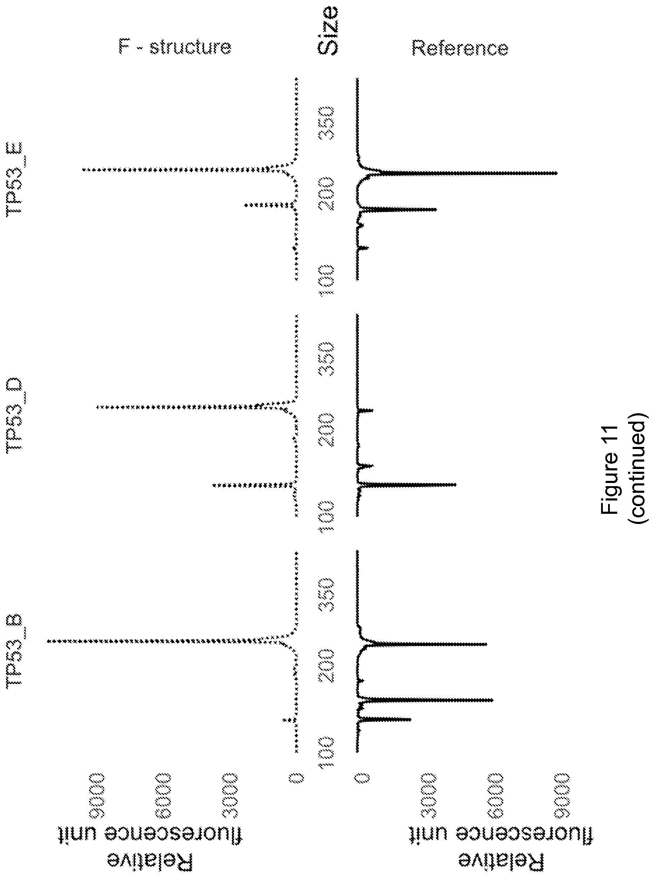

To evaluate assay performance of structured UMI designs, we performed the second adapter PCR as a quantitative PCR and compared data to the reference UMI (Figure 7A). We used six different SiMSen-Seq assays and analyzed 20 ng human genomic DNA as template and water as negative controls. To compare the relative specificity between different structured UMIs we determined the differences in cycle of quantification values between DNA positive and negative samples, where large differences indicate superior performance (Figure 7B). Overall, all structured UMI designs displayed improved specificity compared to the standard UMI. Design (C) performed best with 36 times higher specificity than reference UMI, followed by designs (F), (M) and (G). Next, we evaluated assay performance using capillary gel electrophoresis on final libraries (Figure 7C). We quantified the amount of specific library relative to all generated PCR products. Again, all structured UMI designed were superior to the reference UMI (Figure 7D and Figure 11). Design (F) performed best with 31% more formed specific library compared with reference UMI, followed by designs (C), (H) and (K). To summarize the performance of different structured UMI designs, we ranked them in each evaluation test and summed up their rank score, providing a final ranking list (Figure 7E). Structured UMI design (C) demonstrated best specificity followed by UMI designs (F), (M) and (G). Reduced UMI diversity (A, B, C, H, I and M) showed minor/no general improvements in assay performance compared to designs with maximal UMI diversity. Hence, we selected UMI design (F) for further downstream analysis, since it displayed higher complexity than UMI design (C).

An important feature in the SiMSen-Seq primer design to reduce formation of non-specific PCR products is that the stem structure is closed during the annealing step in barcoding PCR, while open during the annealing step in following adapter PCR (Figures 6A-6B). Melting curve analysis of forward barcoding PCR primer showed that most UMI designs displayed a somewhat lower melting temperature compared to reference UMI, which is caused by the extra nucleotides added to the loop structure (Figures 12A-12B). The melting temperature correlated to the nucleotide length of the loop structure (Figure 12C), but remained $\geq 8°C$ above the annealing temperature of the barcoding PCR, thus ensuring a closed stem structure.

*Validation of improved assay performance using structured UMIs*

In addition to UMI design, assay performance is also dependent on the target DNA sequence of each primer. To determine if structured UMIs are superior to reference UMI, we designed 32 new assays. We used UMI design (F) and tested assay performance using quantitative PCR and capillary gel electrophoresis. Figure 8A shows that 28 of 32 assays displayed improved assay performance with structured UMI design (F) using quantitative PCR. The mean improvement in relative specificity was 8.03. The specificity in library construction based on capillary gel electrophoresis was also improved in 28 of 32 assays with a mean improvement of from 52% to 69% in formation of specific library products (Figure 8B). Out of these 28 assays, 25 were also superior in quantitative PCR. One assay, *PPP6C,* performed poorly in both qPCR and capillary gel electrophoresis.

We also determined the stem melting temperature for all 32 assays (Figure 8C). UMI design (F) decreased the melting temperature with 1.2 °C. Interestingly, we observed a correlation in melting temperature between the assays regardless UMI structure (Spearman's correlation coefficient $= 0.83$, $p < 0.001$). For example, if the melting temperature for UMI design (F) was high the melting temperature was also high for reference UMI for a specific assay.

*Structured UMI design improves the performance of both smaller and larger multiplexes*

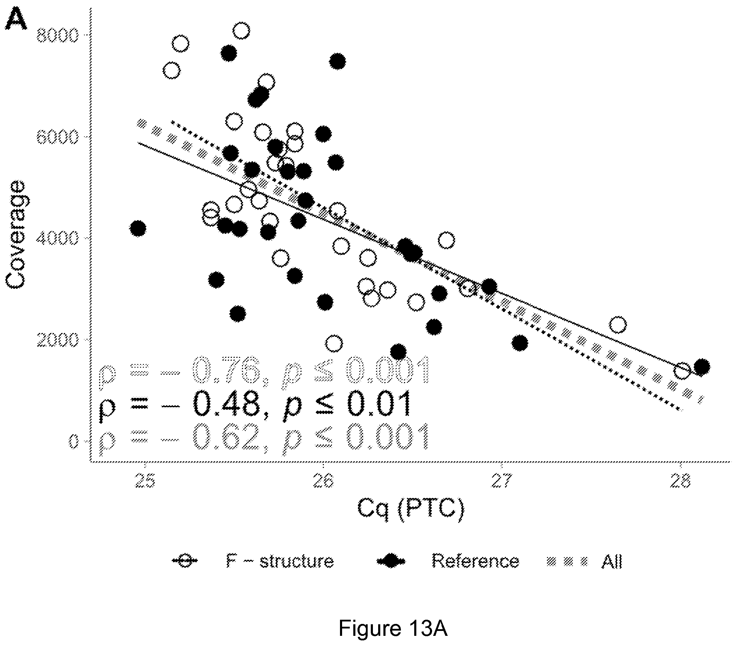

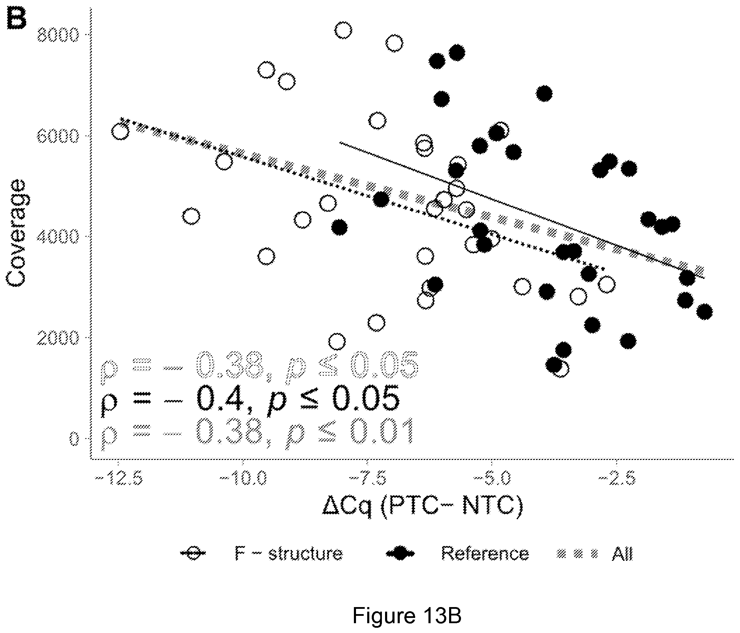

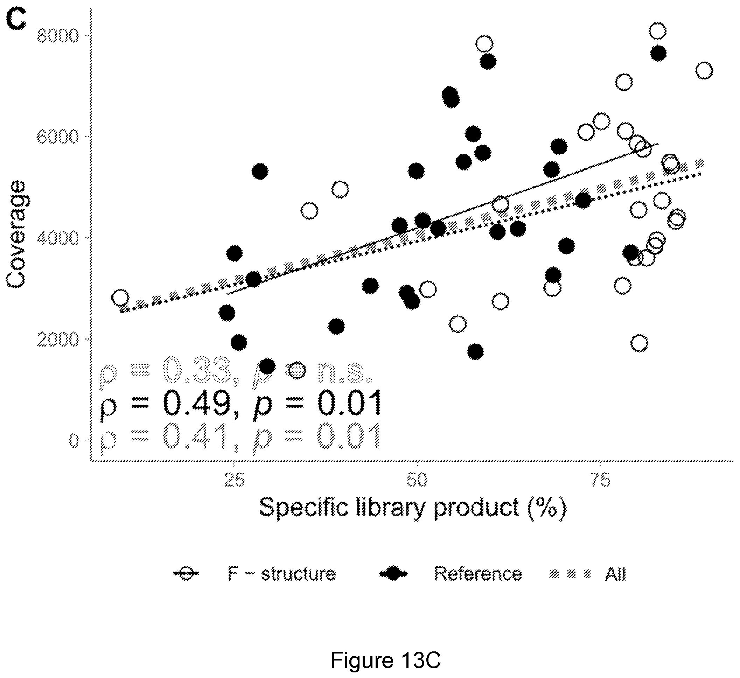

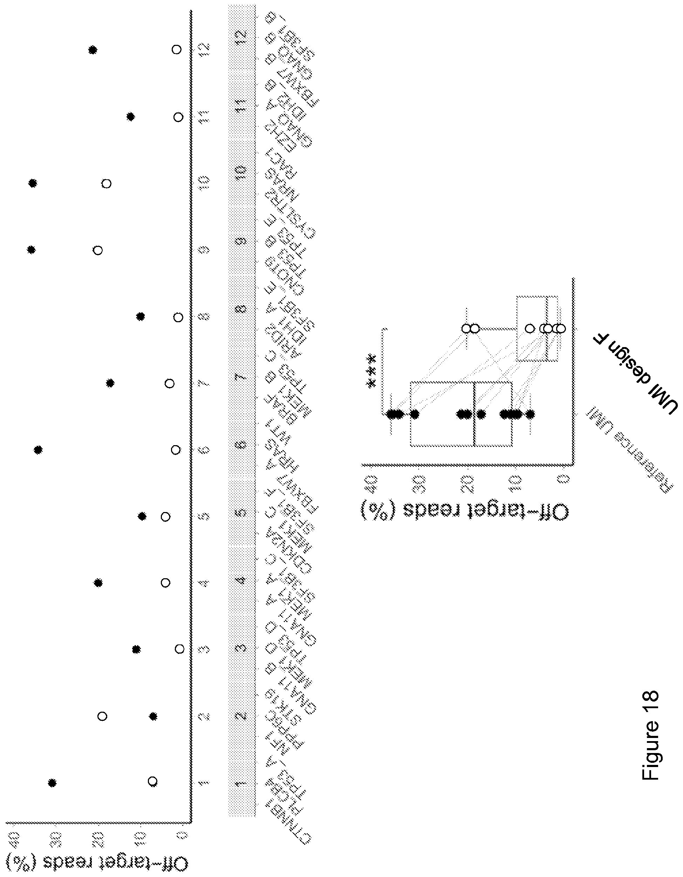

To test the performance of structured UMI design (F) in multiplexing we analyzed 12 different 3-plexes (named 1 to 12). The assays were randomly multiplexed without any overlapping amplicons. Figure 9A shows that the relative specificities were improved in 11 of 12 3-plexes using UMI design (F) compared with reference UMI assessed by quantitative PCR. The mean improvement in relative specificity was 3.9 times. The same assays were also improved when quantifying specificity in library construction using capillary gel electrophoresis. The specific library product improved from 40% to 53%. Next, we sequenced all 3-plexes and determined each assays coverage using consensus reads compared with reference UMI. Twenty-seven out of 36 assays generated higher mean coverage using the UMI design (F). The average assay generated 7.4% higher coverage using UMI design (F) compared with reference UMI. We also compared the coverage of individual assays in 3-plexes with assay characteristics as single-plexes. Overall, all assays with high coverage in 3-plexes performed well as single-plexes as assessed by both quantitative PCR and capillary gel electrophoresis (Figure 13). Another approach to assess assay performance is to quantify the amount of off-target sequencing reads (Figure 18). All but one tri-plex generated lower amounts of off-target reads with UMI design F compared with reference UMI. The mean quantity of off-target reads was reduced from 20% to 6.7%.

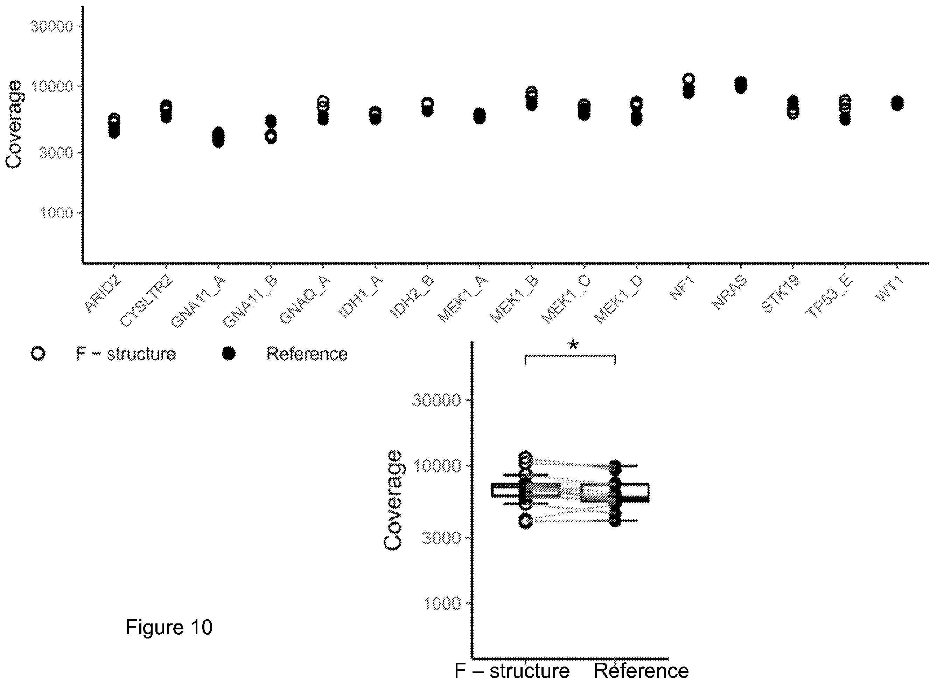

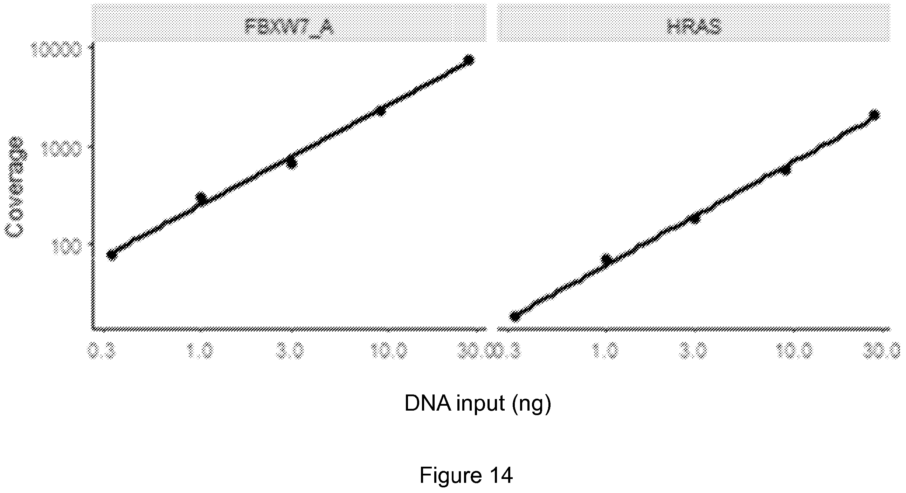

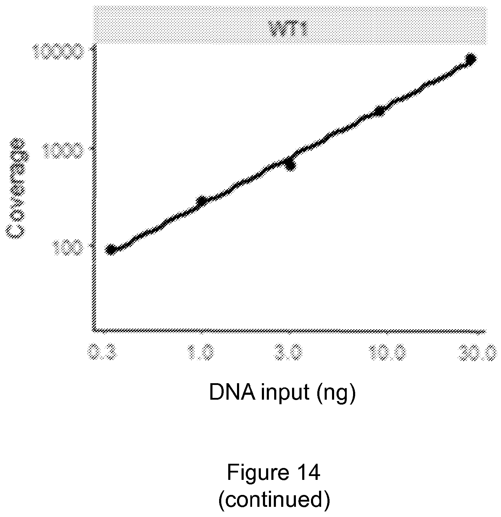

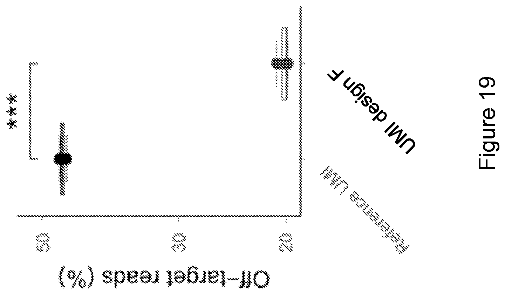

To test the ability to analyze low DNA concentration, we sequenced 3-plex 6 and UMI design (F) applying a dilution series of DNA, ranging from 27 ng to 0.33 ng (Figure 14). All tree assays in 3-plex 6 displayed linearity between observed molecules by sequencing and loaded DNA concentrations. Finally, we sequenced a 16-plex (Figure 10). Twelve of 16 assays generated higher coverage using UMI design (F) than reference UMI with a mean improvement of 10%. The coverage of individual assays in 16-plex correlated weaker with the assay performance of single-plexes compared with 3-plex data (Figure 15). UMIs reduce polymerase-induced errors in sequencing. We observed no difference in errors rates comparing UMI design (F) and reference UMI, neither in the 3-plex nor 16-plex data (Figure 16). The mean number of off-target reads was reduced from 46% to 20% using UMI design F (Figure 19).

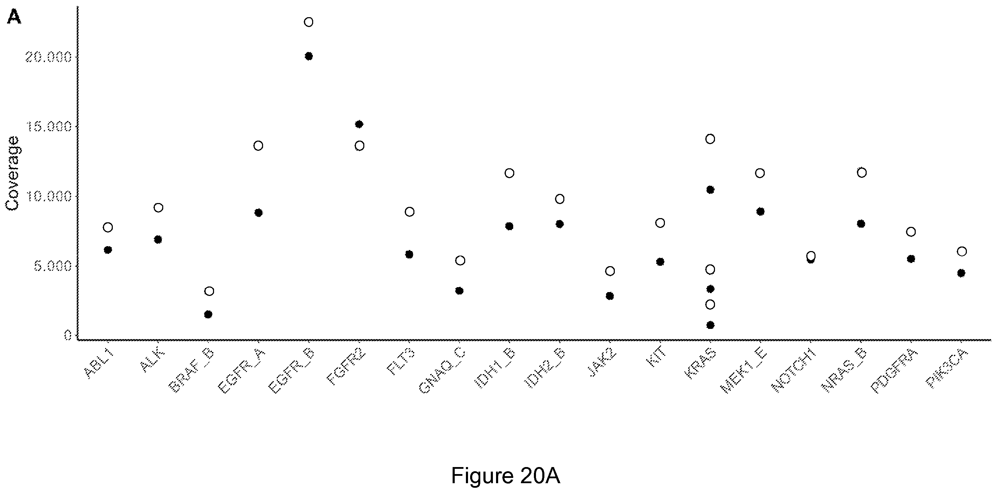

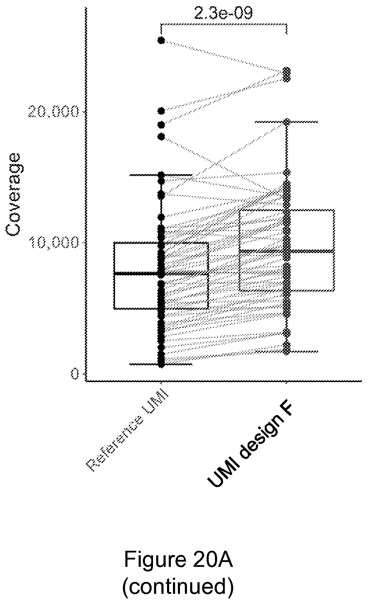

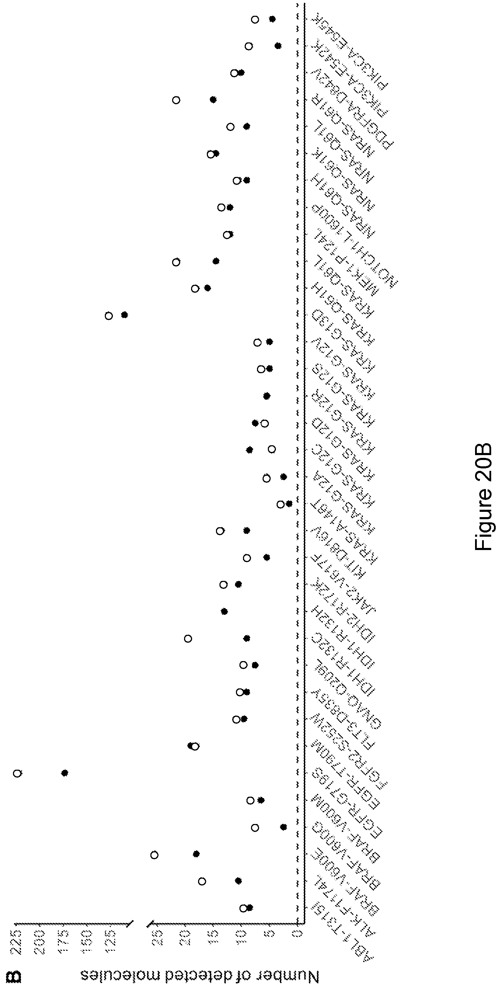

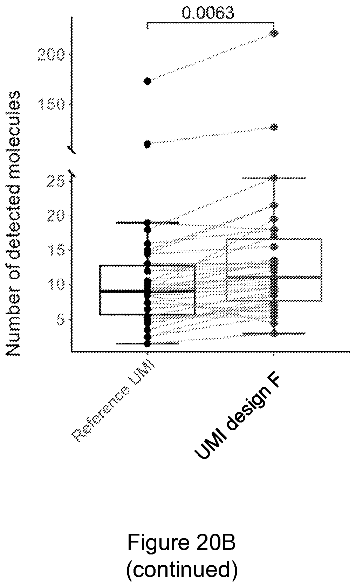

A hot-spot mutation panel consisting of 20 individual assays was analyzed using UMI design F and reference UMI. On-target amplification assessed by digital sequencing is shown in Figure 20A. Number of consensus reads is shown for each individual assay in the hot-spot mutation panel. Mean value for each individual assay is shown, n = 4. Box plots of all mean values are shown to the right. Paired Student's t-test. Panel sensitivity is shown in Figure 20B. The number of detected mutated DNA molecules is shown, n = 4. The number of mutated DNA molecules is assessed as number of consensus reads. Box plots of all mean values are shown to the right. Paired Student's t-test.

The embodiments described above are to be understood as a few illustrative examples of the present invention. It will be understood by those skilled in the art that various modifications, combinations and changes may be made to the embodiments without departing from the scope of the present invention. In particular, different part solutions in the different embodiments can be combined in other configurations, where technically possible. The scope of the present invention is, however, defined by the appended claims.

## Claims

1. A method of amplifying a target nucleic acid molecule in a sample, the method comprising.

    contacting (S1) a sample comprising a target nucleic acid molecule (1) with a forward primer (10) and a reverse primer (20), wherein

    the forward primer (10) comprises, from a 5' end (10A) to a 3' end (10B), an adapter sequence (11) and a target-specific sequence (16);
    the reverse primer (20) comprises, from a 5' end (20A) to a 3' end (20B), an adapter sequence (21) and a target-specific sequence (26);
    the forward primer (10) is a hairpin barcode forward primer (10) and/or the reverse primer (20) is a hairpin barcode reverse primer (20);
    the hairpin barcode forward primer (10) comprises, from the 5' end (10A) to the 3' end (10B), the adapter sequence (11), a unique molecular identifier (UMI) sequence (17) comprising a structured UMI (14), and the target-specific sequence (16) complementary to a portion (2) of the target nucleic acid molecule (1);
    the hairpin barcode reverse primer (20) comprises, from the 5' end (20A) to the 3' end (20B), the adapter sequence (21), a UMI sequence (27) comprising a structured UMI (24), and the target-specific sequence (26) complementary to a portion (3) of the target nucleic acid molecule (1); and
    at least a portion (12, 22) of the adapter sequence (11, 21) of the hairpin barcode forward primer (10) and/or the hairpin barcode reverse primer (20) is complementary to at least a portion (15, 25) of the UMI sequence (17, 27) of the hairpin barcode forward primer (10) and/or the hairpin barcode reverse primer (20), the adapter sequence (11, 21) and the UMI sequence (17, 27) are configured to hybridize to each other at or under a closed annealing temperature and not hybridize to each other at or above an open annealing temperature;

    amplifying (S2) the target nucleic acid molecule (1) by performing polymerase chain reaction (PCR) pre-amplification of the nucleic acid molecule (1) to form a plurality of barcoded PCR products (50), wherein the PCR pre-amplification has an annealing temperature equal to or less than the closed annealing temperature of the hairpin barcode forward primer (10) and/or the hairpin barcode reverse primer (20);
    contacting (S3) the plurality of barcoded PCR products (50) with an adapter-specific forward primer (30) and an adapter-specific reverse primer (40); and
    amplifying (S4) the barcoded PCR products (50) by performing PCR amplification on the barcoded PCR products

(50) to form amplified barcoded PCR products (60), wherein at least a portion of cycles of the PCR amplification has an annealing temperature equal to or greater than the open annealing temperature of the hairpin barcode forward primer (10) and/or the hairpin barcode reverse primer (20).

2. A method for quantifying nucleic acid molecules, the method comprising:

contacting (S10) a sample comprising nucleic acid molecules (1) with P forward primers (10) and Q reverse primers (20), wherein

$P$ is an integer equal to or larger than one and Q is an integer equal to or larger than one;
the P forward primers (10) comprise, from a 5' end (10A) to a 3' end (10B), an adapter sequence (11) and a target-specific sequence (16);
the Q reverse primers (20) comprise, from a 5' end (20A) to a 3' end (20B), an adapter sequence (21) and a target-specific sequence (26);
the P forward primers (10) are P hairpin barcode forward primers (10) and/or the Q reverse primers (20) are Q hairpin barcode reverse primers (20);
each hairpin barcode forward primer (10) comprises, from the 5' end (10A) to the 3' end (10B), the adapter sequence (11), a unique molecular identifier (UMI) sequence (17) comprising a structured UMI (14), and the target-specific sequence (16) complementary to a respective portion (2) of a nucleic acid molecule (1);
each hairpin barcode reverse primer (20) comprises, from the 5' end (20A) to the 3' end (20B), the adapter sequence (21), a UMI sequence (27) comprising a structured UMI (24), and the target-specific sequence (26) complementary to a respective portion (3) of a nucleic acid molecule (1); and
at least a portion (12, 22) of the adapter sequence (11, 21) of the hairpin barcode forward primer (10) and/or the hairpin barcode reverse primer (20) is complementary to at least a portion (15, 25) of the UMI sequence (17, 27) of the hairpin barcode forward primer (10) and/or the hairpin barcode reverse primer (20), the adapter sequence (11, 21) and the UMI sequence (17, 75) are configured to hybridize to each other at or under a closed annealing temperature and not hybridize to each other at or above an open annealing temperature;

amplifying (S11) the nucleic acid molecules (1) by performing polymerase chain reaction (PCR) pre-amplification of the nucleic acid molecules (1) to form a plurality of barcoded PCR products (50), wherein the PCR pre-amplification has an annealing temperature equal to or less than the closed annealing temperature of the hairpin barcode forward primers (10) and/or the hairpin barcode reverse primers (20);
contacting (S12) the plurality of barcoded PCR products (50) with an adapter-specific forward primer (30) and an adapter-specific reverse primer (40);
amplifying (S13) the barcoded PCR products (50) by performing PCR amplification on the barcoded PCR products (50) to form a library of amplified barcoded PCR products (60), wherein at least a portion of cycles of the PCR amplification has an annealing temperature equal to or greater than the open annealing temperature of the hairpin barcode forward primers (10) and/or the hairpin barcode reverse primers (20);
sequencing (S14) at least a respective portion of the amplified barcoded PCR products (60) to form respective sequence reads comprising the UMI(s) (64) and nucleic acid molecule sequence(s) (66);
demultiplexing (S15) the sequence reads based on nucleic acid sequences of the UMIs (64);
mapping (S16) the demultiplexed sequence reads to respective known nucleic acid molecule sequences based on nucleic acid sequences of the nucleic acid molecule sequence(s) (66); and
quantifying (S17) unique nucleic acid molecules (1) based on the demultiplexed and mapped sequence reads.

3. The method according to claim 1 or 2, wherein

the or each hairpin barcode forward primer (10) comprises, from the 5' end (10A) to the 3' end (10B), the adapter sequence (11), the UMI sequence (17) comprising the structured UMI (14) and a stem sequence (15), and the target-specific sequence (16);
the or each hairpin barcode reverse primer (20) comprises, from the 5' end (20A) to the 3' end (20B), the adapter sequence (21), the UMI sequence (27) comprising the structured UMI (24) and a stem sequence (25), and the target-specific sequence (26); and
at least a portion (12, 22) of the adapter sequence (11, 21) of the hairpin barcode forward primer (10) and/or the hairpin barcode reverse primer (20) is complementary to at least a portion of the stem sequence (15, 25) of the hairpin barcode forward primer (10) and/or the hairpin barcode reverse primer (20), the adapter sequence (11, 21) and the stem sequence (15, 25) are configured to hybridize to each other at or under the closed annealing temperature and not hybridize to each other at or above the open annealing temperature.

4. The method according to claim 3, wherein

the or each hairpin barcode forward primer (10) comprises, from the 5' end (10A) to the 3' end (10B), the adapter sequence (11) comprising a 5' stem sequence (12), the UMI sequence (17) comprising the structured UMI (14) and a 3' stem sequence (15), and the target-specific sequence (16);
the or each hairpin barcode reverse primer (20) comprises, from the 5' end (20A) to the 3' end (20B), the adapter sequence (21) comprising a 5' stem sequence (22), the UMI sequence (27) comprising the structured UMI (24) and a 3' stem sequence (25), and the target-specific sequence (26); and
at least a portion of the 5' stem sequence (12, 22) of the hairpin barcode forward primer (10) and/or the hairpin barcode reverse primer (20) is complementary to at least a portion of the 3' stem sequence (15, 25) of the hairpin barcode forward primer (10) and/or the hairpin barcode reverse primer (20), the 5' stem sequence (12, 22) and the 3' stem sequence (15, 25) are configured to hybridize to each other at or under the closed annealing temperature and not hybridize to each other at or above the open annealing temperature.

5. The method according to claim 4, wherein the UMI sequence (17, 27) comprises at least one destabilizing nucleotide (18, 28), preferably at least two destabilizing nucleotides (18, 28), between the structured UMI (14, 24) and the 3' stem sequence (15, 25).

6. The method according to any of the claims 1 to 5, wherein

amplifying (S2, S11) the nucleic acid molecules (1) comprises amplifying (S2, S11) the nucleic acid molecules (1) by performing 1-40 cycles, preferably 1-30 cycles, and more preferably 1 to 25 cycles, such as 2-20 cycles, or 2-15 cycles of PCR pre-amplification of the nucleic acid molecules (1) to form the plurality of barcoded PCR products (50); and/or
amplifying (S4, S13) the barcoded PCR products (50) comprises amplifying (S4, S13) the barcoded PCR products (50) by performing at least 2 cycles, preferably at least 3 cycles, and more preferably at least 5 cycles of PCR amplification on the barcoded PCR products (50) to form a library of amplified barcoded PCR products (60).

7. The method according to any of the claims 1 to 6, further comprising degrading a polymerase used for amplifying the nucleic acid molecules (1) in the PCR pre-amplification prior to amplifying the barcoded PCR products (50) in the PCR amplification.

8. The method according to any one of claims 1 to 7, wherein the structured UMI (14, 24) comprises multiple UMI parts (14A, 14B, 24A, 24B) separated by a respective predefined nucleotide sequence (14C, 24C), preferably wherein

each UMI part (14A, 14B, 24A, 24B) of the structured UMI (14, 24) has a same length in terms of number of nucleotides; and/or
the respective predefined nucleotide sequence (14C, 24C) has a same length in terms of number of nucleotides.

9. The method according to any of the claims 1 to 8, wherein the structured UMI (14, 24) comprises alternating UMI parts (14A, 14B, 24A, 24B), each comprising one or more random nucleotides, and respective predefined nucleotide sequences (14C, 24C), each comprising one or more predefined nucleotides.

10. The method according to any one of claims 1 to 9, wherein the structured UMI (14, 24) is selected from the group consisting of:

$$N_1 M_1 N_2 M_2 N_3 M_3 \cdots N_k;$$

$$N_1 M_1 N_2 M_2 N_3 M_3 \cdots N_k M_k;$$

$$N_1 \cdots N_{k_1} M_1 N_{k_1+1} \cdots N_{k_2} M_2 N_{k_2+1} \cdots N_{k_n};$$

$$N_1 \cdots N_{k_1} M_1 N_{k_1+1} \cdots N_{k_2} M_2 N_{k_2+1} \cdots N_{k_n} M_n;$$

$$N_1 \cdots N_{k_1} M_1 \cdots M_{l_1} N_{k_1+1} \cdots N_{k_2} M_{l_1+1} \cdots M_{l_2} \cdots N_{k_{n-1}+1} \cdots N_{k_n};$$

and

$$N_1 \cdots N_{k_1} M_1 \cdots M_{l_1} N_{k_1+1} \cdots N_{k_2} M_{l_1+1} \cdots M_{l_2} \cdots N_{k_{n-1}+1} \cdots N_{k_n} M_{l_{n-1}+1} \cdots M_{l_n},$$

wherein

each $N$ is independently a random nucleotide among A, T, C and G;

each $M$ is independently a respective predefined nucleotide among A, T, C and G;

$k$ is an integer from 3 up to 20, preferably from 4 up to 20, and more preferably from 6 up to 15;

each $k_i$, i=1...n is independently an integer from 2 up to 8, preferably from 2 up to 6, and more preferably from 2 up to 4;

each $l_j$, j=1...n is independently an integer from 1 up to 8, preferably from 1 up to 6, and more preferably from 1 up to 4;

n is an integer from 3 up to 20, preferably from 4 up to 20, and more preferably from 6 up to 15;

preferably each $l_j$ is equal to or lower than each $k_i$.

11. The method according to claim 12, wherein the UMI (14, 24) is $N_1 \cdots N_{k_1} M_1 \cdots M_{l_1} N_{k_1+1} \cdots N_{k_2} M N_{k_2+1} \cdots N_{k_3} M_{l_1+1} \cdots M_{l_2} N_{k_3+1} \cdots N_{k_4}$;

preferably wherein

each $N$ is independently a random nucleotide among A, T, C and G; and

each $M$ is a respective predefined nucleotide among A, T, C and G, preferably A; and/or preferably wherein

$$k_1 - 1 = k_4 - k_{3+1};$$

$$k_2 - k_{1+1} = k_3 - k_{2+1};$$

and

$$l_1 - 1 = l_2 - l_{1+1};$$

and/or

the UMI (14, 24) is preferably $N_1 N_2 N_3 N_4 M_1 M_2 M_3 N_5 N_6 M_4 N_7 N_8 M_5 M_6 M_7 N_9 N_{10} N_{11} N_{12}$.

12. The method according to any of the claims 1 to 11, wherein the at least a portion (15, 25) of the UMI sequence (17, 27) that is complementary to the at least a portion (12, 22) of the adapter sequence (11, 21) comprises 5-15 nucleotides, preferably 8-15 nucleotides, and more preferably 12-15 nucleotides.

13. The method according to any of the claims 1 to 12, wherein

the adapter-specific forward primer (30) comprises, from a 5' end (30A) to a 3' end (30B), one of a P5 sequence and a P7 sequence (32) and a sequence (33) equal to or complementary to at least a portion of the adapter sequence (11) of the forward primer (10); and

the adapter-specific reverse primer (40) comprises, from a 5' end (40A) to a 3' end (40B), the other of the P5 sequence and the P7 sequence (42) and a sequence (43) equal to or complementary to at least a portion of the adapter sequence (21) of the reverse primer (20), wherein

the P5 sequence preferably comprises AATGATACGGCGACCACCGA as defined in SEQ ID NO: 6, more preferably comprises, such as consists of, AATGATACGGCGACCACCGAGATCTACAC as defined in SEQ ID NO: 7; and

the P7 sequence comprises, such as consists of, CAAGCAGAAGACGGCATACGAGAT as defined in SEQ ID NO: 8.

**14.** The method according to any of the claims 1 to 13, wherein the adapter sequence (11) of the forward primer (10) is different from the adapter sequence (21) of the reverse primer (20).

**15.** The method according to any of the claims 1 to 14, wherein

the closed annealing temperature is lower than a melting temperature of the adapter sequence (11, 21) and the UMI sequence (17, 27); and/or
the open annealing temperature is higher than a melting temperature of the adapter sequence (11, 21) and the UMI sequence (17, 27).

**Patentansprüche**

**1.** Verfahren zum Amplifizieren eines Ziel-Nukleinsäuremoleküls in einer Probe, wobei das Verfahren Folgendes umfasst.

Kontaktieren (S1) einer Probe, die ein Ziel-Nukleinsäuremolekül (1) umfasst, mit einem Vorwärtsprimer (10) und einem Rückwärtsprimer (20), wobei

der Vorwärtsprimer (10), von einem 5'-Ende (10A) bis zu einem 3'-Ende (10B), eine Adaptersequenz (11) und eine zielspezifische Sequenz (16) umfasst;
der Rückwärtsprimer (20), von einem 5'-Ende (20A) bis zu einem 3'-Ende (20B), eine Adaptersequenz (21) und eine zielspezifische Sequenz (26) umfasst;
der Vorwärtsprimer (10) ein Haarnadel-Barcode-Vorwärtsprimer (10) ist und/oder der Rückwärtsprimer (20) ein Haarnadel-Barcode-Rückwärtsprimer (20) ist;
der Haarnadel-Barcode-Vorwärtsprimer (10), von dem 5'-Ende (10A) bis zu dem 3'-Ende (10B), die Adaptersequenz (11), eine eindeutige molekulare Identifikator(UMI)-Sequenz (17), die einen strukturierten UMI (14) umfasst, und die zielspezifische Sequenz (16) komplementär zu einem Abschnitt (2) des Ziel-Nukleinsäuremoleküls (1) umfasst;
der Haarnadel-Barcode-Rückwärtsprimer (20), von dem 5'-Ende (20A) bis zu dem 3'-Ende (20B), die Adaptersequenz (21), eine UMI-Sequenz (27), die einen strukturierten UMI (24) umfasst, und die zielspezifische Sequenz (26) komplementär zu einem Abschnitt (3) des Ziel-Nukleinsäuremoleküls (1) umfasst; und
mindestens ein Abschnitt (12, 22) der Adaptersequenz (11, 21) des Haarnadel-Barcode-Vorwärtsprimers (10) und/oder des Haarnadel-Barcode-Rückwärtsprimers (20) komplementär zu mindestens einem Abschnitt (15, 25) der UMI-Sequenz (17, 27) des Haarnadel-Barcode-Vorwärtsprimers (10) und/oder des Haarnadel-Barcode-Rückwärtsprimers (20) ist, wobei die Adaptersequenz (11, 21) und die UMI-Sequenz (17, 27) dazu konfiguriert sind, bei oder unterhalb einer geschlossenen Annealing-Temperatur miteinander zu hybridisieren und bei oder oberhalb einer offenen Annealing-Temperatur nicht miteinander zu hybridisieren;

Amplifizieren (S2) des Ziel-Nukleinsäuremoleküls (1) durch Durchführen einer Polymerasekettenreaktion(PCR)-Voramplifikation des Nukleinsäuremoleküls (1), um eine Vielzahl von mit Barcode versehenen PCR-Produkten (50) zu bilden, wobei die PCR-Voramplifikation eine Annealing-Temperatur aufweist, die gleich der oder geringer als die geschlossene Annealing-Temperatur des Haarnadel-Barcode-Vorwärtsprimers (10) und/oder des Haarnadel-Barcode-Rückwärtsprimers (20) ist;
Kontaktieren (S3) der Vielzahl von mit Barcode versehenen PCR-Produkten (50) mit einem adapterspezifischen Vorwärtsprimer (30)
und einem adapterspezifischen Rückwärtsprimer (40); und
Amplifizieren (S4) der mit Barcode versehenen PCR-Produkte (50) durch Durchführen einer PCR-Amplifikation an den mit Barcode versehenen PCR-Produkten (50), um amplifizierte mit Barcode versehene PCR-Produkte (60) zu bilden, wobei mindestens ein Abschnitt von Zyklen der PCR-Amplifikation eine Annealing-Temperatur aufweist, die gleich der oder größer als die offene Annealing-Temperatur des Haarnadel-Barcode-Vorwärtsprimers (10) und/oder des Haarnadel-Barcode-Rückwärtsprimers (20) ist.

**2.** Verfahren zum Quantifizieren von Nukleinsäuremolekülen, wobei das Verfahren Folgendes umfasst:

Kontaktieren (S10) einer Probe, die Nukleinsäuremoleküle (1) umfasst, mit P Vorwärtsprimern (10) und Q Rückwärtsprimern (20), wobei

P eine ganze Zahl gleich oder größer als eins ist und Q eine ganze Zahl gleich oder größer als eins ist;
die P Vorwärtsprimer (10), von einem 5'-Ende (10A) bis zu einem 3'-Ende (10B), eine Adaptersequenz (11) und eine zielspezifische Sequenz (16) umfassen;
die Q Rückwärtsprimer (20), von einem 5'-Ende (20A) bis zu einem 3'-Ende (20B), eine Adaptersequenz (21) und eine zielspezifische Sequenz (26) umfassen;
die *P* Vorwärtsprimer (10) *P* Haarnadel-Barcode-Vorwärtsprimer (10) sind und/oder die Q Rückwärtsprimer (20) *Q* Haarnadel-Barcode-Rückwärtsprimer (20) sind;
jeder Haarnadel-Barcode-Vorwärtsprimer (10), von dem 5'-Ende (10A) bis zu dem 3'-Ende (10B), die Adaptersequenz (11), eine eindeutige molekulare Identifikator(UMI)-Sequenz (17), die einen strukturierten UMI (14) umfasst, und die zielspezifische Sequenz (16) komplementär zu einem jeweiligen Abschnitt (2) eines Nukleinsäuremoleküls (1) umfasst;
jeder Haarnadel-Barcode-Vorwärtsprimer (20), von dem 5'-Ende (20A) bis zu dem 3'-Ende (20B), die Adaptersequenz (21), eine UMI-Sequenz (27), die einen strukturierten UMI (24) umfasst, und die zielspezifische Sequenz (26) komplementär zu einem jeweiligen Abschnitt (3) eines Nukleinsäuremoleküls (1) umfasst; und
mindestens ein Abschnitt (12, 22) der Adaptersequenz (11, 21) des Haarnadel-Barcode-Vorwärtsprimers (10) und/oder des Haarnadel-Barcode-Rückwärtsprimers (20) komplementär zu mindestens einem Abschnitt (15, 25) der UMI-Sequenz (17, 27) des Haarnadel-Barcode-Vorwärtsprimers (10) und/oder des Haarnadel-Barcode-Rückwärtsprimers (20) ist, wobei die Adaptersequenz (11, 21) und die UMI-Sequenz (17, 75) dazu konfiguriert sind, bei oder unterhalb einer geschlossenen Annealing-Temperatur miteinander zu hybridisieren und bei oder oberhalb einer offenen Annealing-Temperatur nicht miteinander zu hybridisieren;

Amplifizieren (S11) der Ziel-Nukleinsäuremoleküle (1) durch Durchführen einer Polymerasekettenreaktion(PCR)-Voramplifikation der Nukleinsäuremoleküle (1), um eine Vielzahl von mit Barcode versehenen PCR-Produkten (50) zu bilden, wobei die PCR-Voramplifikation eine Annealing-Temperatur aufweist, die gleich der oder geringer als die geschlossene Annealing-Temperatur der Haarnadel-Barcode-Vorwärtsprimer (10) und/oder der Haarnadel-Barcode-Rückwärtsprimer (20) ist;
Kontaktieren (S12) der Vielzahl von mit Barcode versehenen PCR-Produkten (50) mit einem adapterspezifischen Vorwärtsprimer (30) und einem adapterspezifischen Rückwärtsprimer (40);
Amplifizieren (S13) der mit Barcode versehenen PCR-Produkte (50) durch Durchführen einer PCR-Amplifikation an den mit Barcode versehenen PCR-Produkten (50), um eine Bibliothek von amplifizierten mit Barcode versehenen PCR-Produkten (60) zu bilden, wobei mindestens ein Abschnitt von Zyklen der PCR-Amplifikation eine Annealing-Temperatur aufweist, die gleich der oder größer als die offene Annealing-Temperatur der Haarnadel-Barcode-Vorwärtsprimer (10) und/oder der Haarnadel-Barcode-Rückwärtsprimer (20) ist.
Sequenzieren (S14) mindestens eines jeweiligen Abschnitts der amplifizierten, mit Barcode versehenen PCR-Produkte (60), um jeweilige Sequenzablesungen, die den/die UMI(s) (64) und Nukleinsäuremolekülsequenz(en) (66) umfassen, zu bilden;
Demultiplexen (S15) der Sequenzablesungen basierend auf Nukleinsäuresequenzen der UMIs (64);
Zuordnen (S16) der demultiplexten Sequenzablesungen zu jeweils bekannten Nukleinsäuremolekülsequenzen basierend auf Nukleinsäuresequenzen der Nukleinsäuremolekülsequenz(en) (66); und
Quantifizieren (S17) eindeutiger Nukleinsäuremoleküle (1) basierend auf den demultiplexten und zugeordneten Sequenzablesungen.

3. Verfahren nach Anspruch 1 oder 2, wobei

der oder jeder Haarnadel-Barcode-Vorwärtsprimer (10), von dem 5'-Ende (10A) bis zu dem 3'-Ende (10B), die Adaptersequenz (11), die UMI-Sequenz (17), die den strukturierten UMI (14) und eine Stammsequenz (15) umfasst, und die zielspezifische Sequenz (16) umfasst;
der oder jeder Haarnadel-Barcode-Rückwärtsprimer (20), von dem 5'-Ende (20A) bis zu dem 3'-Ende (20B), die Adaptersequenz (21), die UMI-Sequenz (27), die den strukturierten UMI (24) und eine Stammsequenz (25) umfasst, und die zielspezifische Sequenz (26) umfasst; und
mindestens ein Abschnitt (12, 22) der Adaptersequenz (11, 21) des Haarnadel-Barcode-Vorwärtsprimers (10) und/oder des Haarnadel-Barcode-Rückwärtsprimers (20) komplementär zu mindestens einem Abschnitt der Stammsequenz (15, 25) des Haarnadel-Barcode-Vorwärtsprimers (10) und/oder des Haarnadel-Barcode-Rückwärtsprimers (20) ist, wobei die Adaptersequenz (11, 21) und die Stammsequenz (15, 25) dazu konfiguriert sind, bei oder unterhalb der geschlossenen Annealing-Temperatur miteinander zu hybridisieren und bei oder oberhalb der offenen Annealing-Temperatur nicht miteinander zu hybridisieren.

4. Verfahren nach Anspruch 3, wobei

der oder jeder Haarnadel-Barcode-Vorwärtsprimer (10), von dem 5'-Ende (10A) bis zu dem 3'-Ende (10B), die Adaptersequenz (11), die eine 5'-Stammsequenz (12) umfasst, die UMI-Sequenz (17), welche den strukturierten UMI (14) und eine 3'-Stammsequenz (15) umfasst, und die zielspezifische Sequenz (16) umfasst;
der oder jeder Haarnadel-Barcode-Rückwärtsprimer (20), von dem 5'-Ende (20A) bis zu dem 3'-Ende (20B), die Adaptersequenz (21), die eine 5'-Stammsequenz (22) umfasst, die UMI-Sequenz (27), welche den strukturierten UMI (24) und eine 3'-Stammsequenz (25) umfasst, und die zielspezifische Sequenz (26) umfasst; und
mindestens ein Abschnitt der 5'-Stammsequenz (12, 22) des Haarnadel-Barcode-Vorwärtsprimers (10) und/oder des Haarnadel-Barcode-Rückwärtsprimers (20) komplementär zu mindestens einem Abschnitt der 3'-Stammsequenz (15, 25) des Haarnadel-Barcode-Vorwärtsprimers (10) und/oder des Haarnadel-Barcode-Rückwärtsprimers (20) ist, wobei die 5'-Stammsequenz (12, 22) und die 3'-Stammsequenz (15, 25) dazu konfiguriert sind, bei oder unterhalb der geschlossenen Annealing-Temperatur miteinander zu hybridisieren und bei oder oberhalb der offenen Annealing-Temperatur nicht miteinander zu hybridisieren.

5. Verfahren nach Anspruch 4, wobei die UMI-Sequenz (17, 27) mindestens ein destabilisierendes Nukleotid (18, 28), bevorzugt mindestens zwei destabilisierende Nukleotide (18, 28), zwischen dem strukturierten UMI (14, 24) und der 3'-Stammsequenz (15, 25) umfasst.

6. Verfahren nach einem der Ansprüche 1 bis 5, wobei

Amplifizieren (S2, S11) der Nukleinsäuremoleküle (1) Amplifizieren (S2, S11) der Nukleinsäuremoleküle (1) durch Durchführen von 1-40 Zyklen, bevorzugt 1-30 Zyklen und stärker bevorzugt 1 bis 25 Zyklen, wie etwa 2-20 Zyklen oder 2-15 Zyklen, einer PCR-Voramplifikation der Nukleinsäuremoleküle (1), um die Vielzahl von mit Barcode versehenen PCR-Produkten (50) zu bilden, umfasst; und/oder
Amplifizieren (S4, S13) der mit Barcode versehenen PCR-Produkte (50) Amplifizieren (S4, S13) der mit Barcode versehenen PCR-Produkte (50) durch Durchführen von mindestens 2 Zyklen, bevorzugt mindestens 3 Zyklen und stärker bevorzugt mindestens 5 Zyklen, einer PCR-Amplifikation an den mit Barcode versehenen PCR-Produkten (50), um eine Bibliothek von amplifizierten mit Barcode versehenen PCR-Produkten (60) zu bilden, umfasst.

7. Verfahren nach einem der Ansprüche 1 bis 6, ferner umfassend Denaturieren einer Polymerase, die zum Amplifizieren der Nukleinsäuremoleküle (1) in der PCR-Voramplifikation vor dem Amplifizieren der mit Barcode versehenen PCR-Produkte (50) in der PCR-Amplifikation verwendet wird.

8. Verfahren nach einem der Ansprüche 1 bis 7, wobei der strukturierte UMI (14, 24) mehrere UMI-Teile (14A, 14B, 24A, 24B), die durch eine jeweilige vordefinierte Nukleotidsequenz (14C, 24C) getrennt sind, umfasst, wobei bevorzugt

jeder UMI-Teil (14A, 14B, 24A, 24B) des strukturierten UMI (14, 24) hinsichtlich einer Anzahl von Nukleotiden eine gleiche Länge aufweist; und/oder
die jeweilige vordefinierte Nukleotidsequenz (14C, 24C) hinsichtlich einer Anzahl von Nukleotiden eine gleiche Länge aufweist.

9. Verfahren nach einem der Ansprüche 1 bis 8, wobei der strukturierte UMI (14, 24) alternierende UMI-Teile (14A, 14B, 24A, 24B), die jeweils ein oder mehrere zufällige Nukleotide umfassen, und jeweilige vordefinierte Nukleotidsequenzen (14C, 24C), die jeweils ein oder mehrere vordefinierte Nukleotide umfassen, umfasst.

10. Verfahren nach einem der Ansprüche 1 bis 9, wobei der strukturierte UMI (14, 24) aus der Gruppe ausgewählt ist, die aus Folgendem besteht:

$$N_1 M_1 N_2 M_2 N_3 M_3 \cdots N_k;$$

$$N_1 M_1 N_2 M_2 N_3 M_3 \cdots N_k M_k;$$

$$N_1 \cdots N_{k_1} M_1 N_{k_1+1} \cdots N_{k_2} M_2 N_{k_2+1} \cdots N_{k_n};$$

$$N_1 \cdots N_{k_1} M_1 N_{k_1+1} \cdots N_{k_2} M_2 N_{k_2+1} \cdots N_{k_n} M_n;$$

$$N_1 \cdots N_{k_1} M_1 \cdots M_{l_1} N_{k_1+1} \cdots N_{k_2} M_{l_1+1} \cdots M_{l_2} \cdots N_{k_{n-1}+1} \cdots N_{k_n};$$

and

$$N_1 \cdots N_{k_1} M_1 \cdots M_{l_1} N_{k_1+1} \cdots N_{k_2} M_{l_1+1} \cdots M_{l_2} \cdots N_{k_{n-1}+1} \cdots N_{k_n} M_{l_{n-1}+1} \cdots M_{l_n},$$

wobei

jedes $N$ unabhängig ein zufälliges Nukleotid aus A, T, C und G ist;
jedes M unabhängig ein jeweils vordefiniertes Nukleotid aus A, T, C und G ist;
$k$ eine ganze Zahl aus 3 bis 20, bevorzugt von 4 bis 20 und stärker bevorzugt von 6 bis 15, ist;
jedes $k_i$ i=1...n unabhängig eine ganze Zahl von 2 bis 8, bevorzugt von 2 bis 6 und stärker bevorzugt von 2 bis 4, ist;
jedes $l_j$ j=1...n unabhängig eine ganze Zahl von 1 bis 8, bevorzugt von 1 bis 6 und stärker bevorzugt von 1 bis 4, ist;
n eine ganze Zahl von 3 bis 20, bevorzugt von 4 bis 20 und stärker bevorzugt von 6 bis 15, ist;
bevorzugt jedes $l_j$ gleich oder niedriger als jedes $k_i$ ist.

11. Verfahren nach Anspruch 12, wobei der UMI (14, 24) $N_1 \cdots N_{k_1} M_1 \cdots M_{l_1} N_{k_1+1} \ldots N_{k_2} M N_{k_2+1} \cdots N_{k_3} M_{l_1+1} \cdots M_{l_2} N_{k_3+1} \cdots N_{k_4};$ ist,

wobei bevorzugt

jedes $N$ unabhängig ein zufälliges Nukleotid aus A, T, C und G ist; und
jedes $M$ ein jeweils vordefiniertes Nukleotid aus A, T, C und G, bevorzugt A, ist; und/oder wobei bevorzugt

$$k_1-1 \; = \; k_4-k_{3+1};$$

$$k_2-k_{1+1} \; = \; k_3-k_{2+1};$$

und

$$I_1-1 \; = \; I_2-I_{1+1};$$

und/oder

der UMI (14, 24) bevorzugt Folgendes ist: $N_1 N_2 N_3 N_4 M_1 M_2 M_3 N_5 N_6 M_4 N_7 N_8 M_5 M_6 M_7 N_9 N_{10} N_{11} N_{12}.$

12. Verfahren nach einem der Ansprüche 1 bis 11, wobei mindestens ein Abschnitt (15, 25) der UMI-Sequenz (17, 27), der komplementär zu dem mindestens einen Abschnitt (12, 22) der Adaptersequenz (11, 21) ist, 5-15 Nukleotide, bevorzugt 8-15 Nukleotide und stärker bevorzugt 12-15 Nukleotide, umfasst.

13. Verfahren nach einem der Ansprüche 1 bis 12, wobei

der adapterspezifische Vorwärtsprimer (30), von einem 5'-Ende (30A) bis zu einem 3'-Ende (30B), eine von einer P5-Sequenz und einer P7-Sequenz (32) und eine Sequenz (33), die gleich oder komplementär zu mindestens einem Abschnitt der Adaptersequenz (11) des Vorwärtsprimers (10) ist, umfasst; und
der adapterspezifische Rückwärtsprimer (40), von einem 5'-Ende (40A) bis zu einem 3'-Ende (40B), die andere von der P5-Sequenz und der P7-Sequenz (42) und eine Sequenz (43), die gleich oder komplementär zu mindestens einem Abschnitt der Adaptersequenz (21) des Rückwärtsprimers (20) ist, umfasst, wobei
die P5-Sequenz bevorzugt AATGATACGGCGACCACCGA wie in SEQ ID NO: 6 definiert umfasst, stärker bevorzugt AATGATACGGCGACCACCGAGATCTACAC wie in SEQ ID NO: 7 definiert umfasst, wie etwa aus dieser besteht; und

die P7-Sequenz CAAGCAGAAGACGGCATACGAGAT wie in SEQ ID NO: 8 definiert umfasst, wie etwa aus dieser besteht.

14. Verfahren nach einem der Ansprüche 1 bis 13, wobei sich die Adaptersequenz (11) des Vorwärtsprimers (10) von der Adaptersequenz (21) des Rückwärtsprimers (20) unterscheidet.

15. Verfahren nach einem der Ansprüche 1 bis 14, wobei

die geschlossene Annealing-Temperatur niedriger als eine Schmelztemperatur der Adaptersequenz (11, 21) und der UMI-Sequenz (17, 27) ist; und/oder

die offene Annealing-Temperatur höher als eine Schmelztemperatur der Adaptersequenz (11, 21) und der UMI-Sequenz (17, 27) ist.

**Revendications**

1. Procédé d'amplification d'une molécule d'acide nucléique cible dans un échantillon, le procédé comprenant.

la mise en contact (S1) d'un échantillon comprenant une molécule d'acide nucléique cible (1) avec une amorce directe (10) et une amorce arrière (20), dans lequel

l'amorce directe (10) comprend, d'une extrémité 5' (10A) à une extrémité 3' (10B), une séquence adaptatrice (11) et une séquence spécifique à la cible (16) ;

l'amorce inverse (20) comprend, d'une extrémité 5' (20A) à une extrémité 3' (20B), une séquence adaptatrice (21) et une séquence spécifique à la cible (26) ;

l'amorce directe (10) est une amorce directe de code-barres en épingle à cheveux (10) et/ou l'amorce inverse (20) est une amorce inverse de code-barres en épingle à cheveux (20) ;

l'amorce directe de code-barres en épingle à cheveux (10) comprend, de l'extrémité 5' (10A) à l'extrémité 3' (10B), la séquence adaptatrice (11), une séquence d'identifiant moléculaire unique (UMI) (17) comprenant un UMI structuré (14), et la séquence spécifique à la cible (16) complémentaire d'une partie (2) de la molécule d'acide nucléique cible (1) ;

l'amorce inverse de code-barres en épingle à cheveux (20) comprend, de l'extrémité 5' (20A) à l'extrémité 3' (20B), la séquence adaptatrice (21), une séquence UMI (27) comprenant un UMI structuré (24) et la séquence spécifique à la cible (26) complémentaire d'une partie (3) de la molécule d'acide nucléique cible (1) ; et

au moins une partie (12, 22) de la séquence adaptatrice (11, 21) de l'amorce directe de code-barres en épingle à cheveux (10) et/ou de l'amorce inverse de code-barres en épingle à cheveux (20) est complémentaire d'au moins une partie (15, 25) de la séquence UMI (17, 27) de l'amorce directe de code-barres en épingle à cheveux (10) et/ou de l'amorce inverse de code-barres en épingle à cheveux (20), la séquence adaptatrice (11, 21) et la séquence UMI (17, 27) sont configurées pour s'hybrider l'une à l'autre à ou en dessous d'une température de recuit fermé et ne pas s'hybrider l'une à l'autre à ou au-dessus d'une température de recuit ouvert ;

l'amplification (S2) de la molécule d'acide nucléique cible (1) en effectuant une préamplification par réaction en chaîne par polymérase (PCR) de la molécule d'acide nucléique (1) pour former une pluralité de produits PCR à code-barres (50), la préamplification PCR ayant une température de recuit égale ou inférieure à la température de recuit fermé de l'amorce directe de code-barres en épingle à cheveux (10) et/ou de l'amorce inverse de code-barres en épingle à cheveux (20) ;

la mise en contact (S3) de la pluralité de produits PCR à code-barres (50) avec une amorce directe spécifique à l'adaptateur (30) et une amorce inverse spécifique à l'adaptateur (40) ; et

l'amplification (S4) des produits PCR à code-barres (50) en effectuant une amplification PCR sur les produits PCR à code-barres (50) pour former des produits PCR à code-barres amplifiés (60), dans lequel au moins une partie des cycles de l'amplification PCR a une température de recuit égale ou supérieure à la température de recuit ouvert de l'amorce directe de code-barres en épingle à cheveux (10) et/ou de l'amorce inverse de code-barres en épingle à cheveux (20).

2. Procédé de quantification de molécules d'acide nucléique, le procédé comprenant :

la mise en contact (S10) d'un échantillon comprenant des molécules d'acide nucléique (1) avec des amorces directes P (10) et des amorces inverses Q (20), dans lequel

P est un entier égal ou supérieur à un et Q est un entier égal ou supérieur à un ;
les amorces directes $P$ (10) comprennent, d'une extrémité 5' (10A) à une extrémité 3' (10B), une séquence adaptatrice (11) et une séquence spécifique à la cible (16) ;
les amorces inverses $Q$ (20) comprennent, d'une extrémité 5' (20A) à une extrémité 3' (20B), une séquence adaptatrice (21) et une séquence spécifique à la cible (26) ;
les amorces directes $P$ (10) sont des amorces directes de code-barres en épingle à cheveux $P$ (10) et/ou les amorces inverses $Q$ (20) sont des amorces inverses de code-barres en épingle à cheveux $Q$ (20) ;
chaque amorce directe de code-barres en épingle à cheveux (10) comprend, de l'extrémité 5' (10A) à l'extrémité 3' (10B), la séquence adaptatrice (11), une séquence d'identifiant moléculaire unique (UMI) (17) comprenant un UMI structuré (14), et la séquence spécifique à la cible (16) complémentaire d'une partie respective (2) d'une molécule d'acide nucléique (1) :
chaque amorce inverse de code-barres en épingle à cheveux (20) comprend, de l'extrémité 5' (20A) à l'extrémité 3' (20B), la séquence adaptatrice (21), une séquence UMI (27) comprenant un UMI structuré (24) et la séquence spécifique à la cible (26) complémentaire d'une partie respective (3) d'une molécule d'acide nucléique (1) ; et
au moins une partie (12, 22) de la séquence adaptatrice (11, 21) de l'amorce directe de code-barres en épingle à cheveux (10) et/ou de l'amorce inverse de code-barres en épingle à cheveux (20) est complémentaire d'au moins une partie (15, 25) de la séquence UMI (17, 27) de l'amorce directe de code-barres en épingle à cheveux (10) et/ou de l'amorce inverse de code-barres en épingle à cheveux (20), la séquence adaptatrice (11, 21) et la séquence UMI (17, 75) sont configurées pour s'hybrider l'une à l'autre à ou en dessous d'une température de recuit fermé et ne pas s'hybrider l'une à l'autre à ou au-dessus d'une température de recuit ouvert ;

l'amplification (S11) des molécules d'acide nucléique (1) en effectuant une préamplification par réaction en chaîne par polymérase (PCR) des molécules d'acide nucléique (1) pour former une pluralité de produits PCR à code-barres (50), la préamplification PCR ayant une température de recuit égale ou inférieure à la température de recuit fermé des amorces directes de code-barres en épingle à cheveux (10) et/ou des amorces inverses de code-barres en épingle à cheveux (20) ;
la mise en contact (S12) de la pluralité de produits PCR à code-barres (50) avec une amorce directe spécifique à l'adaptateur (30) et une amorce inverse spécifique à l'adaptateur (40) ;
l'amplification (S13) des produits PCR à code-barres (50) en effectuant une amplification PCR sur les produits PCR à code-barres (50) pour former une bibliothèque de produits PCR à code-barres amplifiés (60), dans lequel au moins une partie des cycles de l'amplification PCR a une température de recuit égale ou supérieure à la température de recuit ouvert des amorces directes de code-barres en épingle à cheveux (10) et/ou des amorces inverses de code-barres en épingle à cheveux (20) ;
le séquençage (S14) d'au moins une partie respective des produits PCR à code-barres amplifiés (60) pour former des lectures de séquences respectives comprenant les UMI (64) et les séquences de molécules d'acide nucléique (66) ;
le démultiplexage (S15) des lectures de séquences en fonction des séquences d'acide nucléique des UMI (64) ;
le mappage (S16) des lectures de séquences démultiplexées sur les séquences de molécules d'acide nucléique connues respectives sur la base des séquences d'acide nucléique de la ou des séquences de molécules d'acide nucléique (66) ; et
la quantification (S17) de molécules d'acide nucléique uniques (1) sur la base des lectures de séquences démultiplexées et mappées.

3. Procédé selon la revendication 1 ou 2, dans lequel

l'amorce ou chaque amorce directe de code-barres en épingle à cheveux (10) comprend, de l'extrémité 5' (10A) à l'extrémité 3' (10B), la séquence adaptatrice (11), la séquence UMI (17) comprenant l'UMI structuré (14) et une séquence tige (15), et la séquence spécifique à la cible (16) ;
l'amorce ou chaque amorce inverse de code-barres en épingle à cheveux (20) comprend, de l'extrémité 5' (20A) à l'extrémité 3' (20B), la séquence adaptatrice (21), la séquence UMI (27) comprenant l'UMI structuré (24) et une séquence tige (25), et la séquence spécifique à la cible (26) ; et

au moins une partie (12, 22) de la séquence adaptatrice (11, 21) de l'amorce directe de code-barres en épingle à cheveux (10) et/ou de l'amorce inverse de code-barres en épingle à cheveux (20) est complémentaire d'au moins une partie de la séquence tige (15, 25) de l'amorce directe de code-barres en épingle à cheveux (10) et/ou de l'amorce inverse de code-barres en épingle à cheveux (20), la séquence adaptatrice (11, 21) et la séquence tige (15, 25) sont configurées pour s'hybrider l'une à l'autre à ou en dessous de la température de recuit fermé et ne pas s'hybrider l'une à l'autre à ou au-dessus de la température de recuit ouvert.

4.  Procédé selon la revendication 3, dans lequel

l'amorce ou chaque amorce directe de code-barres en épingle à cheveux (10) comprend, de l'extrémité 5' (10A) à l'extrémité 3' (10B), la séquence adaptatrice (11) comprenant une séquence tige 5' (12), la séquence UMI (17) comprenant l'UMI structuré (14) et une séquence tige 3' (15), et la séquence spécifique à la cible (16) ;
l'amorce ou chaque amorce inverse de code-barres en épingle à cheveux (20) comprend, de l'extrémité 5' (20A) à l'extrémité 3' (20B), la séquence adaptatrice (21) comprenant une séquence tige 5' (22), la séquence UMI (27) comprenant l'UMI structuré (24) et une séquence tige 3' (25), et la séquence spécifique à la cible (26) ; et
au moins une partie de la séquence tige 5' (12, 22) de l'amorce directe de code-barres en épingle à cheveux (10) et/ou de l'amorce inverse de code-barres en épingle à cheveux (20) est complémentaire d'au moins une partie de la séquence tige 3' (15, 25) de l'amorce directe de code-barres en épingle à cheveux (10) et/ou de l'amorce inverse de code-barres en épingle à cheveux (20), la séquence tige 5' (12, 22) et la séquence tige 3' (15, 25) sont configurées pour s'hybrider l'une à l'autre à ou en dessous de la température de recuit fermé et ne pas s'hybrider l'une à l'autre à ou au-dessus de la température de recuit ouvert.

5.  Procédé selon la revendication 4, dans lequel la séquence UMI (17, 27) comprend au moins un nucléotide déstabilisant (18, 28), de préférence au moins deux nucléotides déstabilisants (18, 28), entre l'UMI structuré (14, 24) et la séquence tige 3' (15, 25).

6.  Procédé selon l'une quelconque des revendications 1 à 5, dans lequel

l'amplification (S2, S11) des molécules d'acide nucléique (1) comprend l'amplification (S2, S11) des molécules d'acide nucléique (1) par la réalisation de 1 à 40 cycles, de préférence de 1 à 30 cycles, et plus préférablement de 1 à 25 cycles, tels que 2 à 20 cycles, ou 2 à 15 cycles de préamplification PCR des molécules d'acide nucléique (1) pour former la pluralité de produits PCR à code-barres (50) ; et/ou
l'amplification (S4, S13) des produits PCR à code-barres (50) comprend l'amplification (S4, S13) des 15 produits PCR à code-barres (50) en effectuant au moins 2 cycles, de préférence au moins 3 cycles, et plus préférablement au moins 5 cycles d'amplification PCR sur les produits PCR à code-barres (50) pour former une bibliothèque de produits PCR à code-barres amplifiés (60).

7.  Procédé selon l'une quelconque des revendications 1 à 6, comprenant en outre la dégradation d'une polymérase utilisée pour amplifier les molécules d'acide nucléique (1) dans la préamplification PCR avant d'amplifier les produits PCR à code-barres (50) dans l'amplification PCR.

8.  Procédé selon l'une quelconque des revendications 1 à 7, dans lequel l'UMI structuré (14, 24) comprend plusieurs parties d'UMI (14A, 14B, 24A, 24B) séparées par une séquence nucléotidique prédéfinie respective (14C, 24C), de préférence dans lequel

chaque partie UMI (14A, 148 , 248, 248) de l'UMI structuré (14, 24) a une même longueur en termes de nombre de nucléotides ; et/ou
la séquence nucléotidique prédéfinie respective (14C, 24C) a une même longueur en termes de nombre de nucléotides.

9.  Procédé selon l'une quelconque des revendications 1 à 8, dans lequel l'UMI structuré (14, 24) comprend des parties UMI alternées (14A, 148, 24A, 24B), chacune comprenant un ou plusieurs nucléotides aléatoires, et des séquences nucléotidiques prédéfinies respectives (14C, 24C), chacune comprenant un ou plusieurs nucléotides prédéfinis.

10. Procédé selon l'une quelconque des revendications 1 à 9, dans lequel l'UMI structuré (14, 24) est sélectionné dans le groupe constitué par :

$$N_1 M_1 N_2 M_2 N_3 M_3 \cdots N_k;$$

$$N_1 M_1 N_2 M_2 N_3 M_3 \cdots N_k M_k;$$

$$N_1 \cdots N_{k_1} M_1 N_{k_1+1} \cdots N_{k_2} M_2 N_{k_2+1} \cdots N_{k_n};$$

$$N_1 \cdots N_{k_1} M_1 N_{k_1+1} \cdots N_{k_2} M_2 N_{k_2+1} \cdots N_{k_n} M_n;$$

$$N_1 \cdots N_{k_1} M_1 \cdots M_{l_1} N_{k_1+1} \cdots N_{k_2} M_{l_1+1} \cdots M_{l_2} \cdots N_{k_{n-1}+1} \cdots N_{k_n}; \qquad \text{et}$$

$$N_1 \cdots N_{k_1} M_1 \cdots M_{l_1} N_{k_1+1} \cdots N_{k_2} M_{l_1+1} \cdots M_{l_2} \cdots N_{k_{n-1}+1} \cdots N_{k_n} M_{l_{n-1}+1} \cdots M_{l_n},$$

dans lequel

chaque $N$ est indépendamment un nucléotide aléatoire parmi A, T, C et G ;
chaque $M$ est indépendamment un nucléotide prédéfini respectif parmi A, T, C et G
$k$ est un nombre entier compris entre 3 et 20, de préférence entre 4 et 20, et plus préférablement entre 6 et 15 ;
chaque $k_i$, i=1...n est indépendamment un entier de 2 à 8, de préférence de 2 à 6, et plus préférablement de 2 à 4 ;
chaque $l_j$, j=1...n est indépendamment un entier de 1 à 8, de préférence de 1 à 6, et plus préférablement de 1 à 4 ;
n est un nombre entier compris entre 3 et 20, de préférence entre 4 et 20, et plus préférablement entre 6 et 15 ;
de préférence, chaque $l_j$ est égal ou inférieur à chaque $k_i$.

**11.** Procédé selon la revendication 12, dans lequel l'UMI (14, 24) est $N_1 \cdots N_{k_1} M_1 \cdots M_{l_1} N_{k_1+1} \ldots N_{k_2} M N_{k_2+1} \cdots N_{k_3} M_{l_1+1} \cdots M_{l_2} N_{k_3+1} \cdots N_{k_4}$;
de préférence dans lequel

chaque $N$ est indépendamment un nucléotide aléatoire parmi A, T, C et G ; et
chaque $M$ est un nucléotide prédéfini respectif parmi A, T, C et G, de préférence A ; et/ou de préférence dans lequel

$$k_1 - 1 = k_4 - k_{3+1} \ ;$$

$$k_2 - k_{1+1} = k_3 - k_{2+1} \ ;$$

et

$$l_1 - 1 = l_2 - l1 + 1 \ ;$$

et/ou l'UMI (14, 24) est de préférence $N_1 N_2 N_3 N_4 M_1 M_2 M_3 N_5 N_6 M_4 N_7 N_8 M_5 M_6 M_7 N_9 N_{10} N_{11} N_{12}$.

**12.** Procédé selon l'une quelconque des revendications 1 à 11, dans lequel l'au moins une partie (15, 25) de la séquence UMI (17, 27) qui est complémentaire de l'au moins une partie (12, 22) de la séquence adaptatrice (11, 21) comprend 5 à 15 nucléotides, de préférence 8 à 15 nucléotides, et plus préférablement 12 à 15 nucléotides.

**13.** Procédé selon l'une quelconque des revendications 1 à 12, dans lequel

l'amorce directe spécifique à l'adaptateur (30) comprend, d'une extrémité 5' (30A) à une extrémité 3' (30B), une séquence P5 ou une séquence P7 (32) et une séquence (33) égale ou complémentaire à au moins une partie de la séquence adaptatrice (11) de l'amorce directe (10) ; et
l'amorce inverse spécifique à l'adaptateur (40) comprend, d'une extrémité 5' (40A) à une extrémité 3' (40B), l'autre séquence parmi la séquence P5 et la séquence P7 (42) et une séquence (43) égale ou complémentaire à au moins une partie de la séquence adaptatrice (21) de l'amorce inverse (20), dans lequel

la séquence P5 comprend de préférence AATGATACGGCGACCACCGA telle que définie dans SEQ ID NO : 6, plus préférablement comprend, ou est constituée par, AATGATACGGCGACCACCGAGATCTACAC telle que définie dans SEQ ID NO : 7 ; et

la séquence P7 comprend, telle que constituée par, CAAGCAGAAGACGGCATACGAGAT telle que définie dans SEQ ID NO : 8.

**14.** Procédé selon l'une quelconque des revendications 1 à 13, dans lequel la séquence adaptatrice (11) de l'amorce directe (10) est différente de la séquence adaptatrice (21) de l'amorce inverse (20).

**15.** Procédé selon l'une quelconque des revendications 1 à 14, dans lequel

la température de recuit fermé est inférieure à une température de fusion de la séquence adaptatrice (11, 21) et de la séquence UMI (17, 27) ; et/ou

la température de recuit ouvert est supérieure à une température de fusion de la séquence adaptatrice (11, 25 21) et de la séquence UMI (17, 27).

| S1 | CONTACT SAMPLE WITH BARCODE PRIMER |
|----|------------------------------------|

↓

| S2 | AMPLIFY TARGET NUCLEIC ACID MOLECULE |
|----|--------------------------------------|

↓

| S3 | CONTACT BARCODED PCR PRODUCTS WITH ADAPTER PRIMERS |
|----|----------------------------------------------------|

↓

| S4 | AMPLIFY BARCODED PCR PRODUCTS |
|----|-------------------------------|

Figure 1

S10 | CONTACT SAMPLE WITH BARCODE PRIMERS

S11 | AMPLIFY TARGET NUCLEIC ACID MOLECULES

S12 | CONTACT BARCODED PCR PRODUCTS WITH ADAPTER PRIMERS

S13 | AMPLIFY BARCODED PCR PRODUCTS

S14 | SEQUENCE AMPLIFED BARCODED PCR PRODUCTS

S15 | DEMULTIPLEX SEQUENCE READS

S16 | MAP DEMULTIPLEXED SEQUENCE READS

S17 | QUANTIFY NUCLEIC ACID MOLECULE(S)

Figure 2

Figure 3A

Figure 3B

Figure 3C

Figure 4A

Figure 4B

Figure 4C

Figure 5A

Figure 5B

Figure 5C

Figure 5D

Figure 6

Figure 6

Figure 7

Figure 7

E

| | N | A | E | D | I | H | L | J | K | B | G | M | F | C |
|---|---|---|---|---|---|---|---|---|---|---|---|---|---|---|
| FINAL | 15 | 14 | 13 | 12 | 11 | 10 | 8.5 | 8.5 | 6.5 | 6.5 | 5 | 4 | 3 | 2 | 1 |
| FA | 15 | 13 | 10 | 14 | 12 | 6 | 3 | 8 | 11 | 4 | 7 | 9 | 5 | 1 | 2 |
| qPCR | 15 | 14 | 13 | 8 | 9 | 11 | 12 | 7 | 3 | 10 | 6 | 2 | 4 | 5 | 1 |
| Reference | N | A | E | D | I | H | L | J | K | B | G | M | F | C |

Figure 7

Figure 8A

Figure 8B

Figure 8C

Figure 9A

Figure 9B

Figure 9C

Figure 10

Figure 11

Figure 11
(continued)

Figure 12

Figure 12

EP 4 573 214 B1

Figure 13A

Figure 13B

Figure 13C

Figure 14

Figure 14
(continued)

Figure 15A

Figure 15B

Figure 15C

Figure 15D

EP 4 573 214 B1

Figure 16A

Figure 16A
(continued)

Figure 16B

EP 4 573 214 B1

Figure 16B
(continued)

Figure 17

**Workflow**

Barcoding PCR

Barcoded PCR product

Protease treatment / Sample dilution

Adapter PCR

Library

Library clean-up → Sequencing → Data analysis

D

| Flanking region | Template specific sequence | Flanking region |
|---|---|---|

TRDV$_n$ segment      TRDJ$_n$ segment

Figure 17

EP 4 573 214 B1

Figure 18

Figure 19

Figure 20A

Figure 20A
(continued)

Figure 20B

Figure 20B
(continued)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 10557134 B
- US 9506119 B
- US 20140255929 A
- WO 2016138080 A

**Non-patent literature cited in the description**

- *Nucleic Acids Research*, 2016, vol. 44 (11), 105
- *Nature Protocols*, 2017, vol. 12 (4), 664-682
- *Genome Research*, 2017, vol. 27 (3), 491-499
- *Clinical Immunology*, 2020, vol. 66 (9), 1228-1237
- *Nat. Protoc*, 2017, vol. 12, 664-682
- *Bioinformatics*, 2009, vol. 25, 1754-1760
- *Nucleic Acids Res*, 2001, vol. 29 (1), 308-311